# EUROPEAN PATENT APPLICATION

(11) **EP 4 844 031 A2**
(43) Date of publication of application: **25.11.2026**
(21) Application number: 26172213.6
(22) Date of filing: 14.04.2026
(51) Int. Cl.: C09B 3/78, C09B 5/00, H10K 50/11

(54) **NOVEL FUSED CYCLIC COMPOUNDS AND ORGANIC LIGHT EMITTING DIODE INCLUDING THE SAME**

(30) Priority: 29.04.2025 KR 20250056089; 04.02.2026 KR 20260022511
(71) Applicant: SFC Co., Ltd., Cheongju-si, Chungcheongbuk-do 28122 (KR)
(72) Inventor: Kim, Tae Young, 28122 Cheongju-si (KR); Kim, Joon Ho, 28122 Cheongju-si (KR); Ryu, Kyeong Hwa, 28122 Cheongju-si (KR); Park, Gi Heon, 28122 Cheongju-si (KR); Lee, Se Jin, 28122 Cheongju-si (KR)
(74) Representative: Epping - Hermann - Fischer

(57) **Abstract**

Disclosed herein are a novel fused cyclic compound useful for an organic light-emitting diode and an organic light-emitting diode comprising same. The fused cyclic compound can be used as a host material for an emission layer in an organic light-emitting diode and can achieve diode characteristics of low voltage driving, high efficiency, and long lifetime.

## Description

### BACKGROUND OF THE INVENTION

### 1. Field of the Invention

The present disclosure relates to a novel fused cyclic compound that can be used in an organic light-emitting diode, and more particularly, to a novel fused cyclic compound that can be used as a host material of an emission layer in an organic light-emitting diode, thereby enabling diode characteristics of low driving voltage, high efficiency, and long lifetime, and to an organic light-emitting diode including same.

### 2. Description of the Related Art

Organic light-emitting diodes (OLEDs), based on self-luminescence, are used to create digital displays with the advantage of having a wide viewing angle and being able to be made thinner and lighter than liquid crystal displays. In addition, an OLED display exhibits a very fast response time. Accordingly, OLEDs find applications in the full color display field or the illumination field.

In general, the term "organic light-emitting phenomenon" refers to a phenomenon in which electrical energy is converted to light energy by means of an organic material. An organic light-emitting diode utilizing the organic light-emitting phenomenon has a structure usually including an anode, a cathode, and an organic material layer interposed therebetween.

In this regard, the organic material layer may have, for the most part, a multilayer structure consisting of different materials, for example, a hole injection layer, a hole transport layer, an emission layer, an electron transport layer, and an electron injection layer in order to enhance the efficiency and stability of the organic light-emitting diode. In the organic light-emitting diode having such a structure, application of a voltage between the two electrodes injects a hole from the anode and an electron from the cathode to the organic layer. In the luminescent zone, the hole and the electron recombine to produce an exciton. When the exciton returns to the ground state from the excited state, the molecule of the organic layer emits light. Such an organic light-emitting diode is known to have characteristics such as self-luminescence, high luminance, high efficiency, low driving voltage, a wide viewing angle, high contrast, and highspeed response.

Materials used as organic material layers in organic light-emitting diodes may be classified, depending on their functions, into light-emitting materials and charge transport materials, such as hole injection materials, hole transport materials, electron transport materials, and electron injection materials.

The light-emitting materials may be classified into polymer-type materials and low-molecular-weight materials according to molecular weight, and may also be classified into fluorescent materials derived from singlet excited states of electrons and phosphorescent materials derived from triplet excited states of electrons according to the emission mechanism.

When only a single material is used as a light-emitting material, intermolecular interactions may cause a shift of the maximum emission wavelength toward a longer wavelength and a reduction in color purity, or may cause emission quenching, thereby reducing device efficiency. Accordingly, a host-dopant system may be used as a light-emitting material in order to improve color purity and increase emission efficiency through energy transfer.

The principle thereof is that when a dopant having a smaller energy band gap than that of the host forming the emission layer is mixed in a small amount into the emission layer, excitons generated in the emission layer are transferred to the dopant, thereby emitting light with high efficiency. At this time, since the wavelength of the host shifts to the wavelength of the dopant, light of a desired wavelength can be obtained depending on the type of dopant used.

In general, in an organic light-emitting diode, electrons and holes are transferred from an electron transport layer to an emission layer and from a hole transport layer to the emission layer, respectively, and recombine each other in the emission layer to form excitons.

By forming the emission layer in an organic light-emitting diode as two or more layers, technologies for enhancing the stability and luminous efficiency of the organic light-emitting diode have been implemented in products. For example, when two emission layers are formed, one of the two emission layers may correspond to an exciton recombination region, and the other may correspond to a triplet-triplet fusion region. In such a structure, energy is transferred from the first emission layer adjacent to the hole transport layer to the second emission layer. In this case, it is advantageous for the material used in the emission layer to have a relatively high triplet energy level and high electron mobility.

For prior art relating to an organic light-emitting diode including two or more emission layers, reference may made to Korean Patent publication No. 10-2021-0077686 A (published on June 25, 2021) that discloses an organic electroluminescent device in which an emission region includes a first emission layer and a second emission layer, the first emission layer and the second emission layer are directly adjacent to each other, the first emission layer is positioned between the anode and the second emission layer, and one of the first emission layer and the second emission layer includes a compound having at least one deuterium atom.

However, despite various attempts in the prior art, including the above reference, to manufacture organic light-emitting diodes, there remains a continuous need for the development of an organic light-emitting diode that includes a plurality of emission layers while achieving low-voltage driving, improved luminous efficiency, and long lifespan characteristics.

### Related Art Documents

### Patent Literature

Korean Patent Publication No. 10-2021-0077686 A (published on June 25, 2021)

### SUMMARY OF THE INVENTION

Accordingly, an aspect of the present disclosure is to provide a novel fused cyclic compound that can be used as a host compound applicable to organic light-emitting diodes including a plurality of emission layers and which allows for the characteristics of low driving voltage, high efficiency, and long lifetime.

In addition, another aspect of the present disclosure is to provide an organic light-emitting diode (OLED) including the fused cyclic compound as a host material therein, which exhibits the characteristics of low driving voltage, high efficiency, and long lifetime.

The present disclosure provides a fused cyclic compound represented by any one of the following Chemical Formulas 1 to 4: 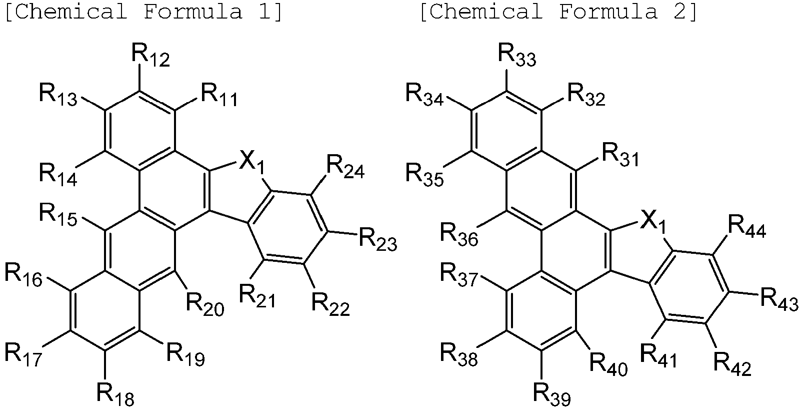 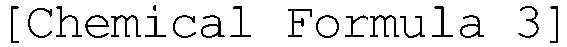 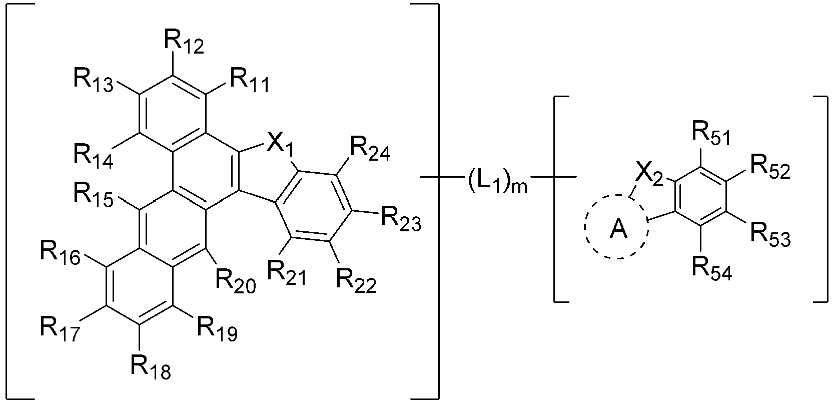 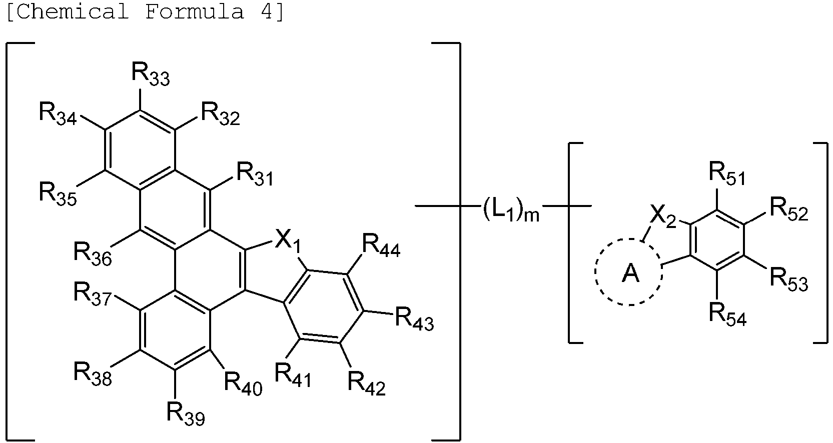 wherein,
X₁ and X₂, which are same or different, are each independently any one selected from O, S, and CR₁R₂,
the substituents R₁, R₂, R₁₁ to R₂₄, R₃₁ to R₄₄, R₅₁ to R₅₄, which are same or different, are each indepdently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₁ and R₂ may be linked to each other to additionally form a mono- or polycyclic aliphatic or aromatic ring,
adjacent substituents in each of the R₁₁ to R₂₄ and the R₃₁ to R₄₄ may be linked to each other to additionally form a mono- or polycyclic aliphatic or aromatic ring,
in Chemical Formulas 3 and 4,
the ring moiety A is a substituted or unsubstituted hydrocarbon ring of 6 to 30 carbon atoms,
linkers L₁, which are same or different, are each independently any one selected from a single bond, a substituted or unsubstituted arylene of 6 to 30 carbon atoms, a substituted or unsubstituted heteroarylene of 3 to 30 carbon atoms, and a substituted or unsubstituted, aliphatic hydrocarbon ring-fused arylene of 8 to 30 carbon atoms,
m is an integer of 1 to 3 wherein when m is 2 or more, the corresponding L₁ are same or different,
any one of R₁₁ to R₂₄, and any one of R₅₁ to R₅₄ and the substituents bonded to the carbon atoms as ring members of the ring moiety A in Chemical Formula 3 are each a single bond to L₁,
   any one of R₃₁ to R₄₄, and any one of R₅₁ to R₅₄ and the substituents bonded to the carbon atoms as ring members of the ring moiety A in Chemical Formula 4 are each a single bond to L₁*,*
wherein the term "substituted" in the expression "a substituted or unsubstituted" used for the compounds of Chemical Formula 1 to Chemical Formula 4 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, and a thionyl of 1 to 30 carbon atoms, with at least one hydrogen atom on the substituent being substitutable with a deuterium or tritium atom.

When employing the fused cyclic compound represented by any one of Chemical Formulas 1 to 4 according to the present disclosure as a host material in an emission layer therein, an organic light-emitting diode having characteristics of lower driving voltage, higher efficiency, and longer lifetime can be provided compared to conventional organic light-emitting diodes. Particularly, as for an organic light-emitting diode with a plurality of emission layers, the characteristics of low driving voltage, high efficiency, and long lifetime can be achieved when the fused cyclic compound employed as a host therein.

For instance, an organic light-emitting diode in which at least one of a plurality of emission layers employs a compound represented by any one of Chemical Formulas 1 to 4 while at least one of the other emission layers employs an anthracene compound represented by Chemical Formula 5 can exhibit low driving voltage, high efficiency, and long lifetime.

### BRIEF DESCRIPTION OF THE DRAWINGS

FIGURE is a schematic diagram of an organic light-emitting diode according to an embodiment of the present disclosure.

### DETAILED DESCRIPTION OF THE INVENTION

Below, a detailed description will be given of the present disclosure. In each drawing of the present disclosure, sizes or scales of components may be enlarged or reduced from their actual sizes or scales for better illustration, and known components may not be depicted therein to clearly show features of the present disclosure. Therefore, the present disclosure is not limited to the drawings. When describing the principle of the embodiments of the present disclosure in detail, details of well-known functions and features may be omitted to avoid unnecessarily obscuring the presented embodiments.

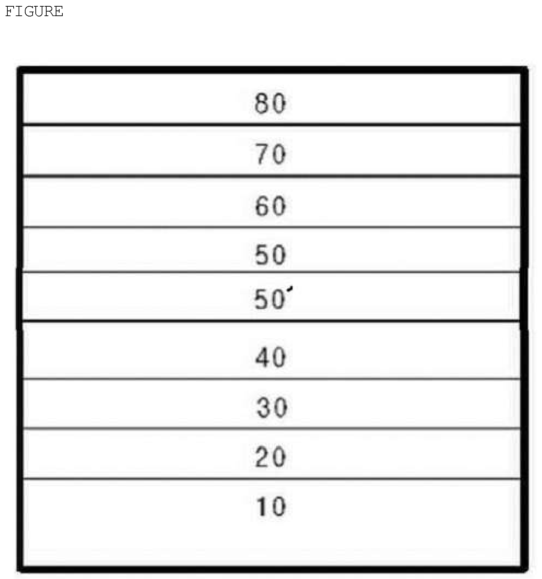

In the drawing, for convenience of description, sizes of components may be exaggerated for clarity. For example, since sizes and thicknesses of components in drawings are arbitrarily shown for convenience of description, the sizes and thicknesses are not limited thereto. Furthermore, throughout the description, the terms "on" and "over" are used to refer to the relative positioning, and mean not only that one component or layer is directly disposed on another component or layer but also that one component or layer is indirectly disposed on another component or layer with a further component or layer being interposed therebetween. Also, spatially relative terms, such as "below", "beneath", "lower", and "between" may be used herein for ease of description to refer to the relative positioning.

Throughout the specification, when a portion may "include" a certain constituent element, unless explicitly described to the contrary, it may not be construed to exclude another constituent element but may be construed to further include other constituent elements. Further, throughout the specification, the word "on" means positioning on or below the object portion, but does not essentially mean positioning on the lower side of the object portion based on a gravity direction.

The present disclosure provides a heterocyclic compound represented by any one of the following Chemical Formulas 1 to 4: 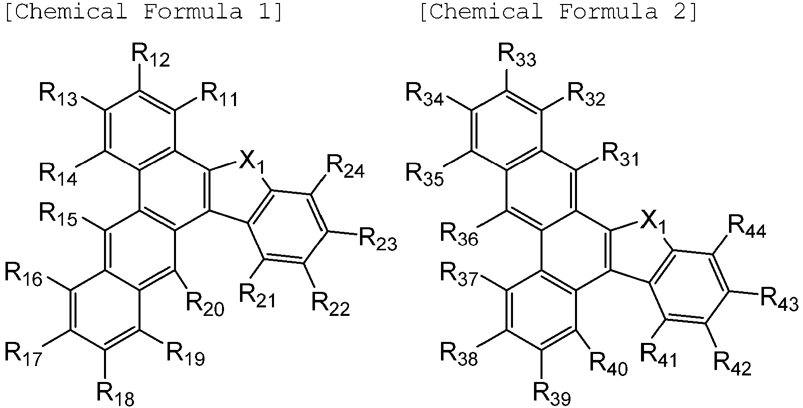 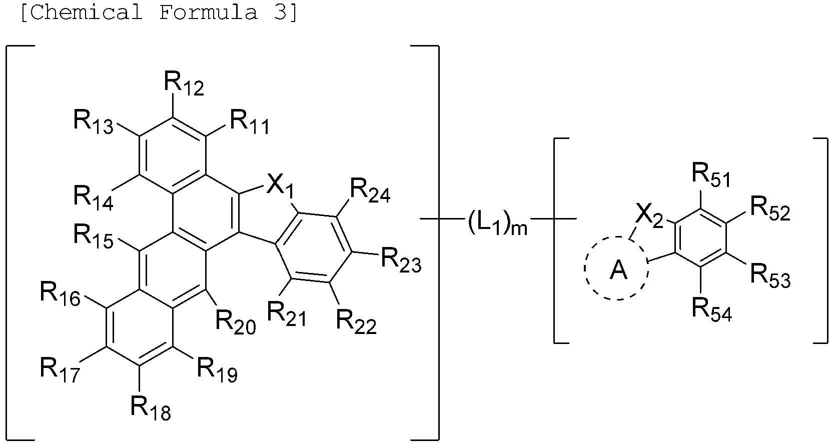 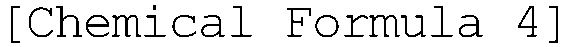 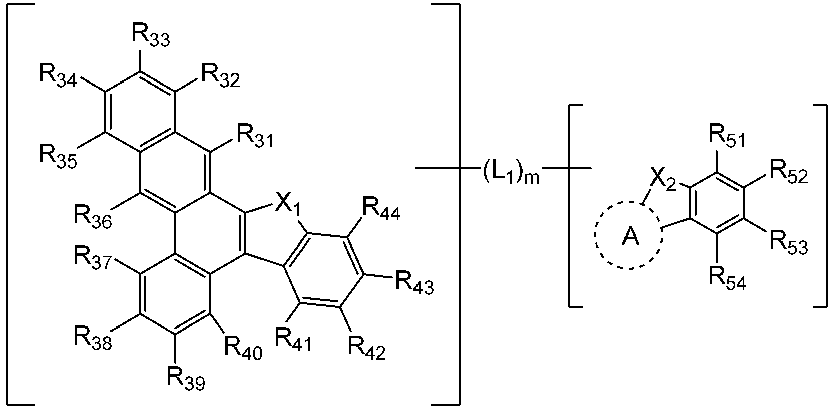 wherein,
X₁ and X₂, which are same or different, are each independently any one of O, S, and CR₁R₂,
R₁, R₂, R₁₁ to R₂₄, R₃₁ to R₄₄, R₅₁ to R₅₄, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₁ and R₂ may be linked to each other to additionally form a mono- or polycyclic aliphatic or aromatic ring,
adjacent substituents in each of the R₁₁ to R₂₄ and the R₃₁ to R₄₄ may be linked to each other to additionally form a mono- or polycyclic aliphatic or aromatic ring,
in Chemical Formulas 3 and 4,
the ring moiety A is a substituted or unsubstituted hydrocarbon ring of 6 to 30 carbon atoms,
linkers L₁, which are same or different, are each independently any one selected from a single bond, a substituted or unsubstituted arylene of 6 to 30 carbon atoms, a substituted or unsubstituted heteroarylene of 3 to 30 carbon atoms, and a substituted or unsubstituted, aliphatic hydrocarbon ring-fused arylene of 8 to 30 carbon atoms,
m is an integer of 1 to 3 wherein when m is 2 or more, the corresponding L₁ are same or different,
any one of R₁₁ to R₂₄, and any one of R₅₁ to R₅₄ and the substituents bonded to the carbon atoms as ring members of the ring moiety A in Chemical Formula 3 are each a single bond to L₁,
   any one of R₃₁ to R₄₄, and any one of R₅₁ to R₅₄ and the substituents bonded to the carbon atoms as ring members of the ring moiety A in Chemical Formula 4 are each a single bond to L₁,
wherein the term "substituted" in the expression "a substituted or unsubstituted" used for the compounds of Chemical Formula 1 to Chemical Formula 4 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, and a thionyl of 1 to 30 carbon atoms, with at least one hydrogen atom on the substituent being substitutable with a deuterium or tritium atom.

The expression indicating the number of carbon atoms, such as in "a substituted or unsubstituted alkyl of 1 to 30 carbon atoms", "a substituted or unsubstituted aryl of 5 to 50 carbon atoms", etc., means the total number of carbon atoms of, for example, the alkyl or aryl radical or moiety alone, exclusive of the number of carbon atoms of substituents attached thereto. For instance, a phenyl group with a butyl at the para position falls within the scope of an aryl of 6 carbon atoms, even though it is substituted with a butyl radical of 4 carbon atoms.

As used herein, the term "aryl" means an organic radical derived from an aromatic hydrocarbon by removing one hydrogen that is bonded to the aromatic hydrocarbon. When the aryl group is substituted, the substituents may be fused with one another to form an additional ring. The aryl group may also include an organic radical obtained by the removal of a single hydrogen atom from an arene ring formed by the fusion of two arene rings.

Concrete examples of the aryl include aromatic radical groups such as phenyl, o-biphenyl, m-biphenyl, p-biphenyl, o-terphenyl, m-terphenyl, p-terphenyl, naphthyl, anthryl, phenanthryl, pyrenyl, indenyl, fluorenyl, spirobifluorenyl, tetrahydronaphthyl, perylenyl, chrysenyl, naphthacenyl, fluoranthenyl, triphenylenyl, and the like, but are not limited thereto. The aryl group may also include organic radicals obtained by the removal of one hydrogen atom from a fused arene ring formed by two arene rings, such as a fluorene ring fused with a phenylene ring or a fluorene ring fused with a phenanthrene ring.

In addition, at least one hydrogen atom in the aryl may be substituted by a deuterium atom, a halogen atom, a hydroxy, a nitro, a cyano, a silyl, an amine, a germanium, an amidino, a hydrazine, a hydrazone, a carboxyl, a sulfonic acid, a phosphoric acid, an alkyl of 1 to 24 carbon atoms, a halogenated alkyl of 1 to 24 carbon atoms, an alkenyl of 2 to 24 carbon atoms, an alkynyl of 2 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, or an alkylheteroaryl of 3 to 24 carbon atoms.

As used herein, the term "aromatic hydrocarbon ring" refers to an aromatic ring composed of carbon and hydrogen atoms and the term "aliphatic hydrocarbon ring" refers to a hydrocarbon ring that is composed of carbon and hydrogen atoms, but does not belong to the aromatic hydrocarbon rings. Particularly, the aliphatic hydrocarbon ring may have a bonding structure of the sp³ orbital for at least 30% of the carbon atoms as ring members, with 0 to 3 double and/or triple bonds within the ring. More particularly, the aliphatic hydrocarbon ring may have a bonding structure of the sp³ orbital for at least 50% of the carbon atoms as ring members, with 0 to 2 double and/or triple bonds within the ring.

As used herein, the term "aliphatic hydrocarbon ring-fused aryl" refers to a cyclic substituent in which two adjacent carbon atoms of the aliphatic hydrocarbon ring are fused with two adjacent carbon atoms of the aryl ring, exclusive of the carbon atom that becomes an organic radical by removal of a hydrogen atom, to form a shared double bond. Specific examples include tetrahydronaphthyl, tetrahydrobenzocycloheptene, tetrahydrophenanthrenyl, tetrahydroanthracenyl, octahydrotriphenylenyl, and the like, but are not limited thereto.

The substituent "heteroaryl" used in the compound of the present disclosure refers to a cyclic aromatic system of 2 to 24 carbon atoms bearing as ring members one to three heteroatoms selected from among N, O, P, Si, S, Ge, Se, and Te. In the aromatic system, two or more rings may be fused. One or more hydrogen atoms on the heteroaryl may be substituted by the same substituents as on the aryl.

Concrete examples of the heteroaryl include thiophenyl, furanyl, pyrrolyl, imidazolyl, thiazolyl, oxazolyl, oxadiazolyl, triazolyl, pyridyl, bipyridyl, pyrimidinyl, triazinyl, acridinyl, carbolinyl, acenaphthoquinoxalinyl, indenoquinazolinyl, indenoisoquinolinyl, indenoquinolinyl, pyridoindolyl, pyridazinyl, pyrazinyl, quinolinyl, quinazolinyl, quinoxalinyl, phthalazinyl, pyridopyrimidinyl, pyridopyrazinyl, pyrazinopyrazinyl, isoquinolinyl, indolyl, carbazolyl, benzoxazolyl, benzimidazolyl, benzothiazolyl, benzocarbazolyl, benzofuranyl, benzothiophenyl, benzoselenophenyl, dibenzothiophenyl, dibenzofuranyl, dibenzoselenophenyl, phenanthrolinyl, thiazolinyl, isoxazolyl, thiadiazolyl, phenoxazinyl, phenothiazinyl, azadibenzofuranyl, azadibenzothiophenyl, azadibenzoselenophenyl, indolocarbazolyl, and the like, but are not limited thereto.

In addition, the term "aromatic heterocyclic ring", as used herein, refers to an aromatic hydrocarbon ring bearing at least one heteroatom as an aromatic ring member. In the aromatic heterocyclic ring, one to three carbon atoms of the aromatic hydrocarbon may be substituted by at least one selected particularly from N, O, P, Si, S, Ge, Se, and Te.

As used herein, the term "aliphatic hydrocarbon ring-fused heteroaryl" refers to the same cyclic substituent as in the aliphatic hydrocarbon ring-fused aryl, with the exception that a heteroaryl, instead of an aryl, is substituted. Specific examples include, but are not limited to, tetrahydroindole, tetrahydrobenzofuranyl, tetrahydrobenzothiophene, tetrahydrocarbazole, tetrahydrodibenzofuranyl, tetrahydroquinoline, and tetrahydroquinoxaline.

In addition, the term "aromatic heterocyclic ring", as used herein, refers to an aromatic hydrocarbon ring bearing at least one heteroatom as an aromatic ring member. In the aromatic heterocyclic ring, one to three carbon atoms of the aromatic hydrocarbon may be substituted by at least one selected particularly from N, O, P, Si, S, Ge, Se, and Te.

As used herein, the term "fused ring in which an aromatic hydrocarbon ring is fused with an aliphatic hydrocarbon ring" means a fused ring in which an aromatic hydrocarbon ring has two adjacent carbon atoms in common with an aliphatic hydrocarbon ring, as exemplified by a tetrahydronaphthalene ring, dihydroindene, and the like, in which the benzene ring shares two adjacent carbon atoms with the cyclohexane ring.

In the present disclosure, the term "fused ring in which a heteroaromatic hydrocarbon ring and an aliphatic hydrocarbon ring are fused to each other" refer to a cyclic radical in which two adjacent carbon atoms as ring members of an aromatic hydrocarbon ring and two adjacent carbon atoms as ring members of an aliphatic hydrocarbon ring are fused with each other to share two carbon atoms therebetween, as exemplified by hexahydrodibenzofuran in which a benzofuran ring and a cyclohexane ring are fused by sharing their respective two adjacent carbon atoms as ring members.

As used herein, the term "alkyl" refers to an alkane missing one hydrogen atom and includes linear or branched structures. Examples of the alkyl substituent useful in the present disclosure include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, and 5-methylhexyl, but are not limited thereto. At least one hydrogen atom of the alkyl may be substituted by the same substituent as in the aryl.

As used herein, the term "halogenated alkyl" refers to an alkyl with a halogen substituted for at least one hydrogen atom. Preferably, the halogen may be a fluorine atom.

The term "cyclo" as used in substituents of the compounds of the present disclosure, such as cycloalkyl, cycloalkoxy, etc., refers to a structure responsible for a mono- or polycyclic ring of saturated hydrocarbons within an alkyl radical, an alkoxy radical, etc. Concrete examples of cycloalkyl include cyclopropyl, cyclobutyl, cyclopentyl, cyclohexyl, methylcyclopentyl, methylcyclohexyl, ethylcyclopentyl, ethylcyclohexyl, adamantyl, dicyclopentadienyl, decahydronaphthyl, norbornyl, bornyl, isobornyl, and so on. One or more hydrogen atoms on the cycloalkyl may be substituted by the same substituents as on the aryl. This is true of the cycloalkoxy.

In the present disclosure, the term "heterocycloalkyl" refers to a cycloalkyl with at least one heteroatom substituted as a ring member for a carbon atom within the ring. Preferably, one to three carbon atoms within the ring may be substituted by at least one selected from N, O, P, S, Si, Ge, Se, and Te.

The term "aromatic hydrocarbon ring- or aromatic heterocyclic ring-fused cycloalkyl", as used herein, refers to a cyclic radical in which two adjacent carbon atoms as ring members of an aromatic or heteroaromatic hydrocarbon ring and two adjacent carbon atoms as ring members of a cycloalkyl are fused with each other to share one double bond therebetween. Specific examples include tetrahydronaphthyl, tetrahydrophenanthrene, tetrahydroquinoline, tetrahydroquinoxaline, cyclopentabenzofuran, and the like, but with no limitations thereto.

The term "aromatic hydrocarbon ring-fused heterocyclic alkyl" is same as the aromatic hydrocarbon ring-fused cycloalkyl, with the exception that at least one carbon atom within the cycloalkyl ring is substituted by a heteroatom. Preferably, one to three carbon atoms within the cycloalkyl ring are substituted by at least one selected from N, O, P, S, Si, Ge, Se, and Te. Specific examples of aromatic hydrocarbon ring-heterocyclic alkyl include hexahydrodibenzofuranyl, hexahydrocarbazole, hexahydrodibenzothiophene, dihydrobenzodioxine, and the like, but with no limitations thereto.

As used herein, the "aliphatic heterocyclic ring-fused aryl or heteroaryl" is same as the aliphatic hydrocarbon ring-fused aryl or heteroaryl, except for an aliphatic heterocyclic ring substituted for the aliphatic hydrocarbon ring. Specific examples include cromane, dihydropyranopyridine, thiocromane, dihydrobenzodioxine, dihydrothiopyranopyridine, dihydropyranopyrimidine, and the like, with no limitations thereto.

The term "aliphatic heterocyclic ring" refers to an aliphatic hydrocarbon bearing at least one heteroatom as a ring member. Preferably, one to three carbon atoms within an aliphatic hydrocarbon are substituted by at least one heteroatom selected from N, O, and S.

The term "alkoxy," as used in the compounds of the present disclosure, refers to an alkyl or cycloalkyl singularly bonded to oxygen. Specific examples of the alkoxy include methoxy, ethoxy, propoxy, isobutoxy, sec-butoxy, pentoxy, iso-amyloxy, hexyloxy, cyclobutyloxy, cyclopentyloxy, adamantyloxy, dicyclopentyloxy, bornyloxy, isobornyloxy, and the like, but are not limited thereto. One or more hydrogen atoms on the alkoxy may be substituted by the same substituents as on the aryl.

Specific examples of the arylalkyl used in the compounds of the present disclosure include phenylmethyl (benzyl), phenylethyl, phenylpropyl, naphthylmethyl, naphthylethyl, and the like, but are not limited thereto. One or more hydrogen atoms on the arylalkyl may be substituted by the same substituents as on the aryl.

Specific examples of the alkylaryl used in the compound of the present disclosure include tolyl, xylenyl, dimethylnaphthyl, t-butylphenyl, t-butylnaphthyl, t-butylphenanthryl, and the like, but are not limited thereto. One or more hydrogen atoms on the alkylaryl may be substituted by the same substituents as on the aryl.

As used herein, the term "alkenyl" refers to an alkyl substituent containing a carbon-carbon double bond between two carbon atoms and the term "alkynyl" refers to an alkyl substituent containing a carbon-carbon triple bond between two carbon atoms.

As used herein, the term "alkylene" refers to an organic radical regarded as derived from an alkane by removal two hydrogen atoms from one carbon atom for methylene or different carbon atoms for ethylene or higher, such as methylene, ethylene, propylene, isopropylene, isobutylene, sec-butylene, tert-butylene, pentylene, isoamylene, hexylene, and the like. One or more hydrogen atoms on the alkylene may be substituted by the same substituents as on the aryl.

In the present disclosure, the term "amine" is intended to encompass or is based on -NH₂, wherein at least one of the two hydrogen atoms bonded to the nitrogen atom in -NH₂ may be substituted with a functional group selected from an alkyl, a cycloalkyl, an aryl, an aliphatic hydrocarbon ring-fused aryl, an arylalkyl, an alkylaryl, a heteroaryl, and an aliphatic hydrocarbon ring-fused heteroaryl. When both of the two hydrogen atoms bonded to the nitrogen atom in -NH₂ are substituted with the above substituents, the respective substituents may be identical to or different from each other. In addition, one or more hydrogen atoms in the alkyl, cycloalkyl, aryl group, aliphatic hydrocarbon ring-fused aryl, arylalkyl, alkylaryl, heteroaryl, or aliphatic hydrocarbon ring-fused heteroaryl group bonded to the nitrogen atom of the amine group may be further substituted with substituents similar to those described above for the aryl group.

Here, each aryl group bonded to the nitrogen atom of the amine, such as aryl, arylheteroaryl, aliphatic hydrocarbon ring-fused aryl, and cycloalkylaryl groups, may be a monocyclic aryl group or a polycyclic aryl group. Likewise, each heteroaryl group in heteroaryl, arylheteroaryl, and the like may be a monocyclic heteroaryl group or a polycyclic heteroaryl group.

Examples of the amine group include an alkylamine in which one or two identical or different alkyl groups are bonded to the nitrogen atom; an arylamine in which one or two identical or different aryl groups are bonded to the nitrogen atom; and an alkylarylamine in which one alkyl group and one aryl group are bonded to the nitrogen atom. Additional examples include a heteroarylamine, an arylheteroarylamine, an alkyl(aliphatic hydrocarbon ring-fused aryl)amine, an aryl(aliphatic hydrocarbon ring-fused aryl)amine, a cycloalkyl(aliphatic hydrocarbon ring-fused aryl)amine, a cycloalkylarylamine, and a heteroaryl(aliphatic hydrocarbon ring-fused aryl)amine.

The substituent "silyl" used in the compound of the present disclosure is intended to encompass or is based on -SiH₃, wherein at least one of the three hydrogen atoms bonded to the silicon atom in -SiH₃ may be substituted with a functional group selected from an alkyl, a cycloalkyl, an aryl, an aliphatic hydrocarbon ring-fused aryl, an arylalkyl, an alkylaryl, a heteroaryl, and an aliphatic hydrocarbon ring-fused heteroaryl. When one, two, or all three hydrogen atoms bonded to the silicon atom in -SiH₃ are substituted with the above substituents, the respective substituents may be identical to or different from each other. In addition, one or more hydrogen atoms in the alkyl, cycloalkyl, aryl, aliphatic hydrocarbon ring-fused aryl, arylalkyl, alkylaryl, heteroaryl, or aliphatic hydrocarbon ring-fused heteroaryl group bonded to the silicon atom of the silyl may be further substituted with substituents similar to those described above for the aryl group.

Here, each aryl group bonded to the silicon atom of the silyl such as aryl, arylheteroaryl, aliphatic hydrocarbon ring-fused aryl, and cycloalkylaryl groups, may be a monocyclic aryl group or a polycyclic aryl group. Likewise, each heteroaryl group in heteroaryl, arylheteroaryl, and the like may be a monocyclic heteroaryl group or a polycyclic heteroaryl group.

Examples of the silyl include an alkylsilyl in which one, two, or three identical or different alkyl groups are bonded to the silicon atom; an arylsilyl in which one, two, or three identical or different aryl groups are bonded to the silicon atom; an alkylarylsilyl in which one alkyl group and one aryl group are bonded to the silicon atom; and an alkylarylheteroarylsilyl in which one alkyl group, one aryl group, and one heteroaryl group are bonded to the silicon atom. Additional examples include alkyl(heteroaryl)silyl, arylheteroarylsilyl, alkyl(aliphatic hydrocarbon ring-fused aryl)silyl, alkyl(aryl)( aliphatic hydrocarbon ring-fused aryl)silyl, cycloalkyl(aliphatic hydrocarbon ring-fused aryl)silyl, cycloalkylarylheteroarylsilyl, and alkyl(heteroaryl)(aliphatic hydrocarbon ring-fused aryl)silyl.

Specific examples of the silyl include trimethylsilyl, triethylsilyl, triphenylsilyl, trimethoxysilyl, dimethoxyphenylsilyl, diphenylmethylsilyl, diphenylvinylsilyl, methylcyclobutylsilyl, and dimethylfurylsilyl.

As used herein, the term "germanium" (or germyl or germane) means a functional group in which the silicon (Si) atom in the above-described silyl is replaced with a germanium (Ge) atom, and the remaining portions are as accounted for by the definitions for the silyl.

Specific examples of the germanium include trimethylgermane, triethylgermane, triphenylgermane, trimethoxygermane, dimethoxyphenylgermane, diphenylmethylgermane, diphenylvinylgermane, methylcyclobutylgermane, and dimethylfurylgermane.

As used herein, the term "oxy" used in the compounds of the present disclosure refers to a substituent in which an oxygen atom is bonded to the terminal position of various substituents, and may specifically be represented by -O-R'. Specific examples include an alkoxy when the terminal substituent (R') is an alkyl group, an aryloxy when R' is an aryl group, a heteroaryloxy when R' is a heteroaryl group, and a cycloalkyloxy when R' is a cycloalkyl group, but with no limitations thereto.

As used herein, the term "thioxy" refers to a substituent in which a sulfur atom is bonded to the terminal position of various substituents, and may specifically be represented by -S-R'. Specific examples include an alkylthioxy when the terminal substituent (R') is an alkyl group, an arylthioxy when R' is an aryl group, a heteroarylthioxy when R' is a heteroaryl group, and a cycloalkylthioxy when R' is a cycloalkyl group, but with no limitations thereto.

As used herein, the term "thionyl" refers to a substituent in which a sulfonyl group is bonded to the terminal position of various substituents, and may specifically be represented by R-(S=O)-R'. Specific examples include an alkylthionyl when the terminal substituent is an alkyl group, an arylthionyl when the terminal substituent is an aryl group, a heteroarylthionyl when the terminal substituent is a heteroaryl group, and a cycloalkylthionyl when the terminal substituent is a cycloalkyl group, but with no limitations thereto.

As used herein, the wording "substituent (B) adjacent to" a substituent (A) in an aromatic ring refers to a substituent (B) bonded to an aromatic ring member (or members) adjacent to the aromatic ring member to which substituent (A) is bonded in the aromatic ring. Likewise, the wording "substituent (B) adjacent to" a substituent (A) in an alicyclic ring refers to a substituent (B) bonded to a ring member (or members) adjacent to the ring carbon atom to which substituent (A) is bonded in the alicyclic ring. Further, the wording "substituent (B) adjacent to" a substituent (A) bonded to a specific carbon atom in an aliphatic chain structure means a substituent (B) bonded to a carbon atom (or atoms) adjacent to the specific carbon atom to which substituent (A) is bonded in the aliphatic chain structure.

In addition, as used herein, the term "alkenyl" refers to an alkyl substituent including one carbon-carbon double bond formed between two carbon atoms, and the term "alkynyl" means an alkyl substituent including one carbon-carbon triple bond formed between two carbon atoms.

As used herein, the term "alkylene" refers to an organic radical regarded as derived from an alkane by removal two hydrogen atoms from one carbon atom for methylene or different carbon atoms for ethylene or higher, such as methylene, ethylene, propylene, isopropylene, isobutylene, sec-butylene, tert-butylene, pentylene, isoamylene, hexylene, and the like. One or more hydrogen atoms on the alkylene may be substituted by the same substituents as on the aryl.

In the present disclosure, a more preferred example of the substituted or unsubstituted aromatic hydrocarbon ring-fused cycloalkyl of 7 to 30 carbon atoms may be a substituted or unsubstituted cycloalkyl group fused with an aromatic hydrocarbon ring and having 9 to 20 carbon atoms.

In the present disclosure, a more preferred example of the substituted or unsubstituted aromatic heterocyclic ring-fused cycloalkyl of 5 to 30 carbon atoms may be a substituted or unsubstituted cycloalkyl group fused with an aromatic heterocyclic ring and having 7 to 20 carbon atoms.

In the present disclosure, a more preferred example of the substituted or unsubstituted aromatic ring-fused heterocycloalkyl of 6 to 30 carbon atoms may be a substituted or unsubstituted heterocycloalkyl group fused with an aromatic hydrocarbon ring and having 9 to 20 carbon atoms.

In the present disclosure, a more preferred example of the substituted or unsubstituted aliphatic hydrocarbon ring-fused aryl of 8 to 30 carbon atoms may be a substituted or unsubstituted aryl group fused with an aliphatic hydrocarbon ring and having 9 to 20 carbon atoms.

In the present disclosure, a more preferred example of the substituted or unsubstituted aliphatic hydrocarbon ring-fused heteroaryl of 5 to 30 carbon atoms may be a substituted or unsubstituted heteroaryl group fused with an aliphatic hydrocarbon ring and having 7 to 20 carbon atoms.

In the present disclosure, a more preferred example of the substituted or unsubstituted aliphatic heterocyclic ring-fused aryl of 6 to 30 carbon atoms may be a substituted or unsubstituted aryl group fused with an aliphatic heterocyclic ring and having 7 to 20 carbon atoms.

In the present disclosure, a more preferred example of the substituted or unsubstituted aliphatic heterocyclic ring-fused heteroaryl of 5 to 30 carbon atoms may be a substituted or unsubstituted heteroaryl group fused with an aliphatic heterocyclic ring and having 6 to 20 carbon atoms.

In a preferable embodiment, the substituent accounted for by the term "substituted" in the expression "substituted or unsubstituted" used for compounds of Chemical Formulas 1 to 4 may be at least one selected from the group consisting of a deuterium atom, a tritium atom, a cyano, a halogen, a thiol, a hydroxy, a nitro, an alkyl of 1 to 12 carbon atoms, a halogenated alkyl of 1 to 12 carbon atoms, an alkenyl of 2 to 12 carbon atoms, an alkynyl of 2 to 12 carbon atoms, a cycloalkyl of 3 to 12 carbon atoms, a heteroalkyl of 1 to 12 carbon atoms, an aryl of 6 to 18 carbon atoms, an arylalkyl of 7 to 20 carbon atoms, an alkylaryl of 7 to 20 carbon atoms, a heteroaryl of 2 to 18 carbon atoms, a heteroarylalkyl of 3 to 18 carbon atoms, an alkyl heteroaryl of 3 to 18 carbon atoms, an aromatic hydrocarbon ring-fused cycloalkyl of 9 to 20 carbon atoms, an aromatic heterocyclic ring-fused cycloalkyl of 7 to 20 carbon atoms, an aromatic hydrocarbon ring-fused heterocycloalkyl of 9 to 20 carbon atoms, an aliphatic hydrocarbon ring-fused aryl of 9 to 20 carbon atoms, an aliphatic hydrocarbon ring-fused heteroaryl of 7 to 20 carbon atoms, an amine of 1 to 18 carbon atoms, a silyl of 1 to 18 carbon atoms, a germanium of 1 to 18 carbon atoms, an oxy of 1 to 18 carbon atoms, a thioxy of 1 to 18 carbon atoms, and a thionyl of 1 to 18 carbon atoms. At least one hydrogen atom within each substituent may be substituted with a deuterium or tritium atom.

In a more preferable embodiment of the present disclosure, the substituted or unsubstituted aromatic hydrocarbon ring-fused cycloalkyl of 7 to 30 carbon atoms may be a substituted or unsubstituted aromatic hydrocarbon ring-fused cycloalkyl of 9 to 20 carbon atoms.

In a more preferable embodiment of the present disclosure, the substituted or unsubstituted aromatic heterocyclic ring-fused cycloalkyl of 5 to 30 carbon atoms may be a substituted or unsubstituted aromatic heterocyclic ring-fused cycloalkyl of 7 to 20 carbon atoms.

In a more preferable embodiment of the present disclosure, the substituted or unsubstituted aromatic hydrocarbon ring-fused heterocycloalkyl of 6 to 30 carbon atoms may be a substituted or unsubstituted aromatic hydrocarbon ring-fused heterocycloalkyl of 9 to 20 carbon atoms.

In a more preferable embodiment of the present disclosure, the substituted or unsubstituted aliphatic hydrocarbon ring-fused aryl of 8 to 30 carbon atoms may be a substituted or unsubstituted aliphatic hydrocarbon ring-fused aryl of 9 to 20 carbon atoms.

In a more preferable embodiment of the present disclosure, the substituted or unsubstituted aliphatic hydrocarbon ring-fused heteroaryl of 5 to 30 carbon atoms may be a substituted or unsubstituted aliphatic hydrocarbon ring-fused heteroaryl of 7 to 20 carbon atoms.

In a more preferable embodiment of the present disclosure, the substituted or unsubstituted aliphatic heterocyclic ring-fused aryl of 6 to 30 carbon atoms may be a substituted or unsubstituted aliphatic heterocyclic ring-fused aryl of 7 to 20 carbon atoms.

In a more preferable embodiment of the present disclosure, the substituted or unsubstituted aliphatic heterocyclic ring-fused heteroaryl of 5 to 30 carbon atoms may be a substituted or unsubstituted aliphatic heterocyclic ring-fused heteroaryl of 6 to 20 carbon atoms.

The fused ring compound, represented by any one of Chemical Formula 1 to 4, which are organic light-emitting compounds available as host materials in the organic light-emitting diode of the present disclosure is technically characterized by the structure in which: the second hexagonal ring in a benz[a]anthracene framework is fused with a hexagonal benzene ring such that a pentagonal ring bearing X₁ as a ring member is formed therebetween, leading to a fused ring structure (Chemical Formulas 1 and 2); or the fused-ring structure represented by Chemical Formula 1 or 2 is linked through L₁ to a specific fused ring structure in which a hexagonal benzene ring is fused with A ring such that a pentagonal ring bearing X₂ as a ring member is formed therebetween (Chemical Formulas 3 and 4).

When a compound represented by any one of Chemical Formulas 1 to 4 is used in an organic light-emitting diode employing a plurality of emission layers, excitons are formed in the first emission layer, and after the excitons are transferred to the second emission layer, triplet-triplet fusion occurs, thereby enabling efficient energy transfer. Accordingly, the diode is expected to exhibit high efficiency and long lifespan characteristics. In contrast, in a conventional diode employing a single emission layer, since all processes of organic light emission must occur within a single layer, the probability of triplet-triplet fusion is reduced, and diode degradation may occur, resulting in decreased efficiency and shortened lifespan. The present disclosure addresses such drawbacks and provides advantages of low-voltage driving, high efficiency, and long lifespan.

In an embodiment, R₁ and R₂ in Chemical Formulas 1 to 4 may be identical to or different from each other, and each independently may be selected from a substituted or unsubstituted alkyl group of 1 to 14 carbon atoms, a substituted or unsubstituted halogenated alkyl group of 1 to 14 carbon atoms, a substituted or unsubstituted aryl group of 6 to 20 carbon atoms, a substituted or unsubstituted cycloalkyl group of 3 to 14 carbon atoms, and a substituted or unsubstituted aliphatic hydrocarbon ring-fused aryl of 7 to 20 carbon atoms, with preference for a substituted or unsubstituted alkyl group of 1 to 10 carbon atoms, a substituted or unsubstituted cycloalkyl group of 3 to 10 carbon atoms, and a substituted or unsubstituted aryl group of 6 to 20 carbon atoms and more preference for a substituted or unsubstituted alkyl group of 1 to 10 carbon atoms.

In an embodiment, in Chemical Formulas 1 to 4, substituents R₁, R₂, R₁₁ to R₂₄, R₃₁ to R₄₄, R₅₁ to R₅₄ may be identical to or different from each other, and each independently may be selected from a hydrogen atom, a deuterium atom, a substituted or unsubstituted aryl of 6 to 30 carbon atoms, a substituted or unsubstituted heteroaryl of 2 to 30 carbon atoms, a substituted or unsubstituted cycloalkyl of 3 to 30 carbon atoms, a substituted or unsubstituted aliphatic hydrocarbon ring-fused aryl of 7 to 30 carbon atoms, a substituted or unsubstituted silyl of 1 to 30 carbon atoms, and a substituted or unsubstituted germanium of 1 to 30 carbon atoms, and more preferably from a hydrogen atom, a deuterium atom, a substituted or unsubstituted aryl of 6 to 20 carbon atoms, a substituted or unsubstituted heteroaryl of 2 to 20 carbon atoms, a substituted or unsubstituted cycloalkyl of 3 to 15 carbon atoms, a substituted or unsubstituted aliphatic hydrocarbon ring-fused aryl of 7 to 20 carbon atoms, a substituted or unsubstituted silyl of 1 to 20 carbon atoms, and a substituted or unsubstituted germanium of 1 to 20 carbon atoms.

In a more preferred embodiment of the present disclosure, when substituents R₁, R₂, R₁₁ to R₂₄, R₃₁ to R₄₄, R₅₁ to R₅₄ in Chemical Formulas 1 to 4 are identical to or different from each other and each independently selected from a hydrogen atom, a deuterium atom, a substituted or unsubstituted aryl of 6 to 30 carbon atoms, a substituted or unsubstituted heteroaryl of 2 to 30 carbon atoms, a substituted or unsubstituted cycloalkyl of 3 to 30 carbon atoms, a substituted or unsubstituted aliphatic hydrocarbon ring-fused aryl of 7 to 30 carbon atoms, a substituted or unsubstituted silyl of 1 to 30 carbon atoms, and a substituted or unsubstituted germanium of 1 to 30 carbon atoms, at least one of R₁₁ to R₂₄ in Chemical Formula 1 is one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms; at least one of R₃₁ to R₄₄ in Chemical Formula 2 may be one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms; at least one of R₁₁ to R₂₄, and R₅₁ to R₅₄ in Chemical Formula 3 may be one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms; and at least one of R₃₁ to R₄₄, and R₅₁ to R₅₄ in Chemical Formula 4 may be one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms.

More preferably, at least one of R₁₁ to R₂₄ in Chemical Formula 1 may be one selected from a substituted or unsubstituted aryl of 6 to 20 carbon atoms, a substituted or unsubstituted heteroaryl of 3 to 20 carbon atoms, a substituted or unsubstituted aliphatic hydrocarbon ring-fused aryl of 8 to 20 carbon atoms, and a substituted or unsubstituted silyl of 1 to 20 carbon atoms; at least one of R₃₁ to R₄₄ in Chemical Formula 2 may be one selected from a substituted or unsubstituted aryl of 6 to 20 carbon atoms, a substituted or unsubstituted heteroaryl of 3 to 20 carbon atoms, a substituted or unsubstituted aliphatic hydrocarbon ring-fused aryl of 8 to 20 carbon atoms, and a substituted or unsubstituted silyl of 1 to 20 carbon atoms; at least one of R₁₁ to R₂₄ and R₅₁ to R₅₄ in Chemical Formula 3 may be one selected from a substituted or unsubstituted aryl of 6 to 20 carbon atoms, a substituted or unsubstituted heteroaryl of 3 to 20 carbon atoms, a substituted or unsubstituted aliphatic hydrocarbon ring-fused aryl of 8 to 20 carbon atoms, and a substituted or unsubstituted silyl of 1 to 20 carbon atoms; and at least one of R₃₁ to R₄₄ and R₅₁ to R₅₄ in Chemical Formula 4 may be one selected from a substituted or unsubstituted aryl of 6 to 20 carbon atoms, a substituted or unsubstituted heteroaryl of 3 to 20 carbon atoms, a substituted or unsubstituted aliphatic hydrocarbon ring-fused aryl of 8 to 20 carbon atoms, and a substituted or unsubstituted silyl of 1 to 20 carbon atoms.

In an embodiment, m in Chemical Formulas 1 to 4 may be 1.

In addition, according to an embodiment of the present disclosure, in Chemical Formula 1, at least 12 of R₁₁ to R₂₄ may be hydrogen or deuterium; in Chemical Formula 2, at least 12 of R₃₁ to R₄₄ may be hydrogen or deuterium; in Chemical Formula 3, at least 12 of R₁₁ to R₂₄ may be hydrogen or deuterium; and in Chemical Formula 4, at least 12 of R₃₁ to R₄₄ may be hydrogen or deuterium.

According to a more preferred embodiment of the present disclosure, in Chemical Formula 1, at least 13 of R₁₁ to R₂₄ are hydrogen or deuterium; in Chemical Formula 2, at least 13 of R₃₁ to R₄₄ are hydrogen or deuterium; in Chemical Formula 3, at least 13 of R₁₁ to R24 may be hydrogen or deuterium; and in Chemical Formula 4, at least 13 of R₃₁ to R₄₄ may be hydrogen or deuterium.

According to an embodiment of the present disclosure, ring moieties A in Chemical Formulas 3 and 4 may each independently be a substituted or unsubstituted aromatic hydrocarbon ring of 6 to 18 carbon atoms, and more preferably a substituted or unsubstituted benzene ring or a substituted or unsubstituted naphthalene ring.

Further, according to an embodiment of the present disclosure, in Chemical Formulas 3 and 4, ring moieties A may each independently be a substituted or unsubstituted aromatic hydrocarbon ring of 18 to 24 carbon atoms, and more preferably a substituted or unsubstituted benz[a]anthracene ring.

In addition, according to an embodiment of the present disclosure, the fused-ring compound represented by any one of Chemical Formulas 1 to 4 may include at least one deuterium atom.

Further, the compound represented by any one of Chemical Formulas 1 to 4 according to the present disclosure may be selected from the group consisting of the following Compound 1-1 to Compound 1-180, but with no limitations thereto:

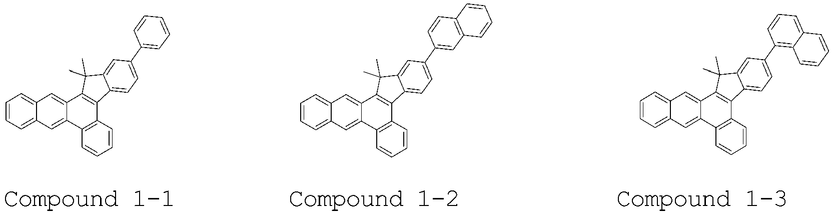

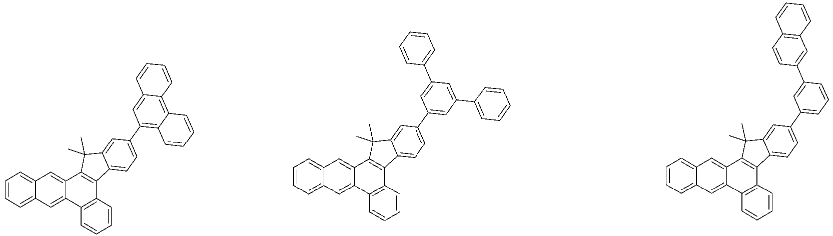

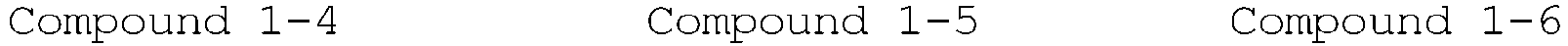

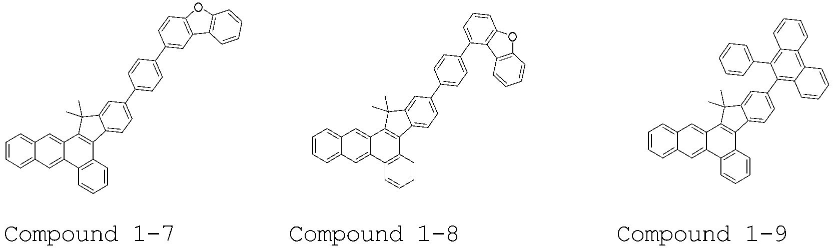

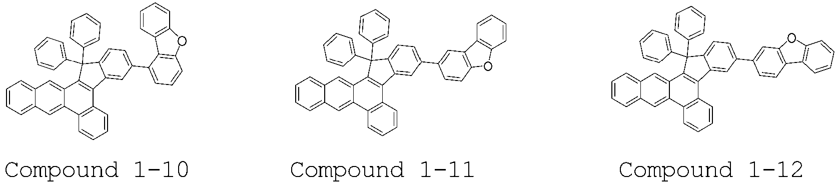

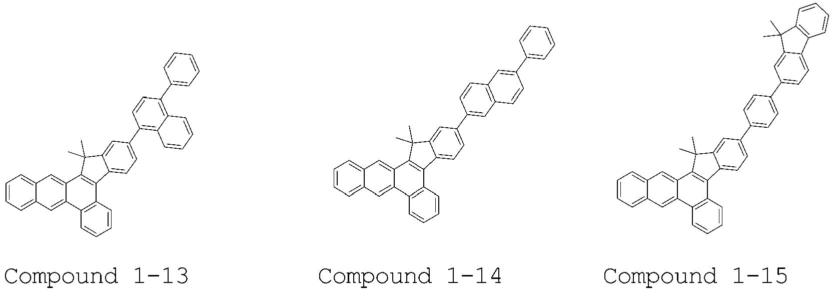

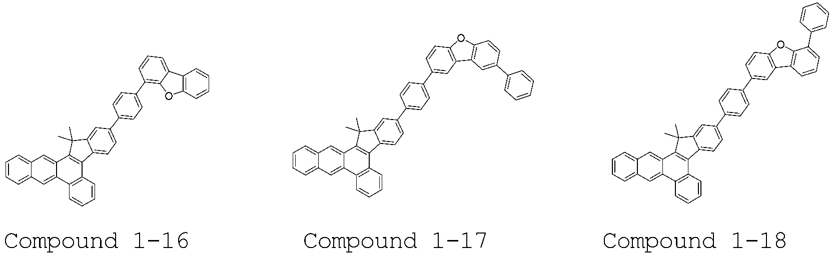

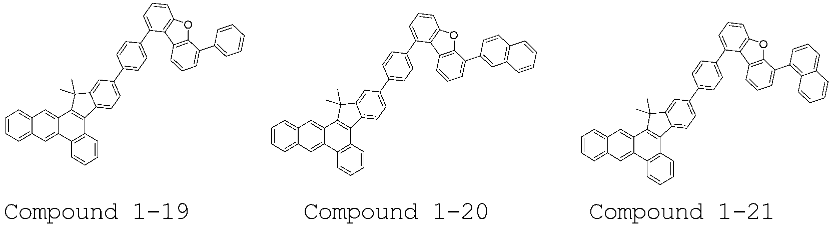

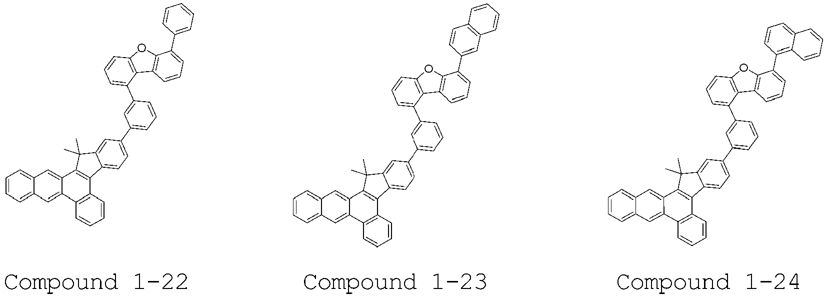

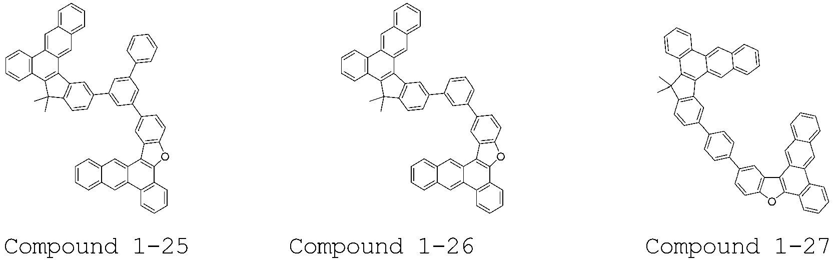

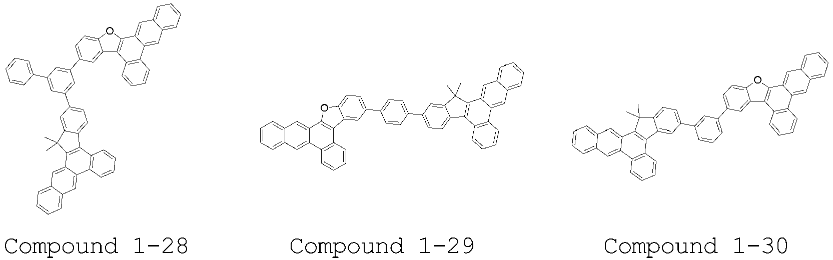

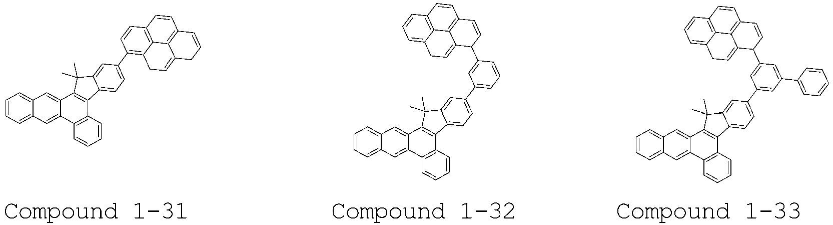

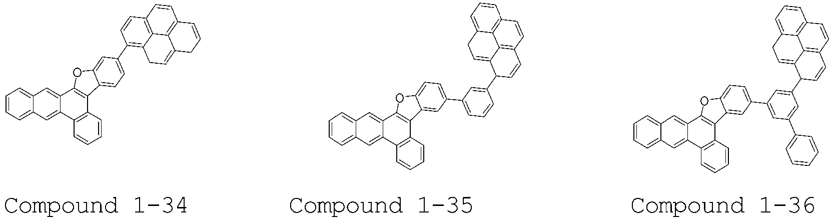

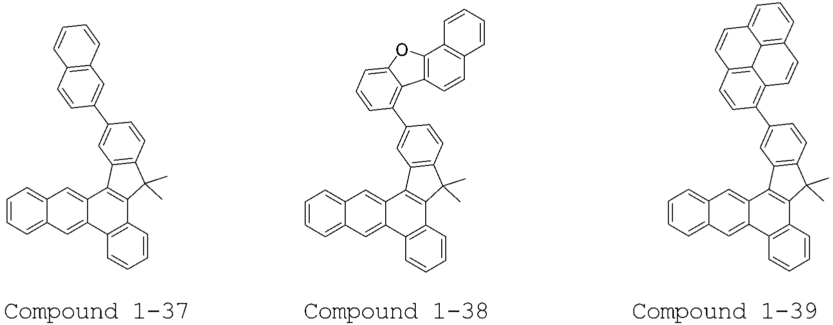

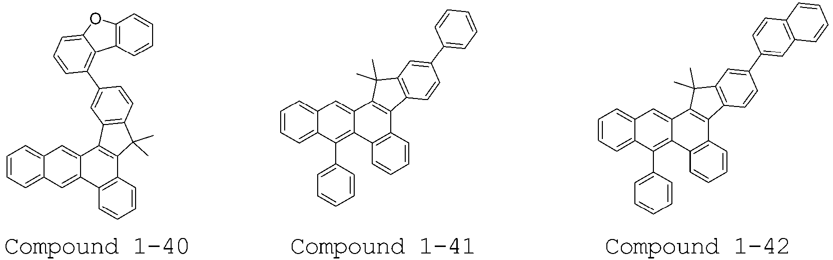

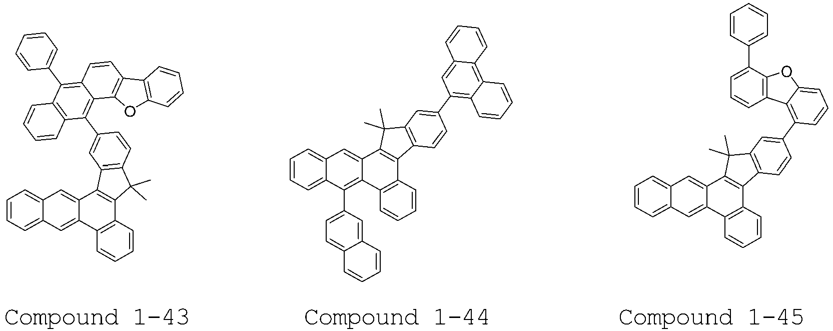

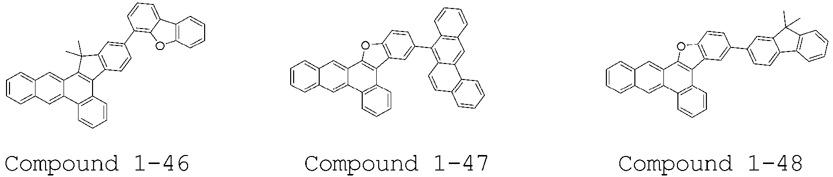

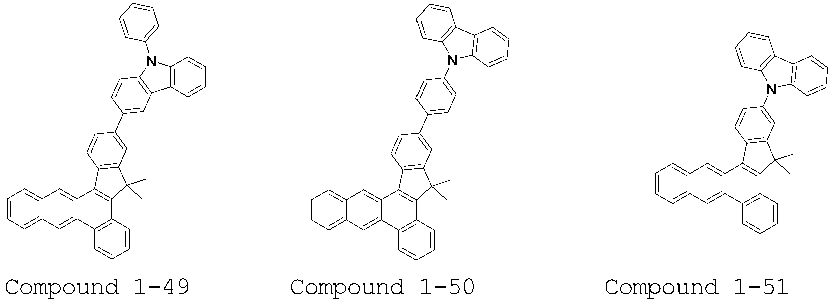

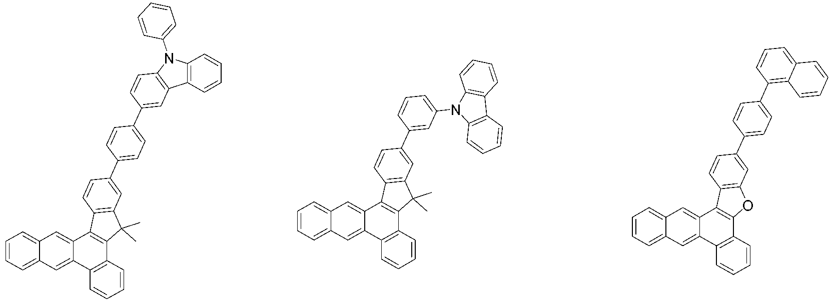

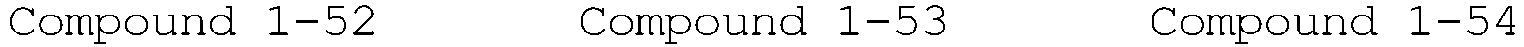

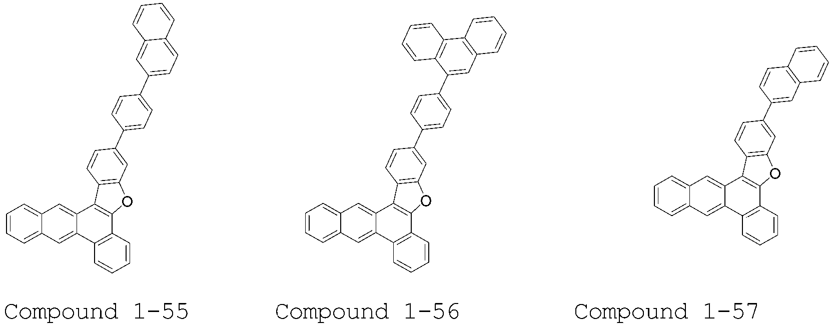

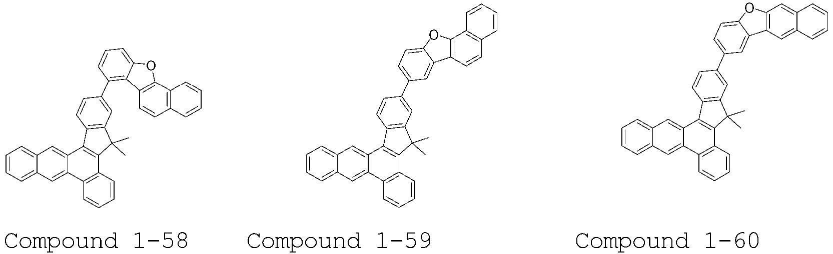

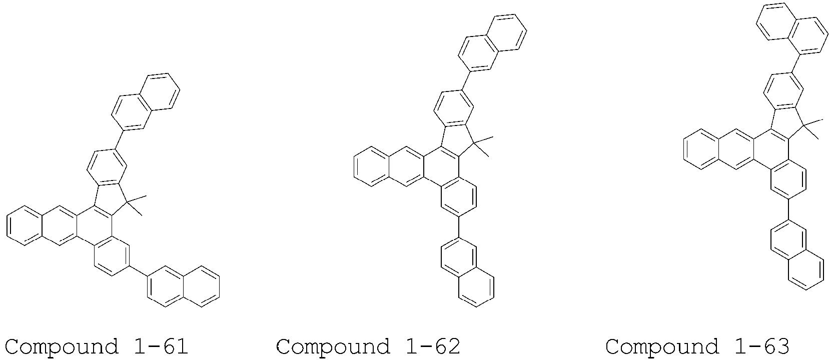

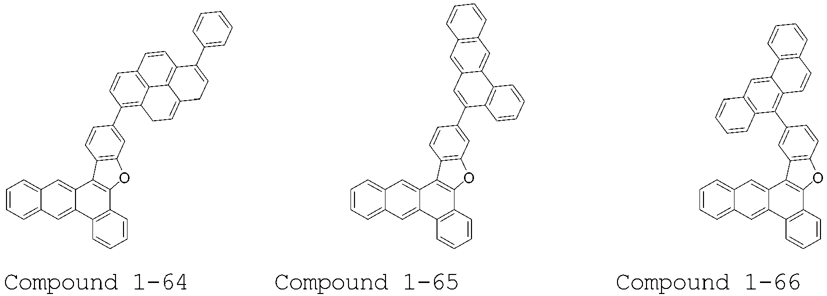

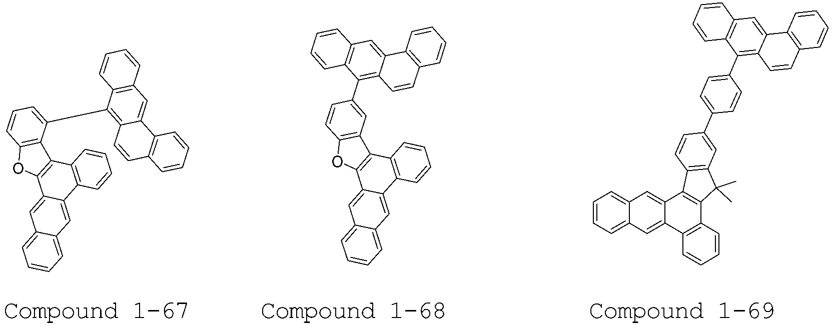

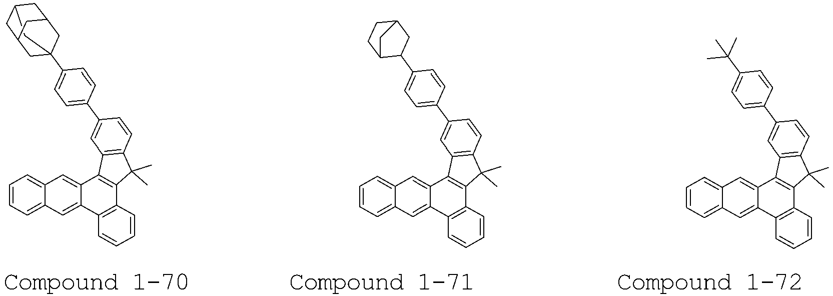

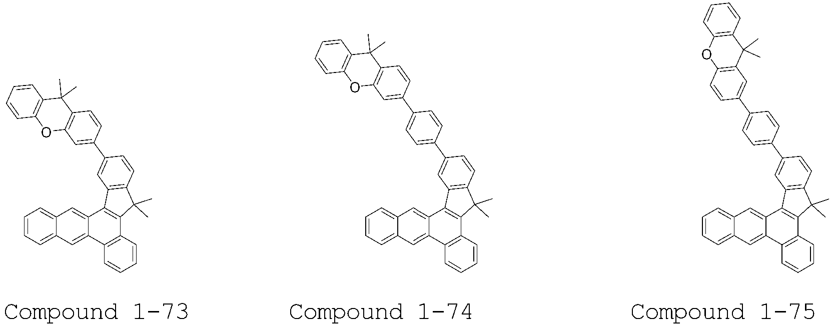

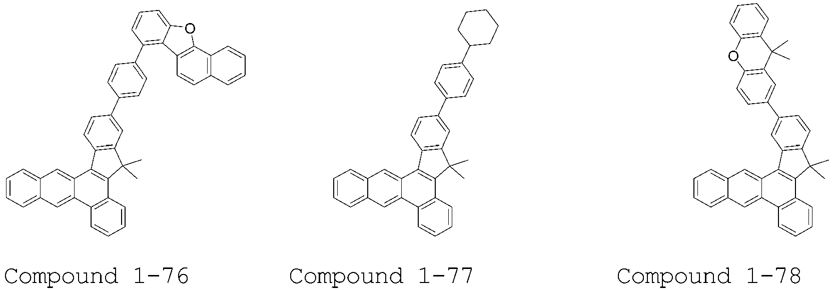

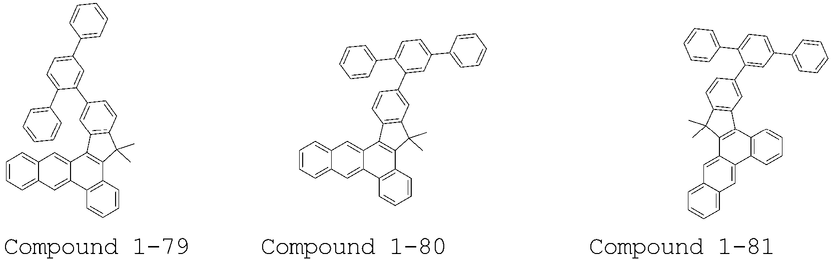

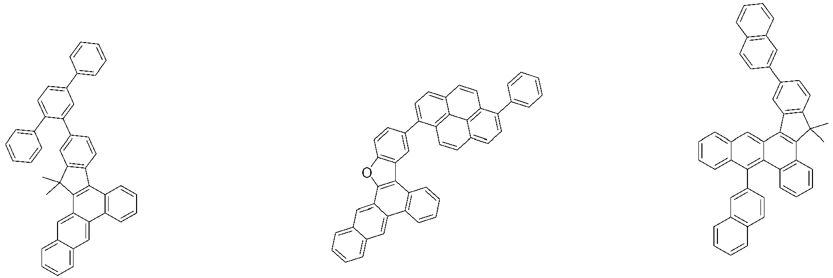

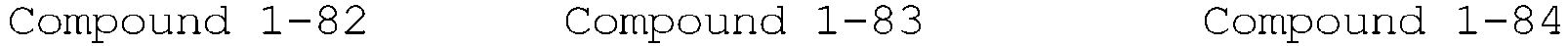

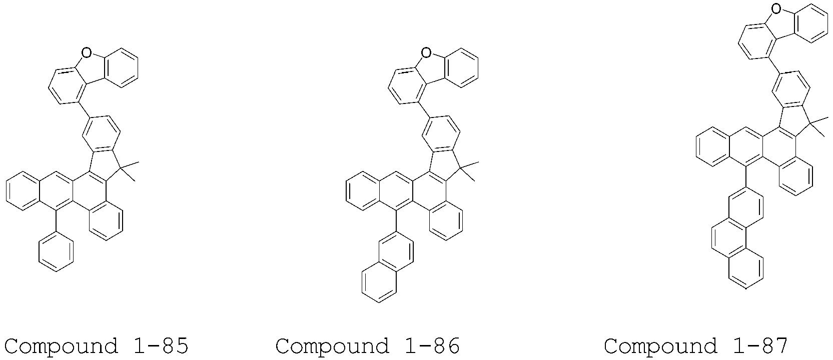

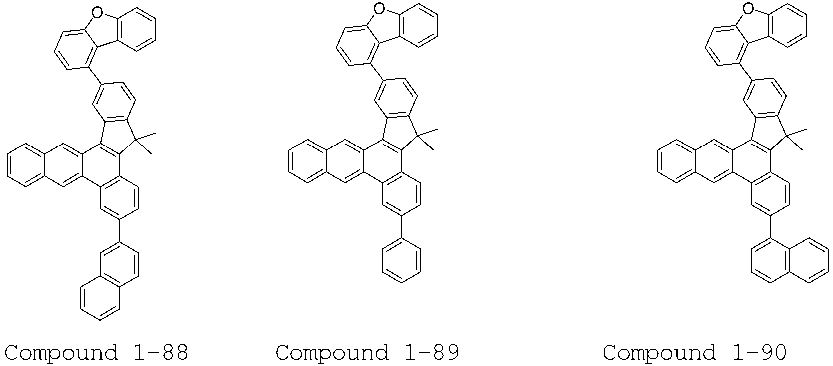

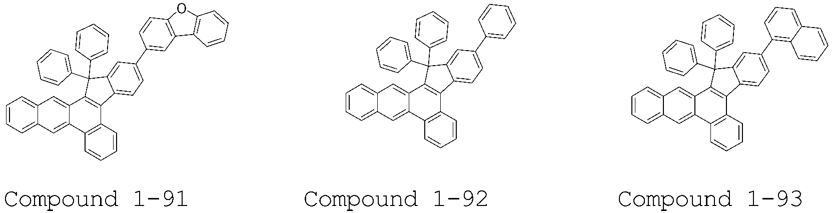

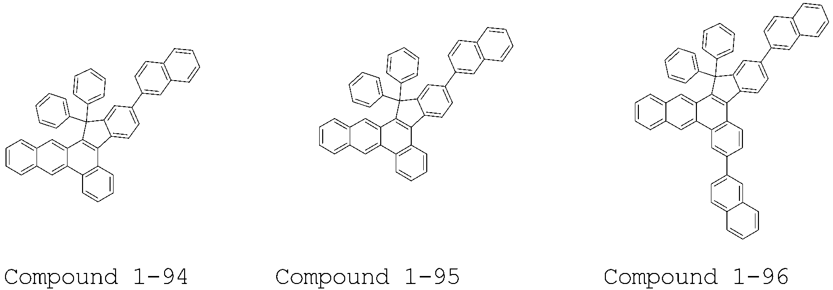

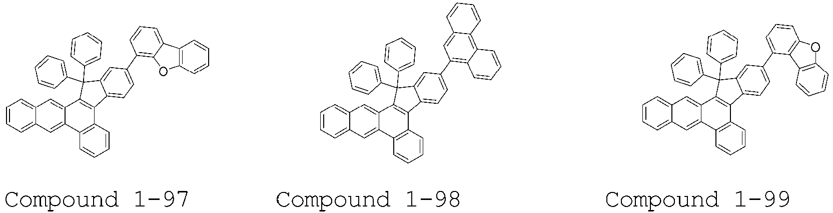

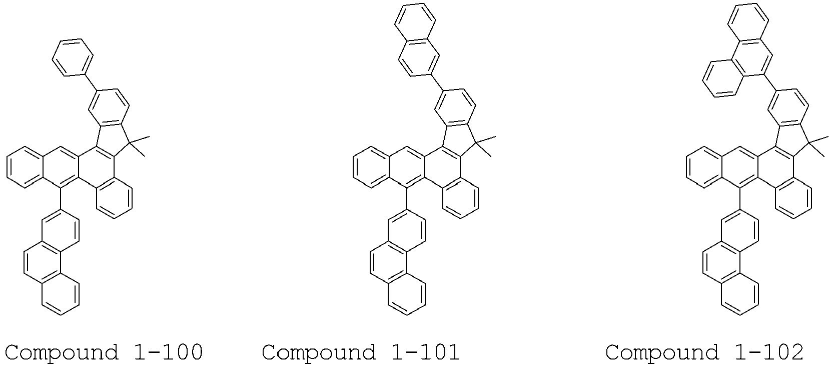

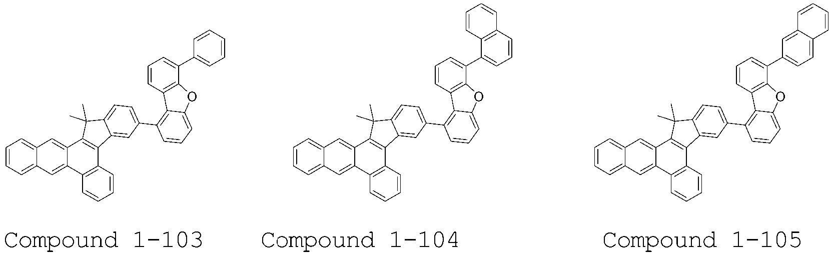

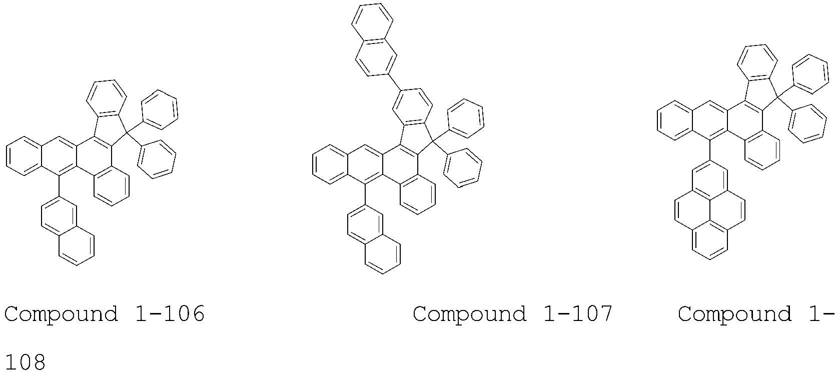

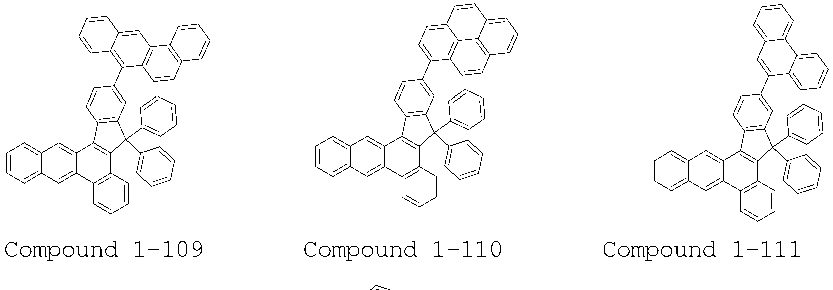

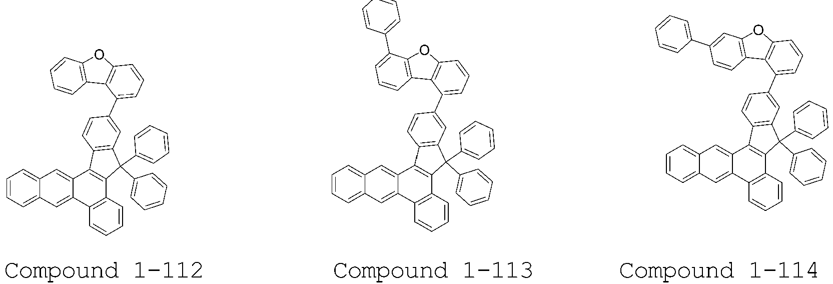

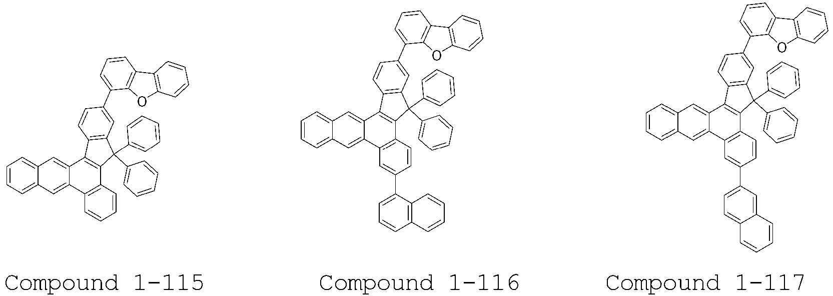

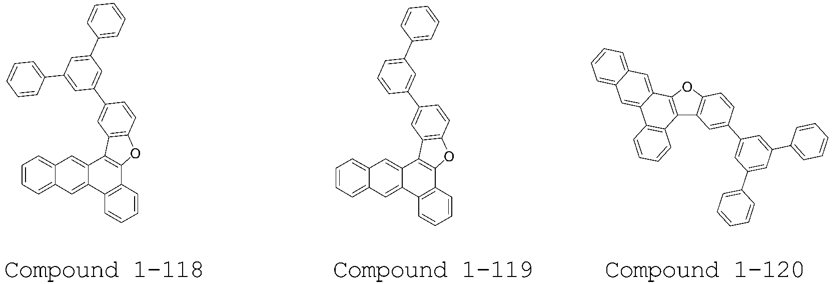

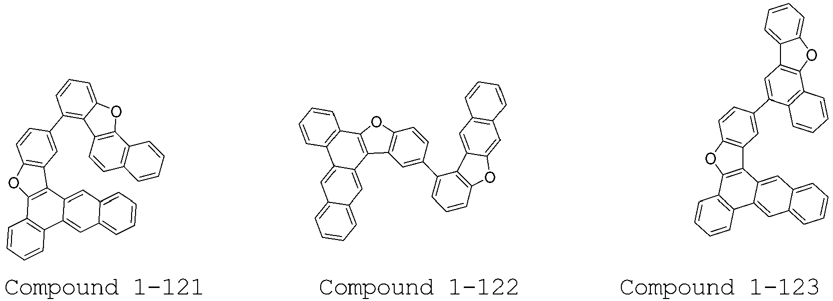

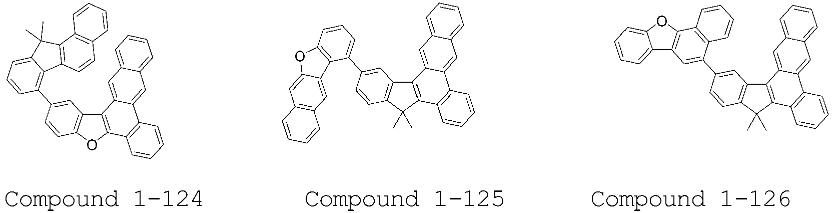

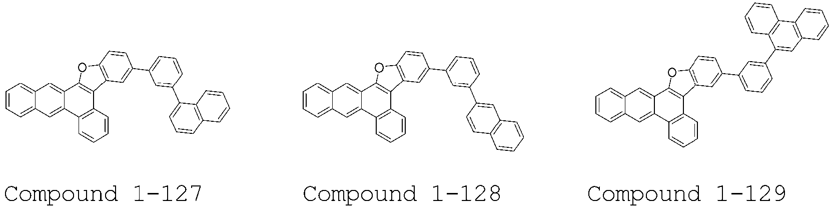

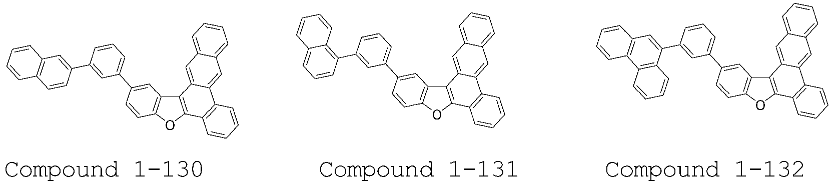

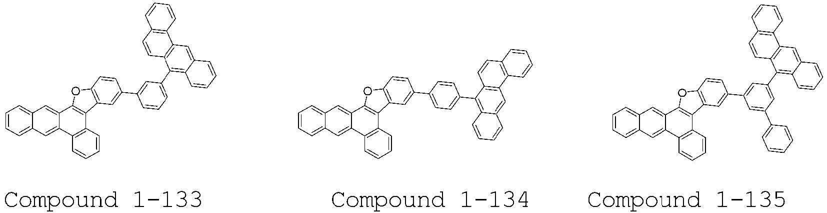

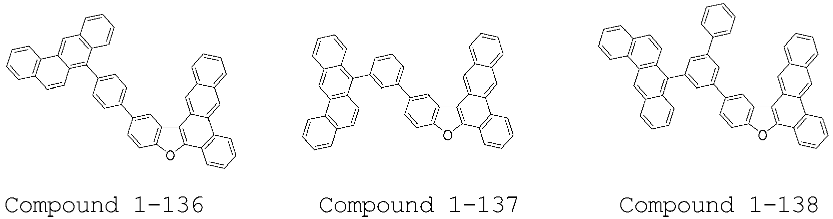

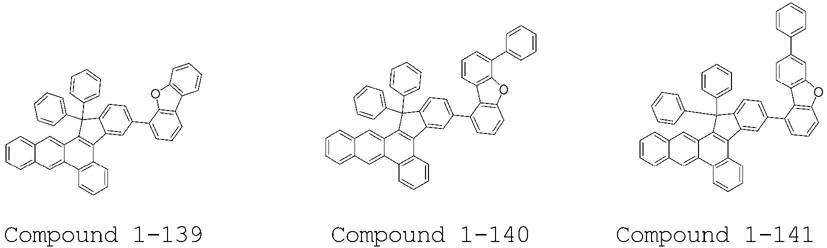

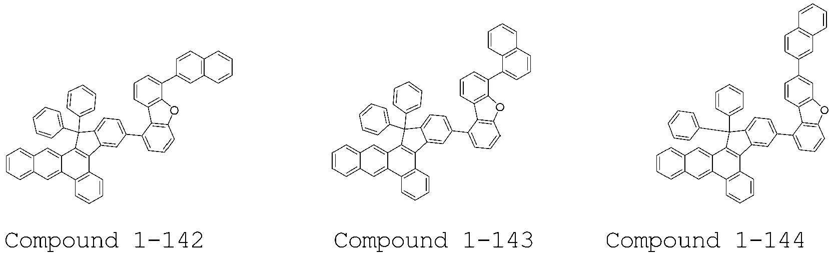

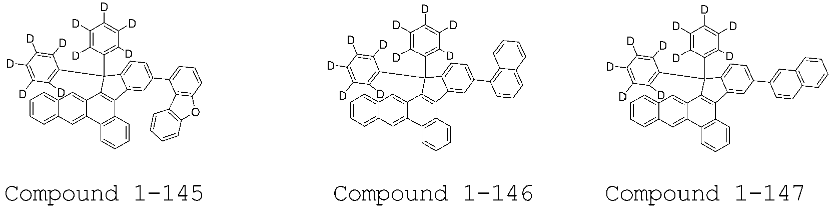

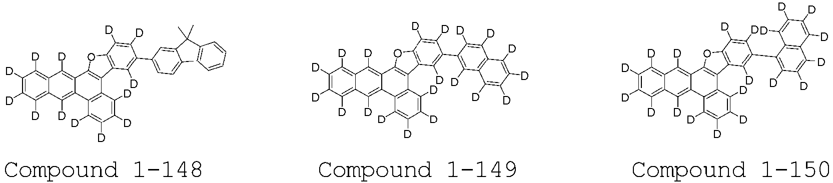

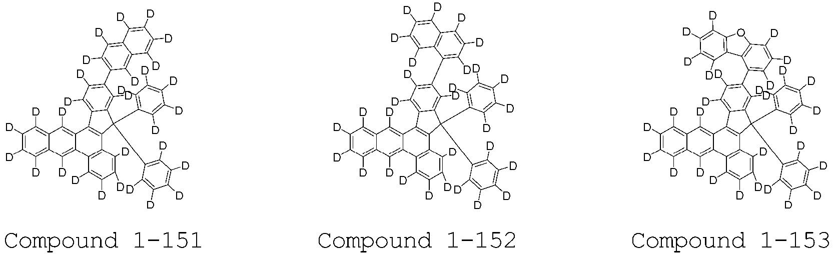

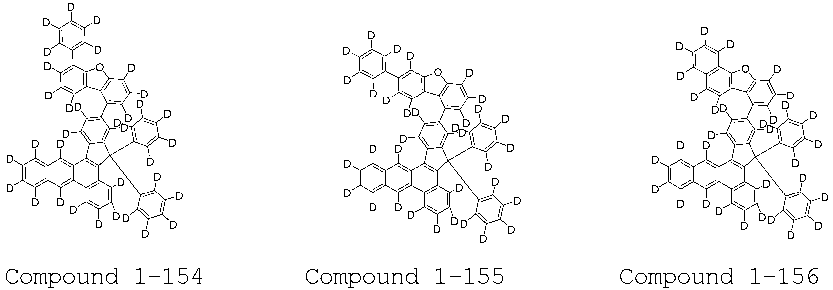

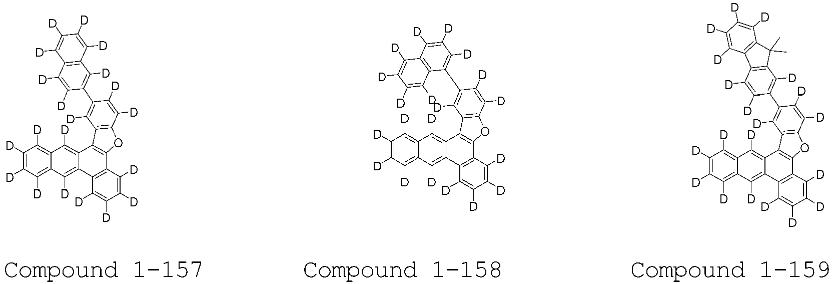

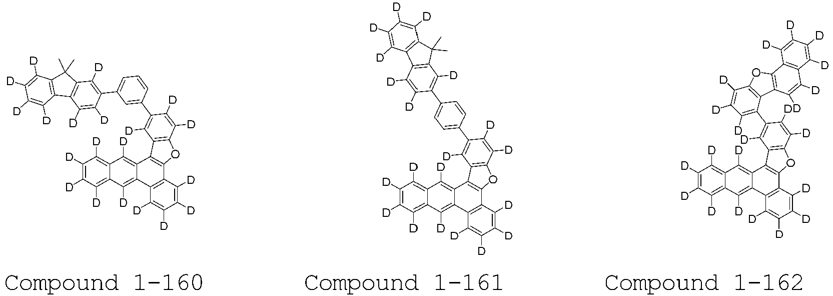

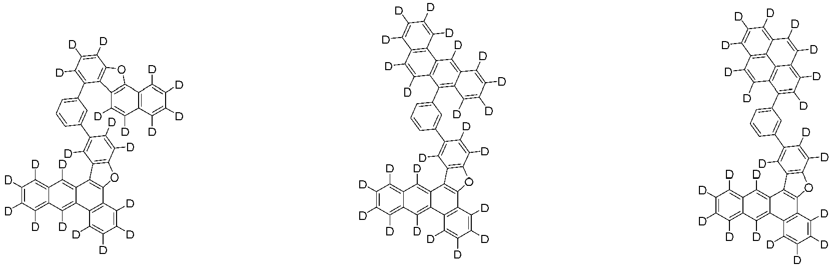

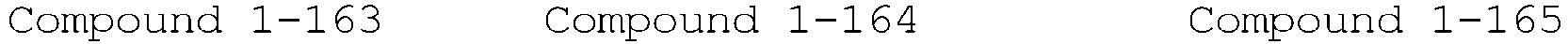

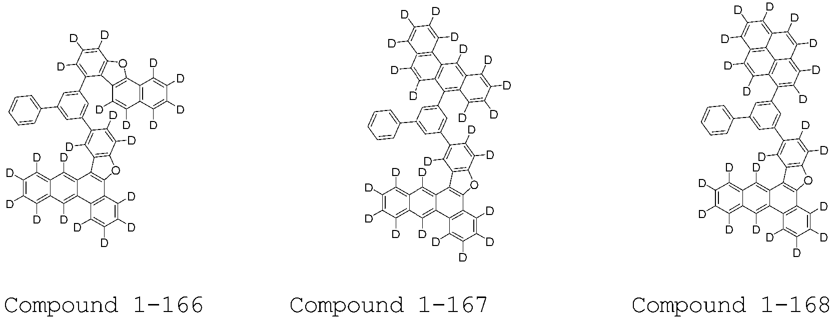

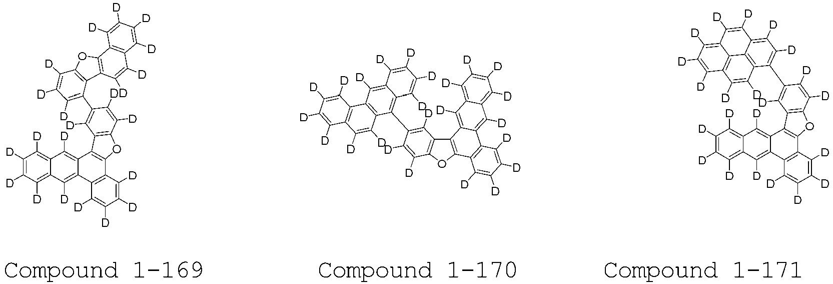

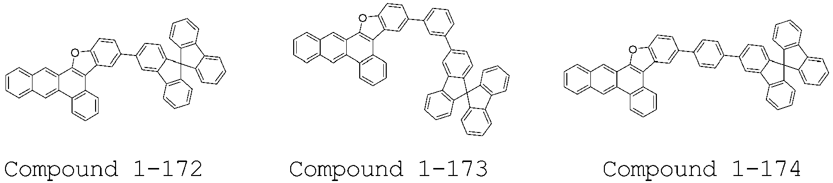

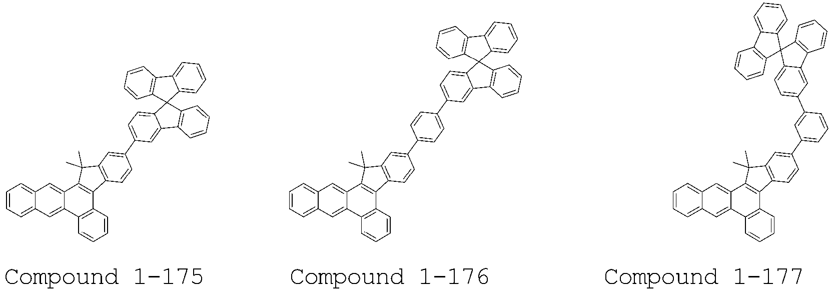

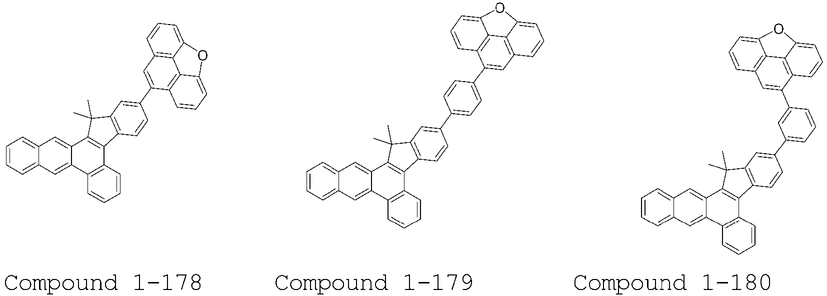

In addition, the present disclosure provides an organic light-emitting diode, including: a first electrode; a second electrode facing the first electrode; a first emission layer disposed between the first electrode and the second electrode and containing a first host and a first dopant; and a second emission layer containing a second host and a second dopant, wherein at least one of the first host and the second host include at least one of fused cyclic compounds represented by any one of Chemical Formulas 1 to 4 as described above. The organic light-emitting diode can exhibit low-voltage driving, high efficiency, and long lifespan characteristics.

Herein, the organic light-emitting layer according to the present disclosure may further include at least one layer selected from a hole injection layer, a hole transport layer, a functional layer capable of both hole injection and hole transport, an electron blocking layer, an electron transport layer, an electron injection layer, a functional layer capable of both electron injection and electron transport, and a hole blocking layer.

Throughout the description of the present disclosure, the phrase "(organic layer or emission layer) includes at least one organic compound" may be construed to mean that "(organic layer or emission layer) may include a single organic compound species or two or more different species of organic compounds falling within the scope of the present disclosure".

The compound represented by any one of Chemical Formulas 1 to 4 may be used as a host in the first or second emission layer. That is, the organic light-emitting diode according to the present disclosure may include one or more of the compounds represented by Chemical Formulas 1 to 4 as a host material in the emission layer.

Of the first and second hosts within the organic light-emitting diode according to the present disclosure, one may include a compound represented by any one of Chemical Formulas 1 to 4 while the other may include an anthracene compound represented by the following Chemical Formula 5. For instance, the first host within the organic light-emitting diode according to the present disclosure may include a compound represented by any one of Chemical Formulas 1 to 4 while the second host may include an anthracene compound represented by Chemical Formula 5, and *vice versa*. 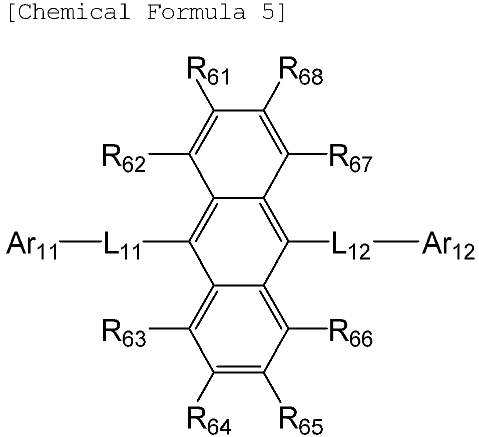 wherein,
the substituents R₆₁ to R₆₈, which are identical to or different from each other, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁₁ and L₁₂, which are identical to or different from each other, are each independently any one linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
Ar₁₁ and Ar₁₂, which are identical to or different from each other, are each independently any one selected from a substituted or unsubstituted aryl of 6 to 30 carbon atoms, a substituted or unsubstituted heteroaryl of 3 to 30 carbon atoms, a substituted or unsubstituted cycloalkyl of 3 to 30 carbon atoms, and a substituted or unsubstituted aliphatic hydrocarbon ring-fused aryl of 8 to 24 carbon atoms,
with the hydrogen atoms bonded to the carbon atoms within Chemical Formula 5 being substitutable with 0 to 60 deuterium atoms or tritium atoms,
wherein the term "substituted" in the expression "substituted or unsubstituted" used for the compound of Chemical Formula 5 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with one or more hydrogen atoms within the substituents being substitutable with deuterium atoms or tritium atoms.

In an embodiment, R₆₁ to R₆₈ within Chemical Formula 5, which are identical to or different from each other, are each independently any one selected from a hydrogen atom or a deuterium atom.

In an embodiment, the compound represented by Chemical Formula 5 may contain at least one deuterium atom.

When the first host or the second host in the organic light-emitting diode of the present disclosure contains at least one of the anthracene compounds represented by Chemical Formula 5, Ar₁₂ in the anthracene compound represented by Chemical Formula 5 may be a substituent represented by the following Structural Formula 1: 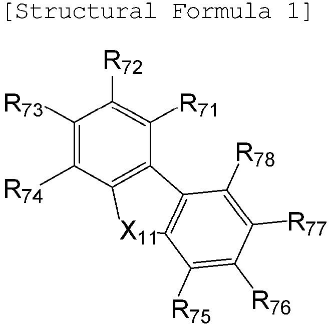 wherein,
X₁₁ is O or S,
one of R₇₁ to R₇₈ is a single bond to L₁₂ in Chemical Formula 5, with preference for R₇₁ or R₇₂ being a single bond to L₁₂,
R₇₁ to R₇₈ exclusive of the single bond to L₁₂ are identical to or different from each other and are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
adjacent substituents of R₇₁ to R₇₈ may be linked to each other to additionally form a mono- or polycyclic aliphatic or aromatic ring,
wherein the term "substituted" in the expression "substituted or unsubstituted" used for the compound of Structural Formula 1 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with one or more hydrogen atoms within the substituents being substitutable with deuterium atoms or tritium atoms.

In a more preferred embodiment, X₁₁ in Structural Formula 1 may be O.

In a more preferred embodiment, R₇₁ to R₇₈ in Structural formula 1 may each be a hydrogen atom, a deuterium atom, a substituted or unsubstituted aryl of 6 to 30 carbon atoms, a substituted or unsubstituted heteroaryl of 3 to 30 carbon atoms, and a substituted or unsubstituted aliphatic hydrocarbon ring-fused aryl of 8 to 24 carbon atoms.

In a more preferred embodiment, R₇₁ to R₇₈ in Structural Formula 1, which are identical to or different from each other, may each be independently any one selected from a hydrogen atom, a deuterium atom, and a substituted or unsubstituted aryl of 6 to 18 carbon atoms.

In the organic light-emitting diode according to an embodiment of the present disclosure, one of the first host and the second host may include a compound represented by any one of Chemical Formulas 1 to 4, and the other may include two or more anthracene compounds represented by Chemical Formula 5, wherein the anthracene compounds represented by Chemical Formula 5 may be different from each other. For example, in the organic light-emitting diode according to the present disclosure, the first host may include a compound represented by any one of Chemical Formulas 1 to 4, and the second host may include two or more anthracene compounds represented by Chemical Formula 5. In this regard, the compounds represented by Chemical Formula 5 may include two or more different anthracene compounds, and vice versa.

In an embodiment, when one of the first host and the second host in the organic light-emitting diode according to the present disclosure includes a compound represented by any one of Chemical Formulas 1 to 4 and the other includes two or more anthracene compounds represented by Chemical Formula 5, at least one of the anthracene compounds represented by Chemical Formula 5 may have Ar₁₂ as a substituted or unsubstituted aryl group of 6 to 30 carbon atoms, preferably a substituted or unsubstituted aryl group of 6 to 18 carbon atoms, and at least one of the additionally included anthracene compounds represented by Chemical Formula 5 may have Ar₁₂ as a substituted or unsubstituted heteroaryl group of 2 to 30 carbon atoms, preferably a substituted or unsubstituted heteroaryl group of 3 to 18 carbon atoms.

In an embodiment, more preferably, when one of the first host and the second host in the organic light-emitting diode according to the present disclosure includes a compound represented by any one of Chemical Formulas 1 to 4 and the other includes two or more anthracene compounds represented by Chemical Formula 5, at least one of the anthracene compounds represented by Chemical Formula 5 may have Ar₁₂ as a substituted or unsubstituted aryl group of 6 to 30 carbon atoms, and at least one of the additionally included anthracene compounds represented by Chemical Formula 5 may have Ar₁₂ as a substituent represented by Structural Formula 1.

In the organic light-emitting diode according to an embodiment of the present disclosure, the first emission layer may include, as a first host, at least one compound represented by any one of Chemical Formulas 1 to 4, and the second emission layer may include, as a second host, at least one compound represented by Chemical Formula 5.

When one of the first host and the second host in the organic light-emitting diode according to an embodiment of the present disclosure includes a compound represented by any one of Chemical Formulas 1 to 4 and the other includes an anthracene compound represented by Chemical Formula 5, the first emission layer may include, as the first host, a compound represented by any one of Chemical Formulas 1 to 4; and a host compound different from the compound represented by any one of Chemical Formulas 1 to 4 in a ratio of 1:9 to 9:1, and more preferably in a ratio of 3:7 to 7:3.

The first emission layer in the organic light-emitting diode according to an embodiment of the present disclosure may further include, as a first host, a compound represented by any one selected from Chemical Formulas 1 to 4; and a host compound different from the compound represented by any one selected from Chemical Formulas 1 to 4. In this regard, the first host used in the first emission layer may include a compound represented by any one selected from Chemical Formulas 1 to 4 and a compound different from the compound represented by any one selected from Chemical Formulas 1 to 4 in a ratio of 1:9 to 9:1, and more preferably in a ratio of 3:7 to 7:3.

In this case, when the first host is composed of two or more compounds, they may be used by being mixed and deposited, co-deposited, or stacked. That is, the first host according to the present disclosure may further include at least one additional compound different from the compound represented by any one of Chemical Formulas 1 to 4, in addition to one compound represented by any one of Chemical Formulas 1 to 4, such that two or more first host compounds may be mixed and deposited, co-deposited, or stacked. In the case of stacking, a compound different from the compound represented by any one of Chemical Formulas 1 to 4 may be stacked above or below a layer including the compound represented by any one of Chemical Formulas 1 to 4.

Herein, the case where a host compound different from the compound represented by any one of Chemical Formulas 1 to 4 is mixed and deposited means that the compounds are mixed in a single deposition source and deposited by sublimation or vaporization. In addition, in the case of co-deposition, it means that two or more hosts are deposited together by being respectively sublimed or vaporized from a plurality of deposition sources.

That is, the emission layer may be formed by depositing a plurality of host materials from different deposition sources (co-deposition) to form a thin film, or by mixing the plurality of hosts in advance and depositing the mixed hosts, or by forming the emission layer by a stacking method of the hosts.

Herein, the host compound different from the compound represented by any one of Chemical Formulas 1 to 4 may be a compound having a structural formula different from that of any one of Chemical Formulas 1 to 4. Alternatively, as long as it is different from the compound already used (i.e., a compound already used as the compound represented by any one of Chemical Formulas 1 to 4), a compound having a structure included within any one of Chemical Formulas 1 to 4 but different from the already used compound may also be used.

Examples of the compound having a structural formula different from any one of Chemical Formulas 1 to 4 used in the first emission layer may include conventionally known anthracene-based compounds, benz[a]anthracene-based compounds, phenanthrene-based compounds, pyrene-based compounds, fluorene-based compounds, spirofluorene-based compounds, chrysene-based compounds, carbazole-based compounds, and biphenyl-based compounds. Herein, the anthracene-based compound refers to a compound including at least one anthracene group in the molecule, preferably one to three anthracene groups. Likewise, the benz[a]anthracene-based compound, phenanthrene-based compound, pyrene-based compound, fluorene-based compound, spirofluorene-based compound, chrysene-based compound, carbazole-based compound, and biphenyl-based compound refer to compounds including at least one corresponding group in the molecule, preferably one to three corresponding groups.

When one of the first host and the second host in the organic light-emitting diode according to an embodiment of the present disclosure includes at least one compound represented by any one of Chemical Formulas 1 to 4 and the other includes an anthracene compound represented by Chemical Formula 5, the other one of the first host and the second host may include two or more different anthracene compounds represented by Chemical Formula 5. Preferably, the second emission layer in the organic light-emitting diode may include, as the second host, two different anthracene compounds represented by Chemical Formula 5 in a ratio of 1:9 to 9:1, preferably 3:7 to 7:3.

When the first emission layer in the organic light-emitting diode according to an embodiment of the present disclosure includes at least one compound represented by any one of Chemical Formulas 1 to 4 as a first host, and the second emission layer includes at least one compound represented by Chemical Formula 5 as a second host, the second emission layer may further include, as the second host, a host compound different from the compound represented by Chemical Formula 5 in addition to the compound represented by Chemical Formula 5.

In this regard, the second host used in the second emission layer may include the compound represented by Chemical Formula 5 and a host compound different from the compound represented by Chemical Formula 8 in a ratio of 1:9 to 9:1, more preferably 3:7 to 7:3.

When the second host is composed of two or more compounds, they may be mixed in a single deposition source and deposited by sublimation or vaporization, or may be respectively sublimed or vaporized from a plurality of deposition sources and co-deposited on the second emission layer.

Herein, the host compound different from the compound represented by Chemical Formula 5 may be a compound having a structural formula different from that of Chemical Formula 5. Alternatively, as long as it is different from the compound already used (i.e., a compound already used as the compound represented by Chemical Formula 5), a compound having a structure included in Chemical Formula 5 but different from the already used compound may also be used.

Further, examples of compounds having a structural formula different from that of Chemical Formula 5 used in the second emission layer may include conventionally known anthracene-based compounds, benz[a]anthracene-based compounds, phenanthrene-based compounds, pyrene-based compounds, fluorene-based compounds, spirofluorene-based compounds, chrysene-based compounds, carbazole-based compounds, and biphenyl-based compounds, and descriptions thereof are same as those described above.

In the organic light-emitting diode according to an embodiment of the present disclosure, the first host in the first emission layer may include a compound represented by any one of Chemical Formulas 1 to 4 used alone, and the second host in the second emission layer may include a compound represented by Chemical Formula 5 and a compound different therefrom in a ratio of 1:9 to 9:1, more preferably 3:7 to 7:3.

In the organic light-emitting diode according to an embodiment of the present disclosure, the first host in the first emission layer may include a compound represented by any one of Chemical Formulas 1 to 4 and a compound different therefrom in a ratio of 1:9 to 9:1, more preferably 3:7 to 7:3, and the second host in the second emission layer may include the compound represented by Chemical Formula 5 used alone.

In the organic light-emitting diode according to an embodiment of the present disclosure, the first host in the first emission layer may include a compound represented by any one of Chemical Formulas 1 to 4 and a host compound different therefrom in a ratio of 1:9 to 9:1, more preferably 3:7 to 7:3, and the second host in the second emission layer may include a compound represented by Chemical Formula 5 and a compound different therefrom in a ratio of 1:9 to 9:1, more preferably 3:7 to 7:3.

In an embodiment, concrete examples of the compound represented by Chemical Formula 5 include, but are not limited to:

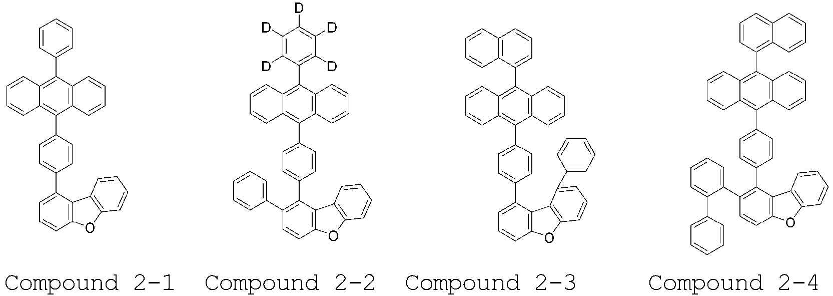

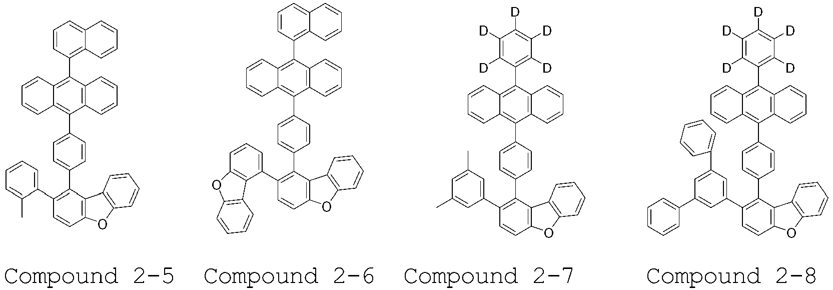

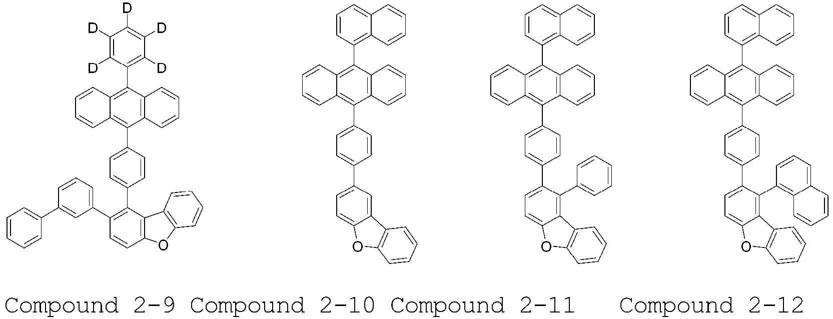

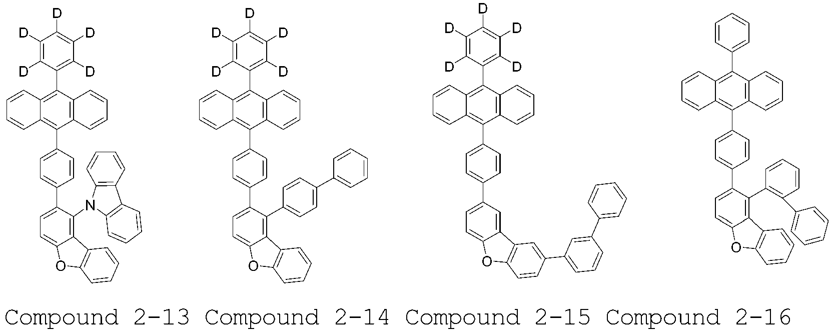

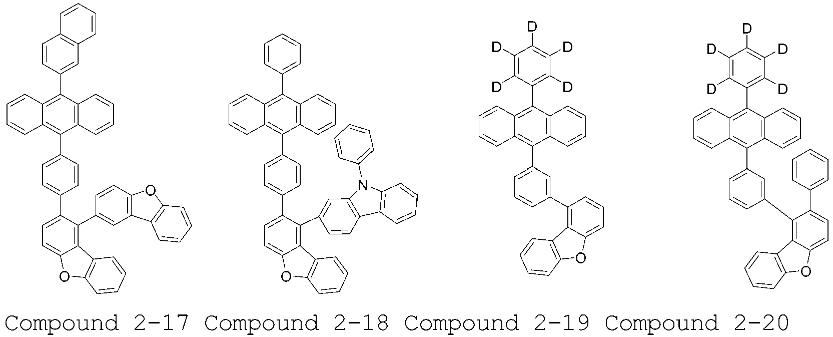

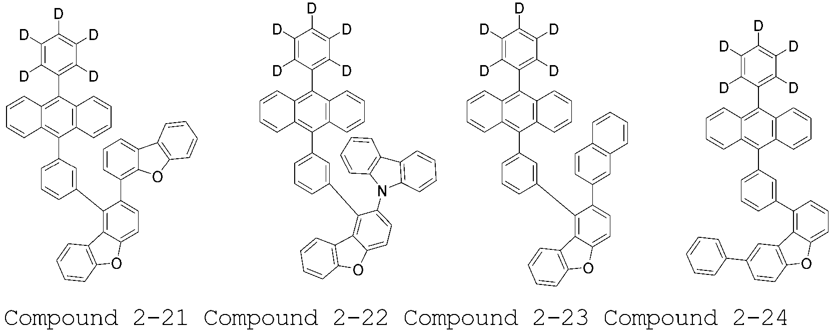

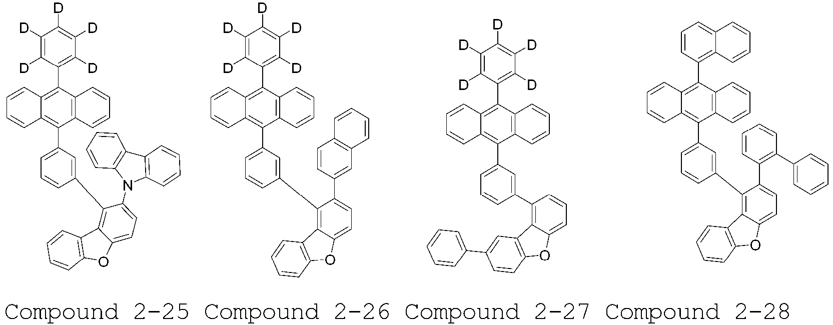

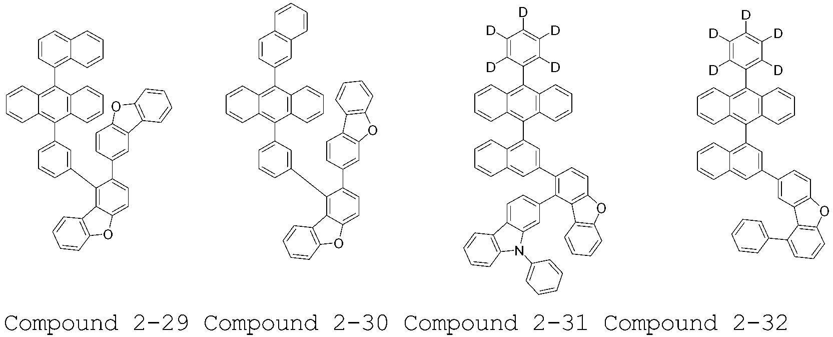

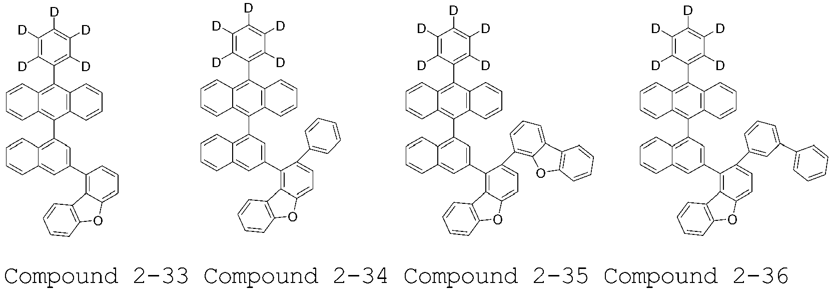

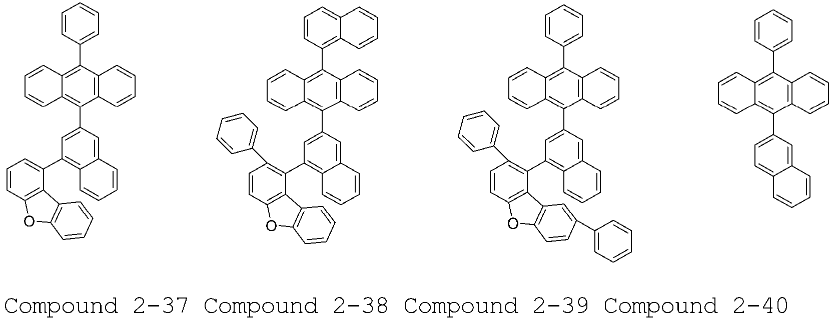

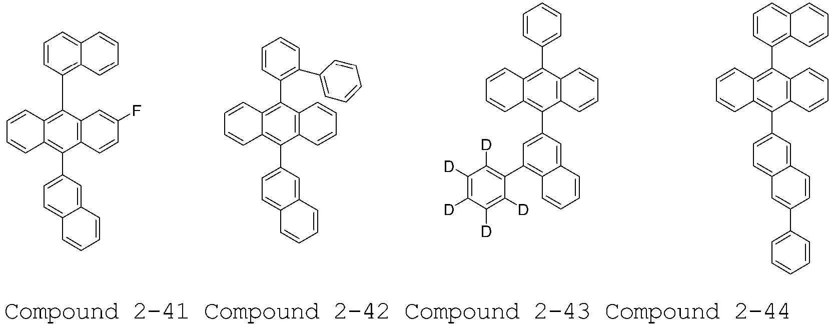

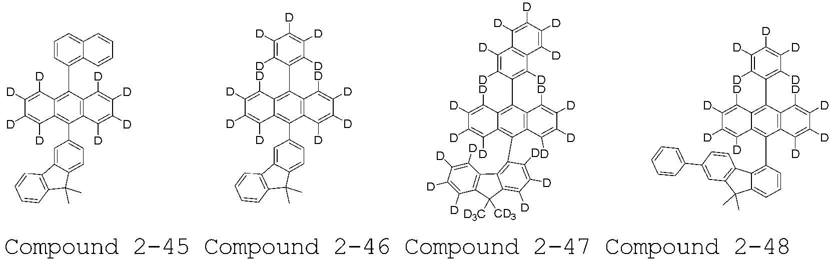

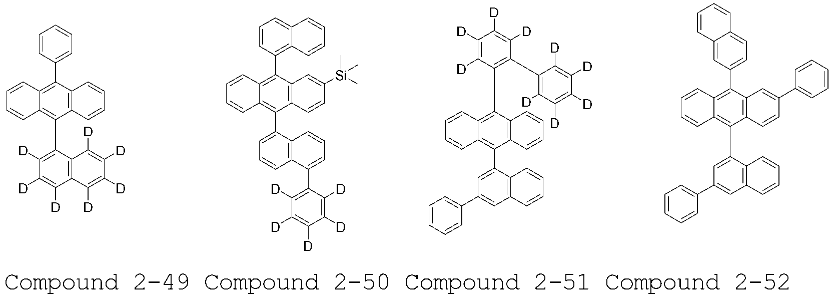

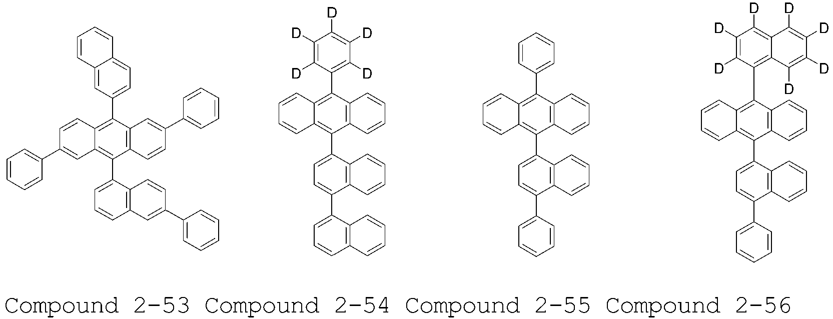

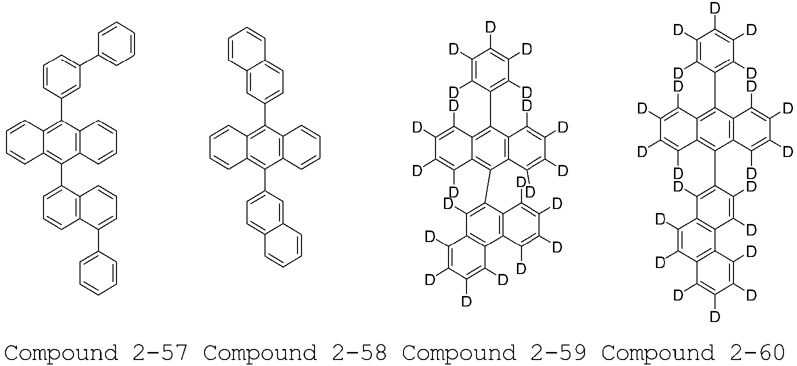

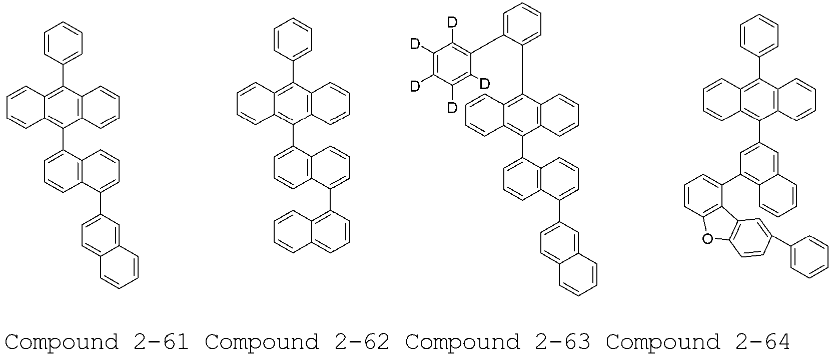

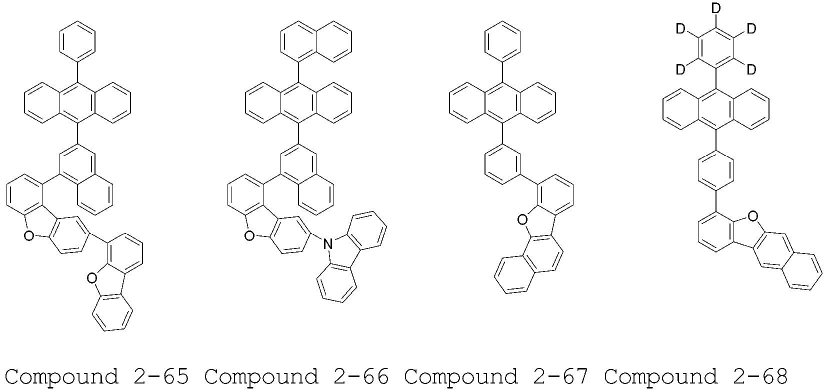

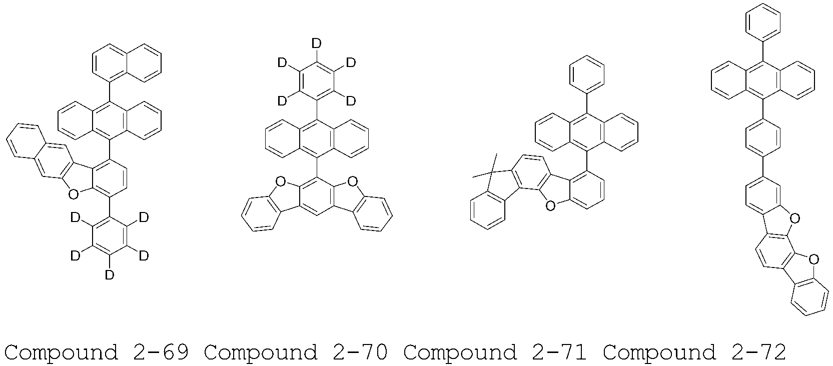

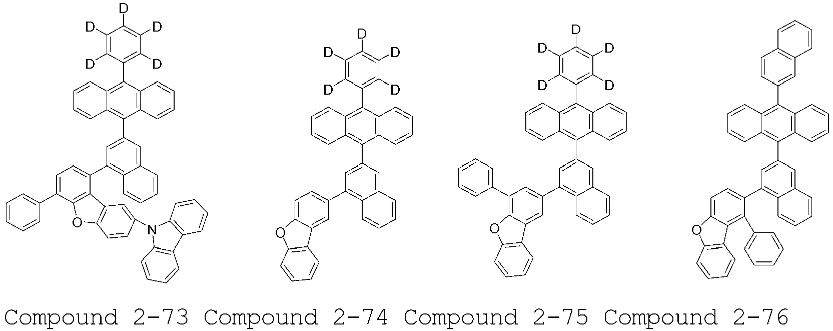

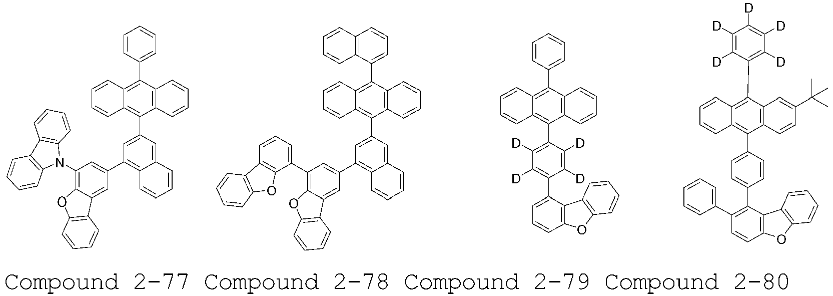

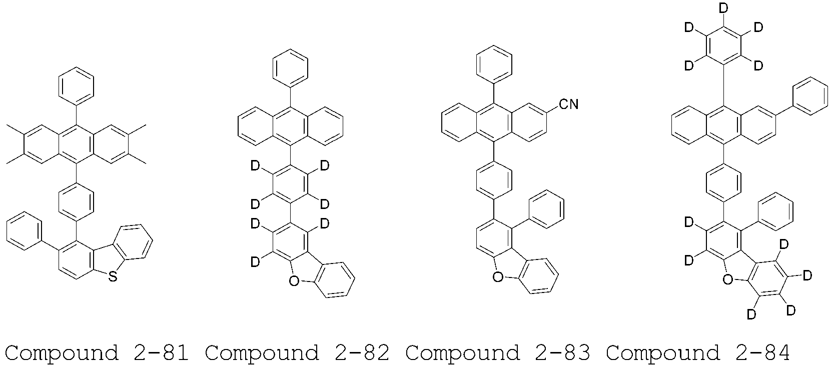

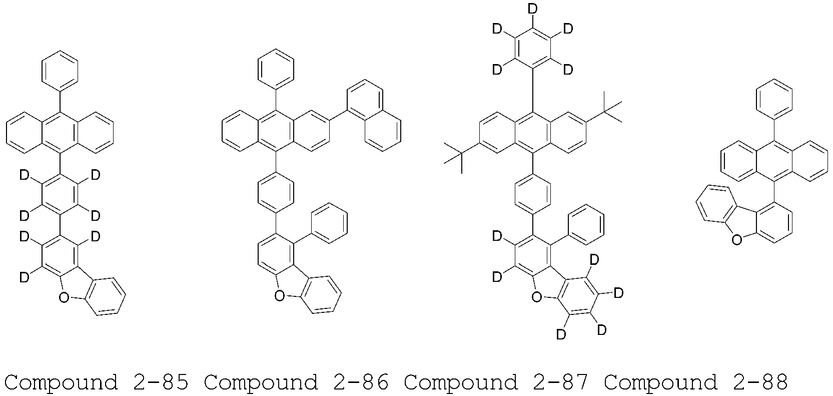

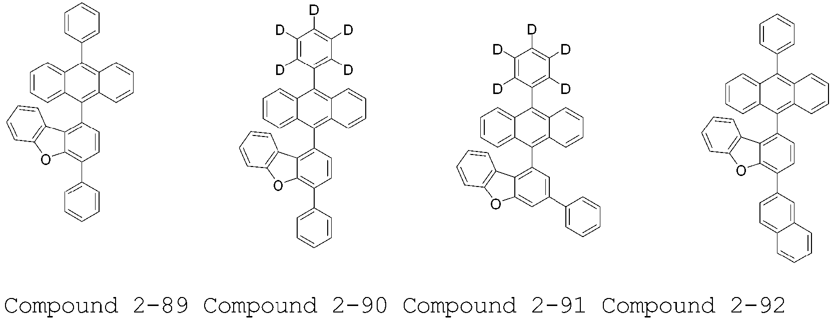

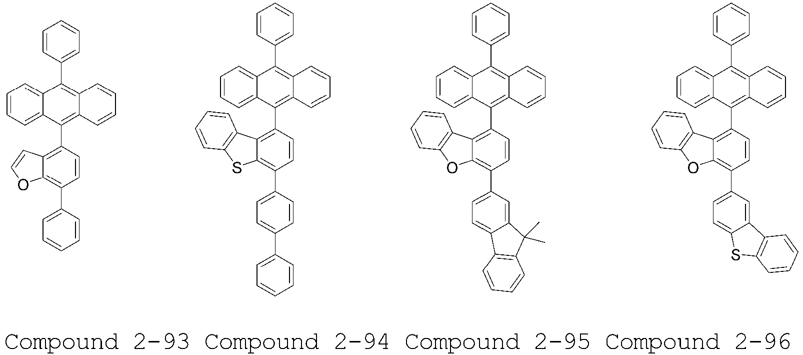

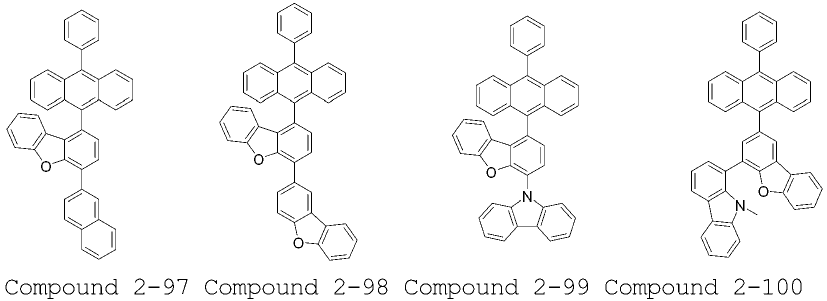

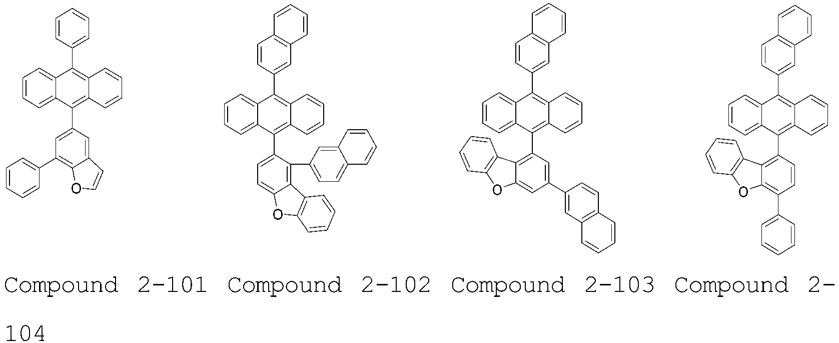

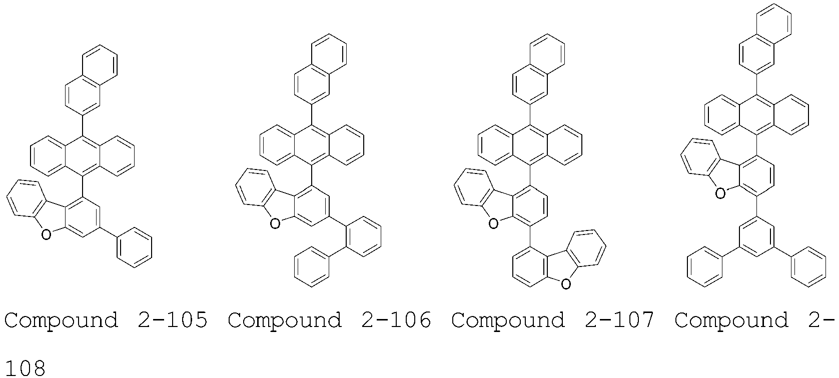

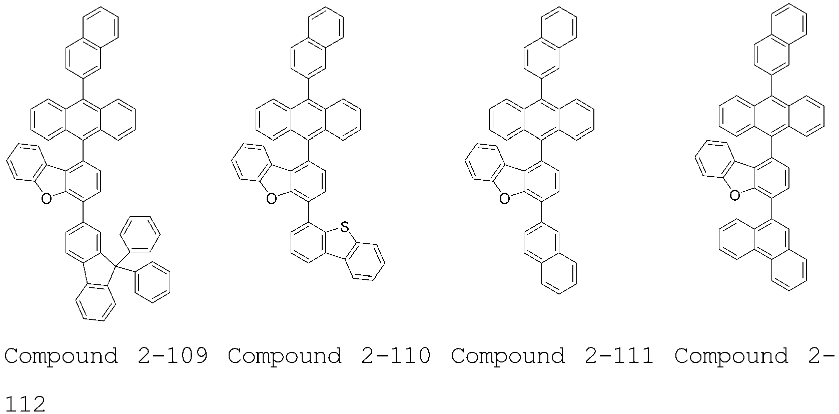

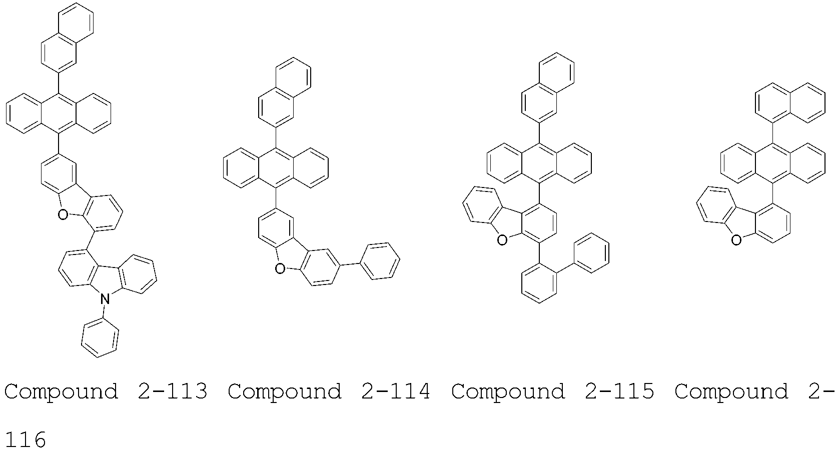

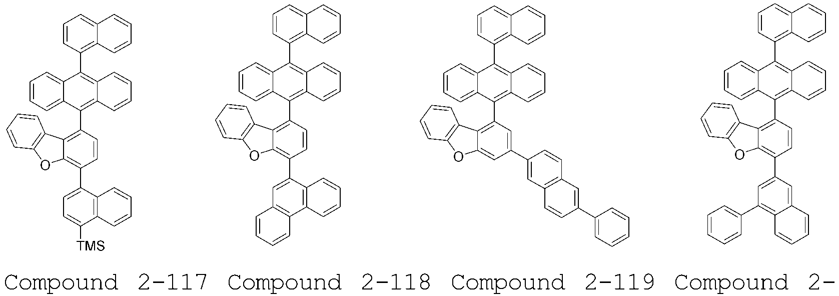

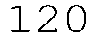

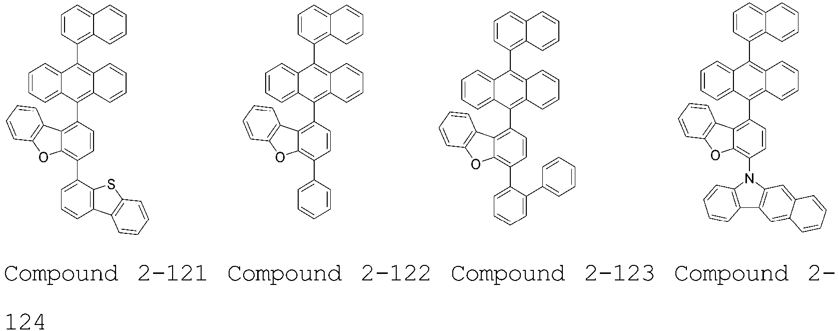

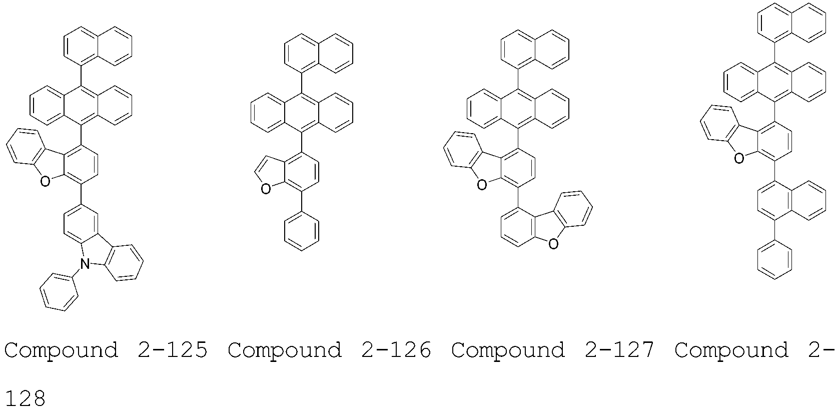

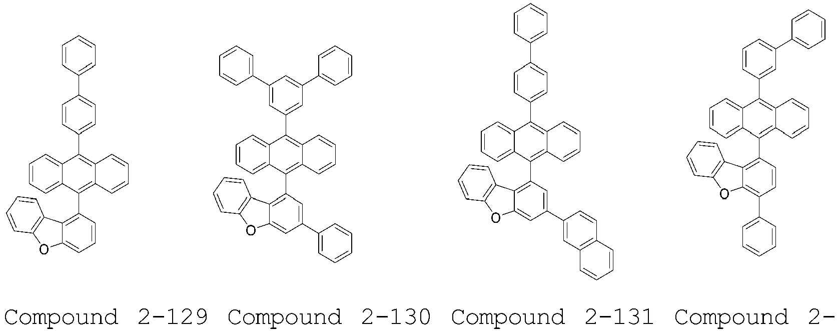

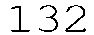

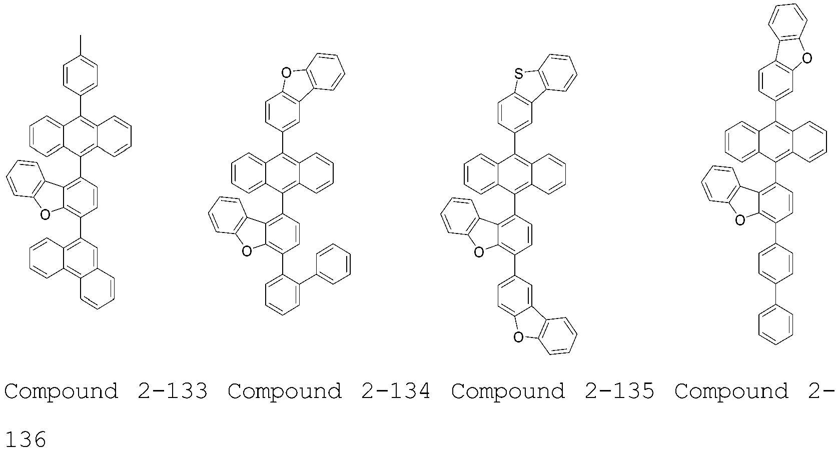

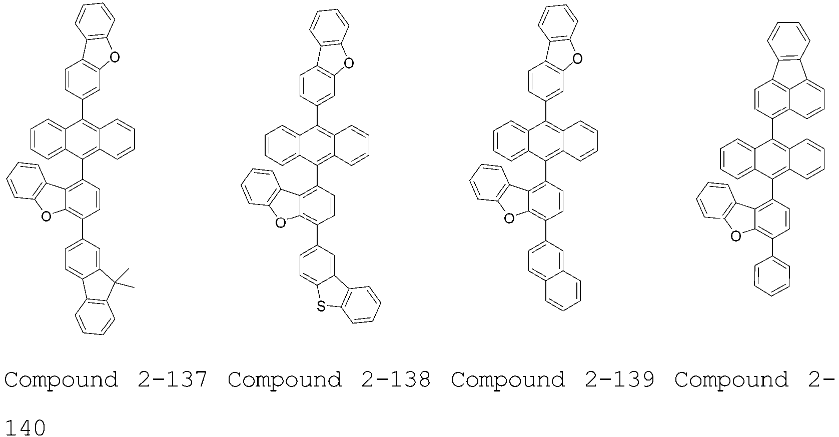

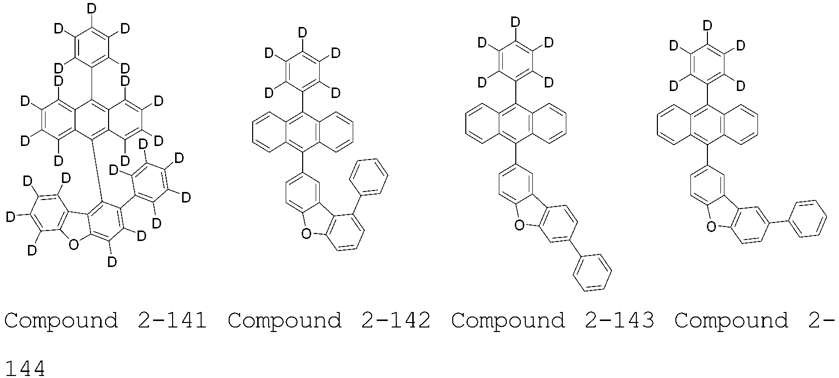

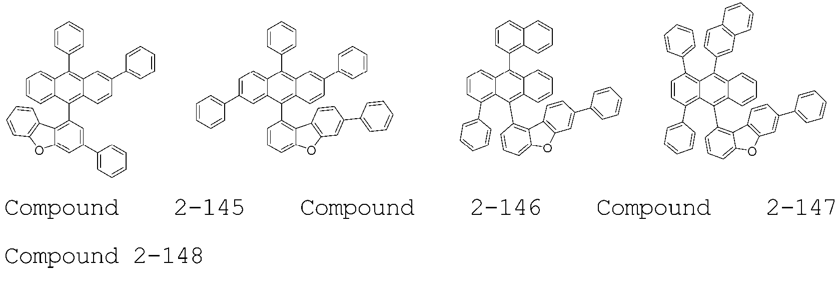

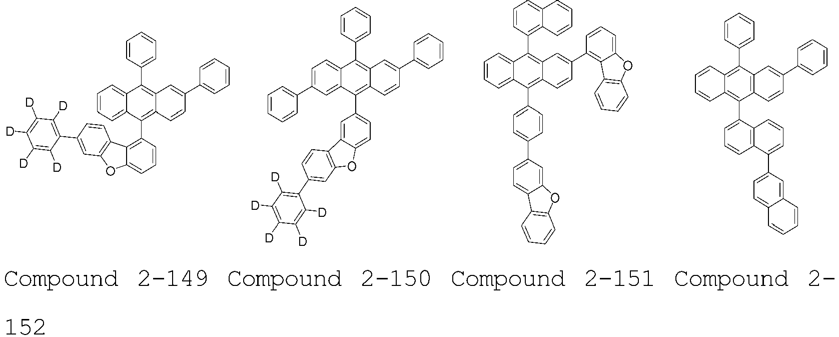

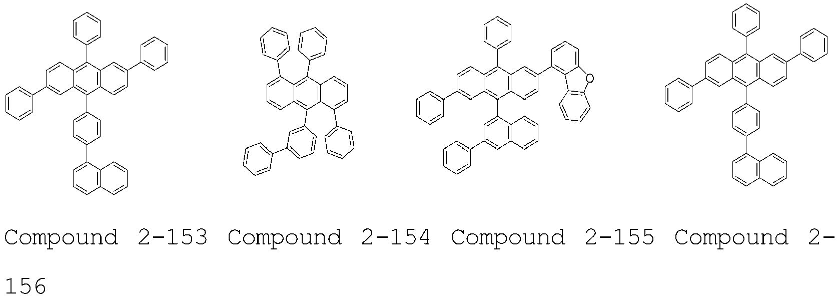

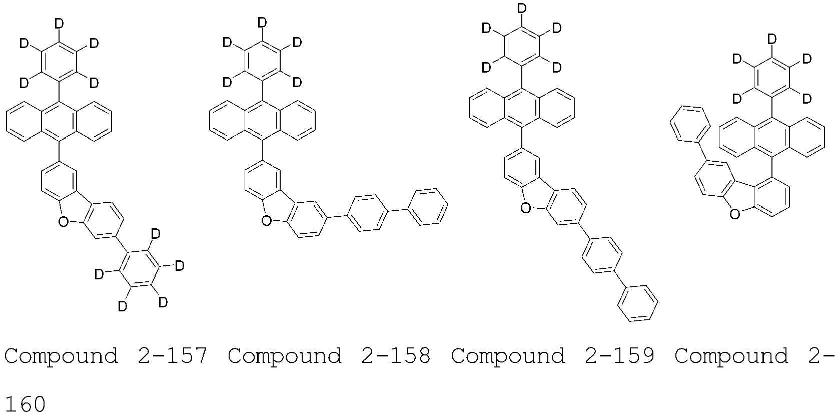

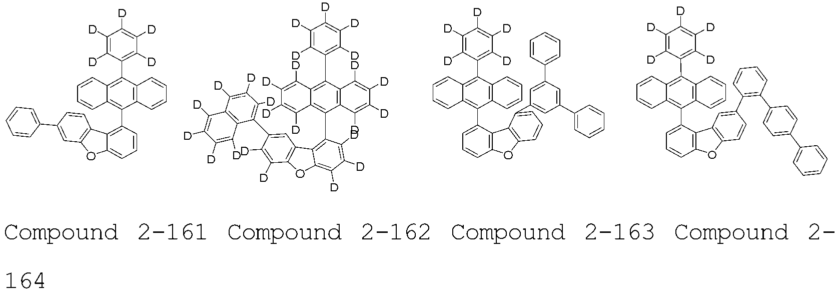

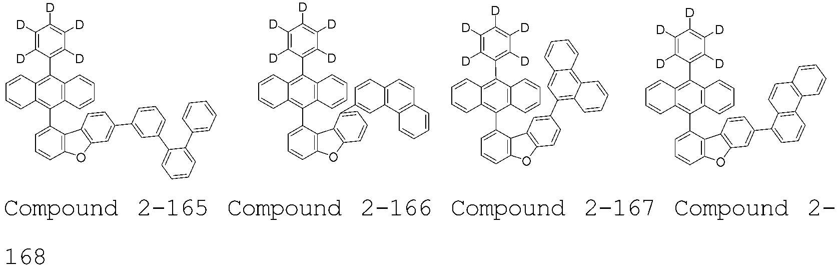

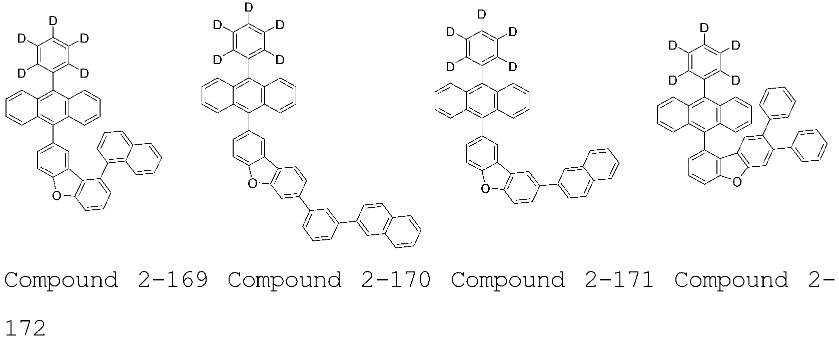

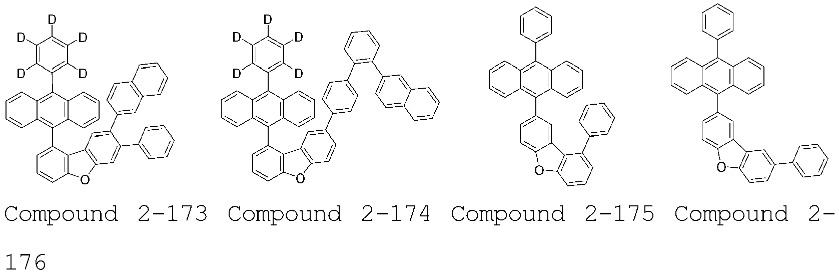

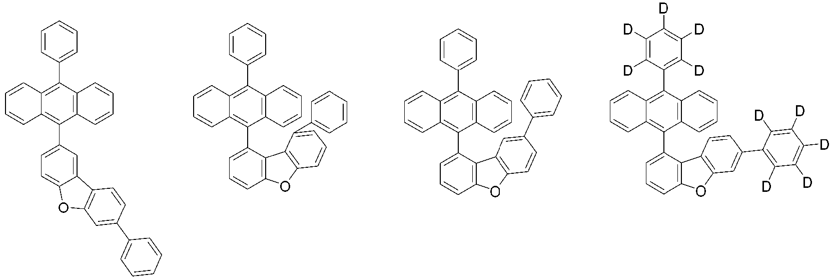

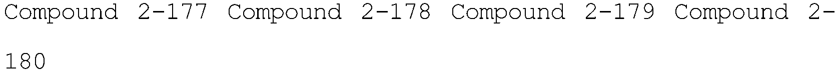

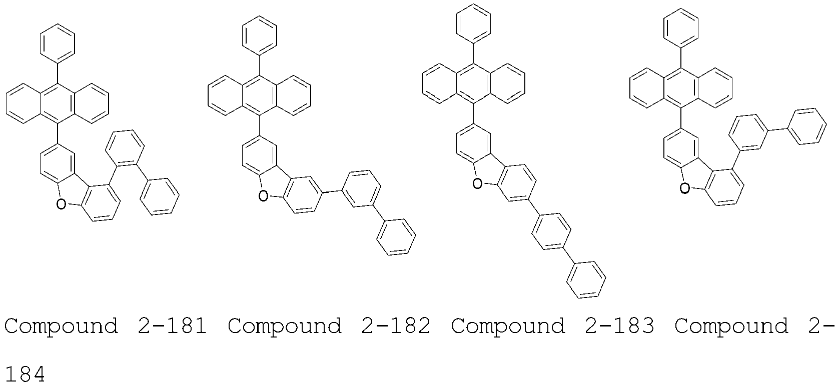

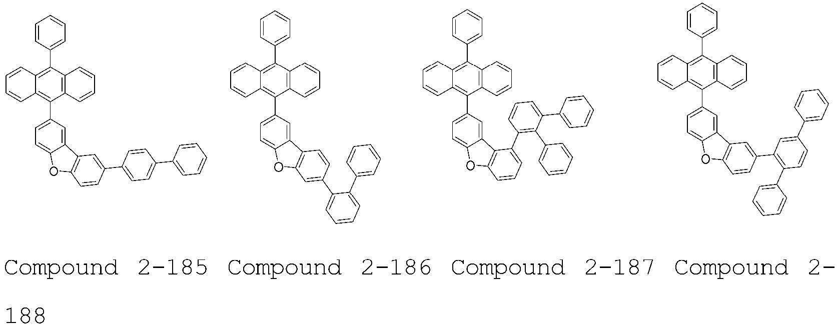

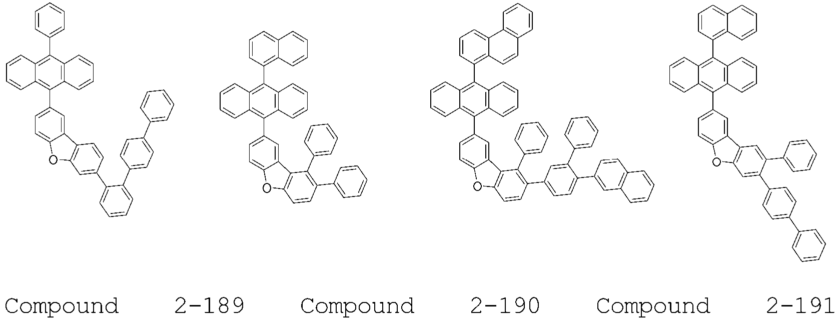

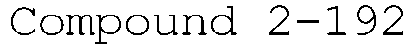

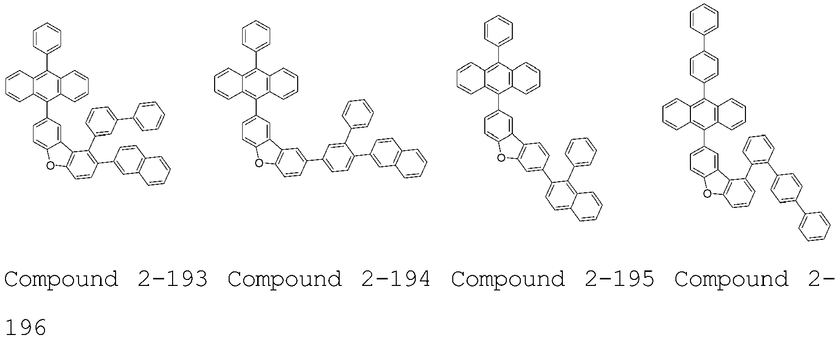

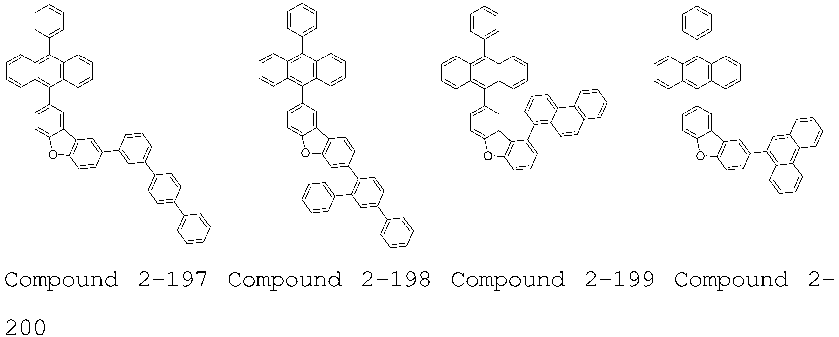

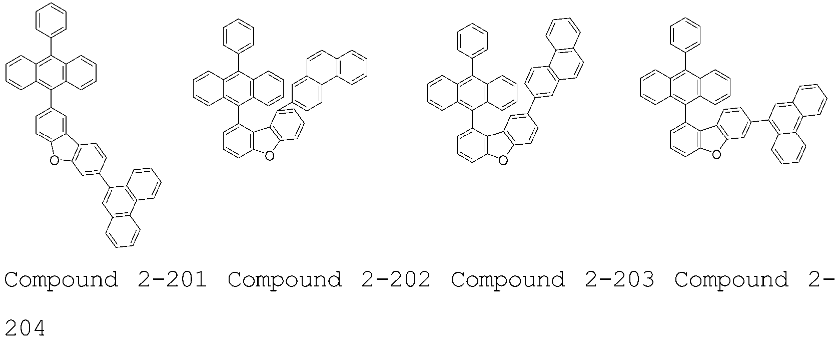

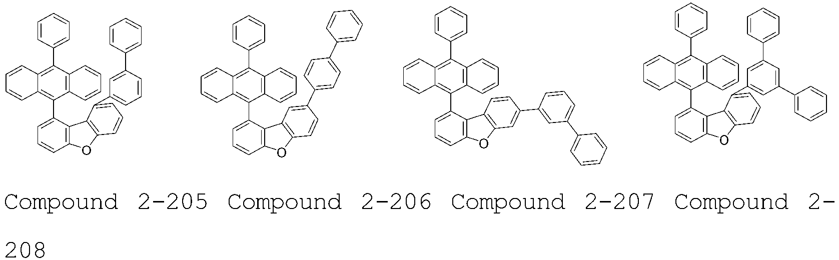

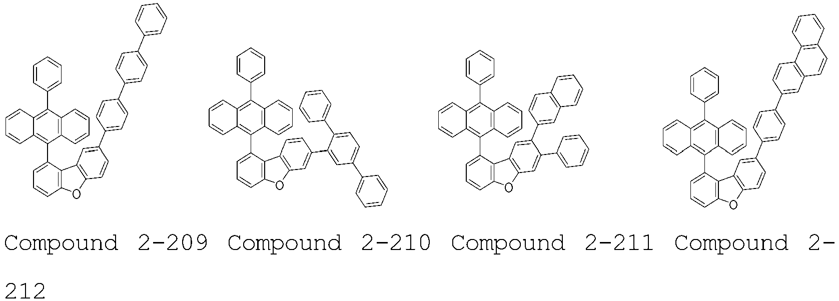

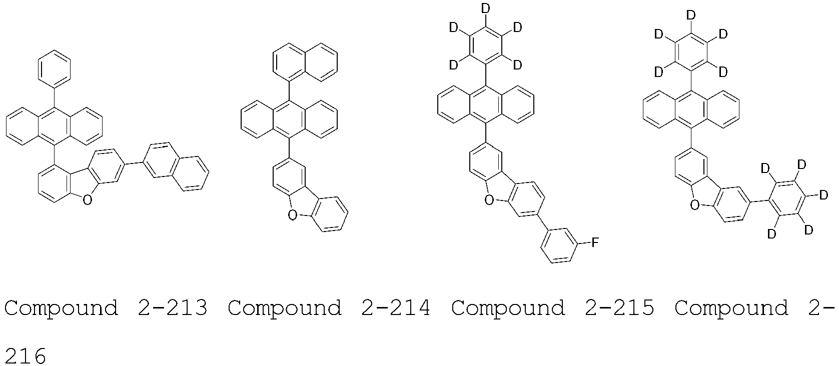

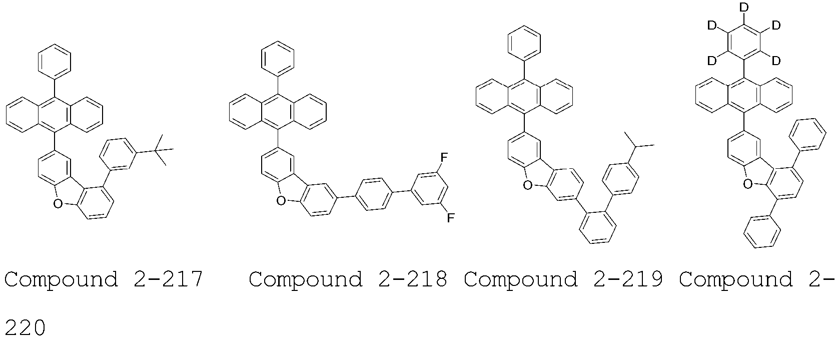

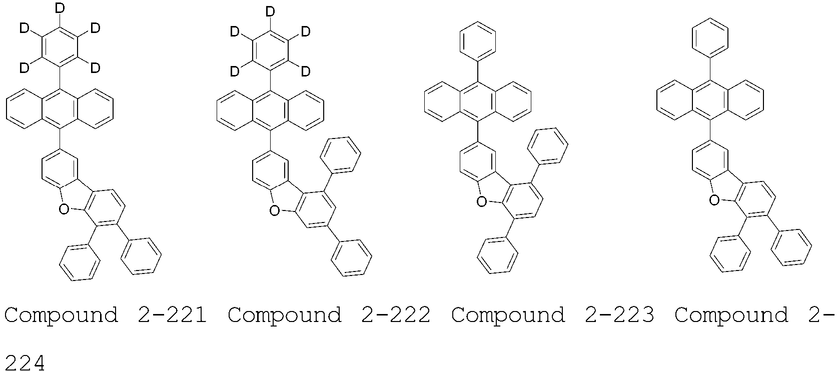

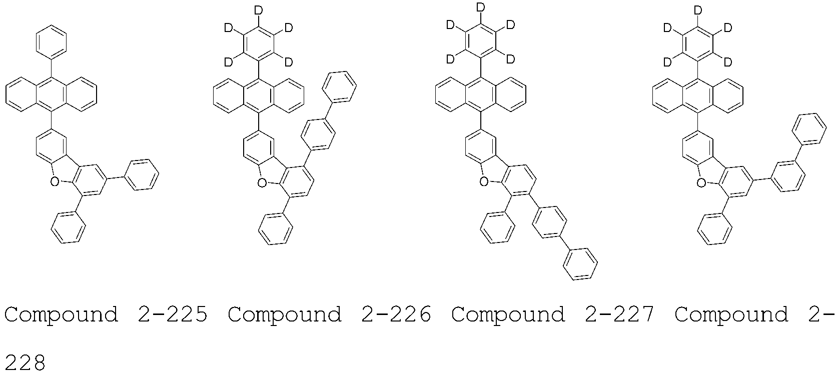

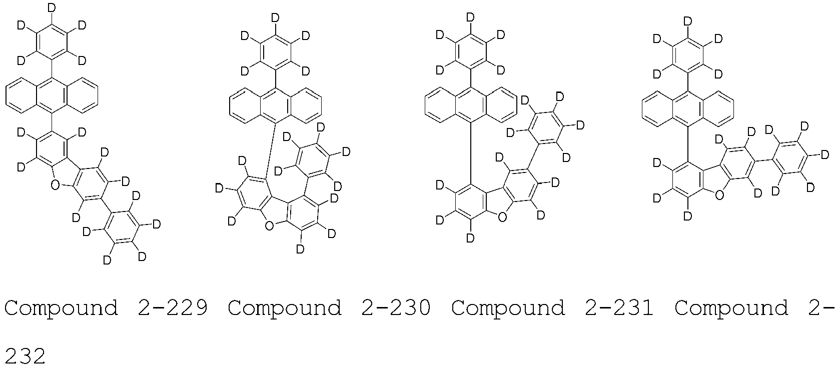

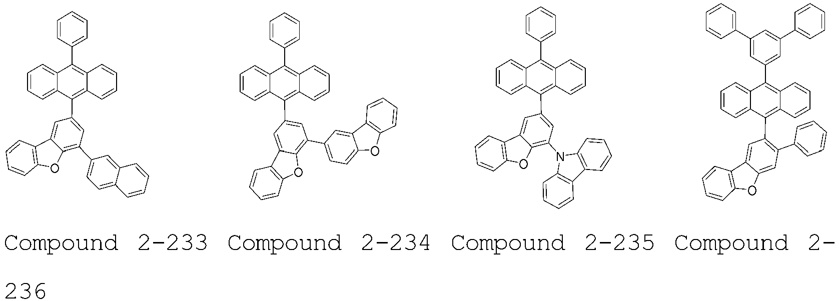

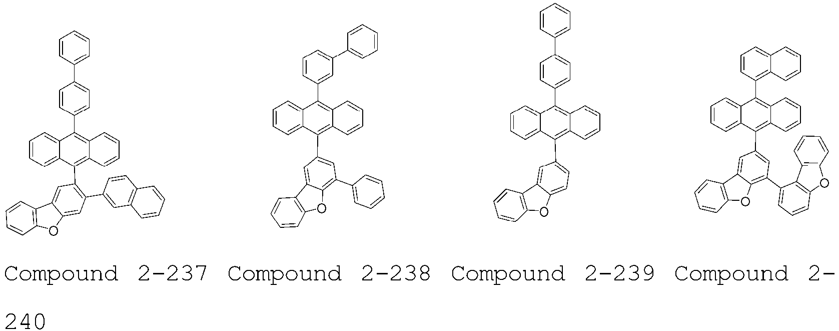

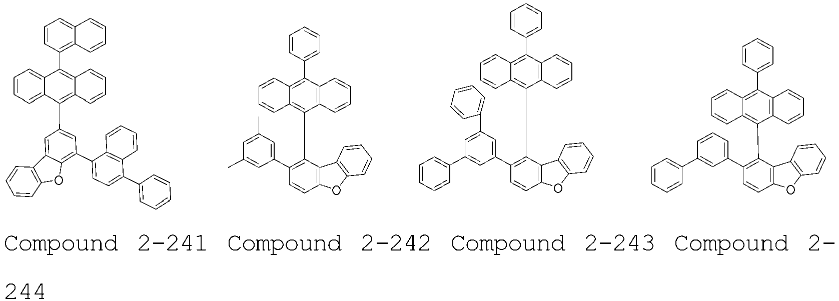

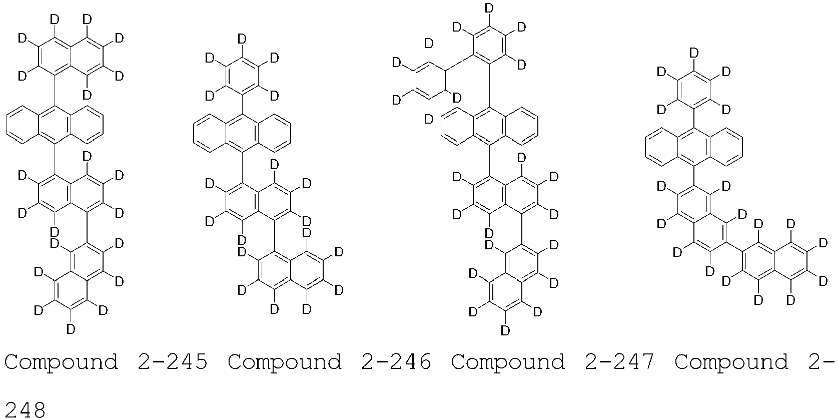

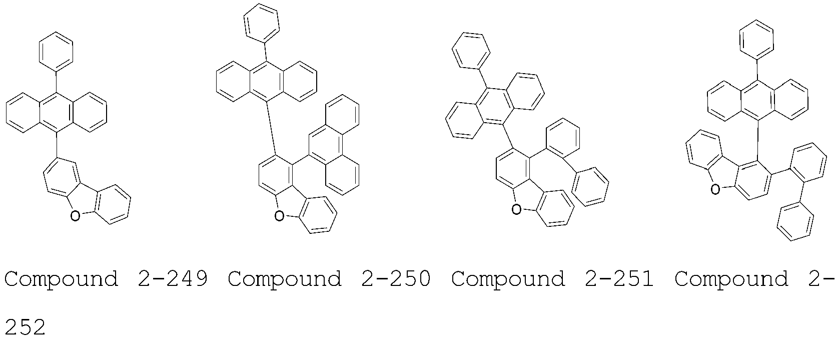

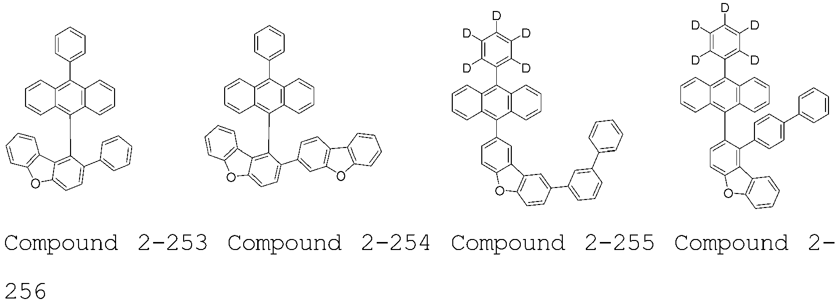

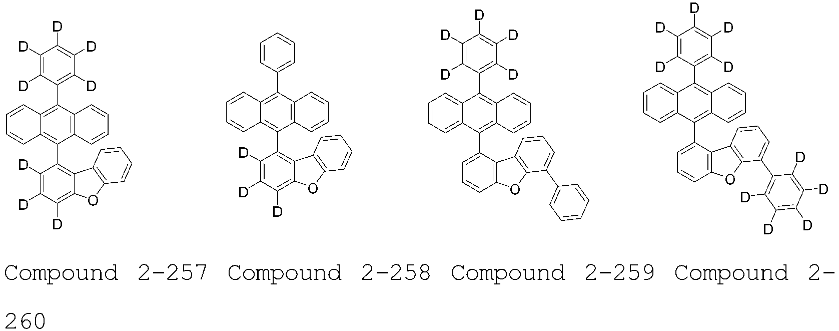

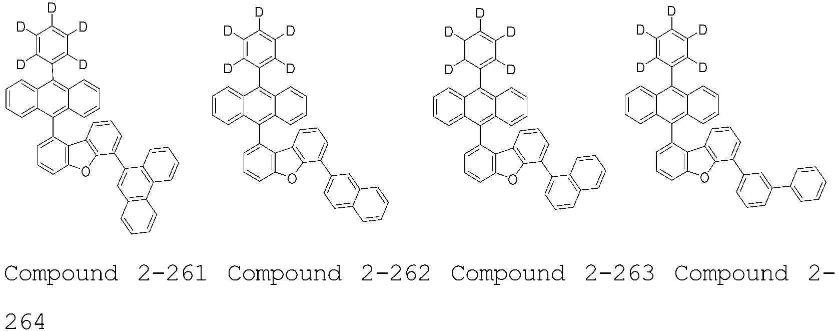

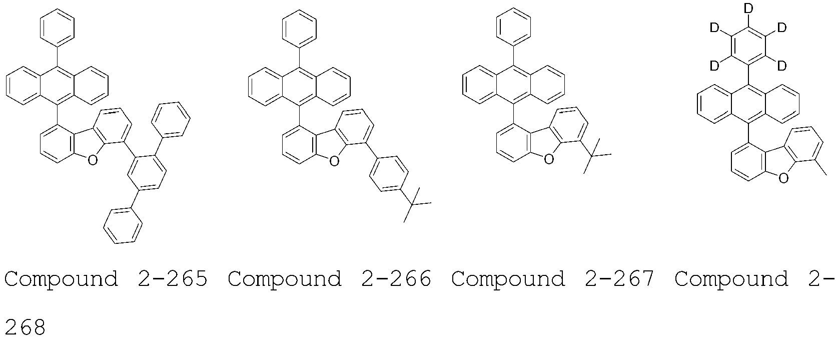

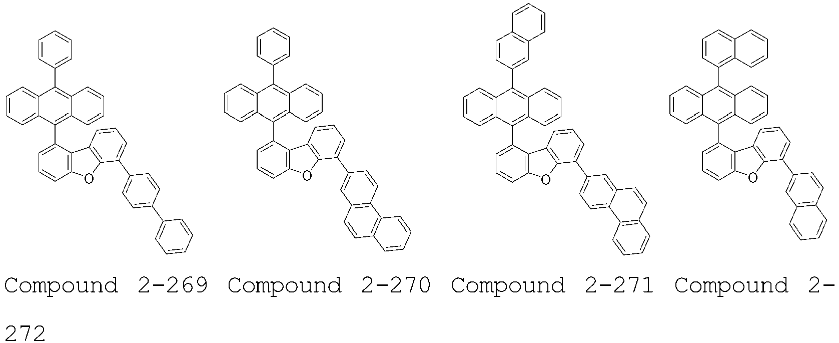

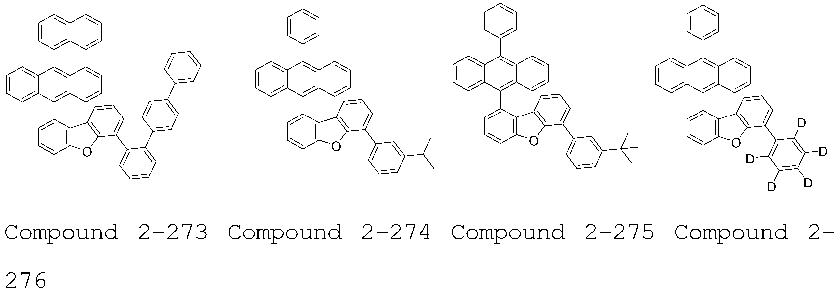

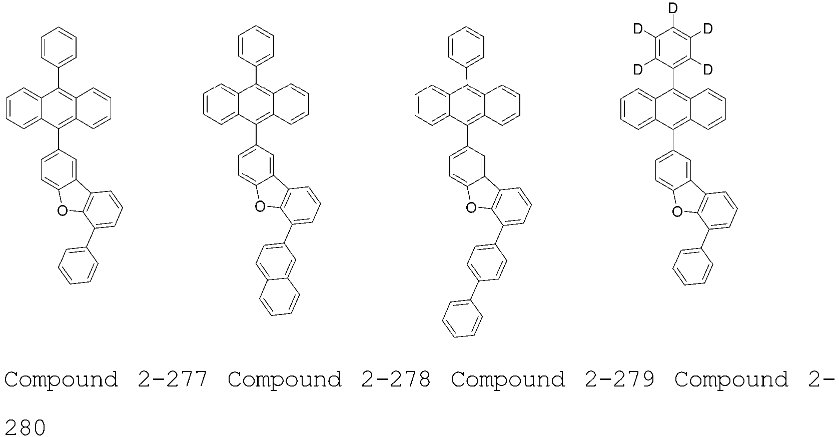

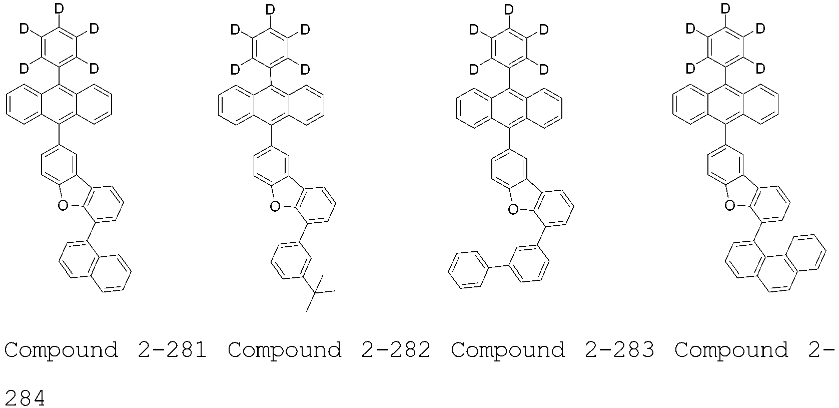

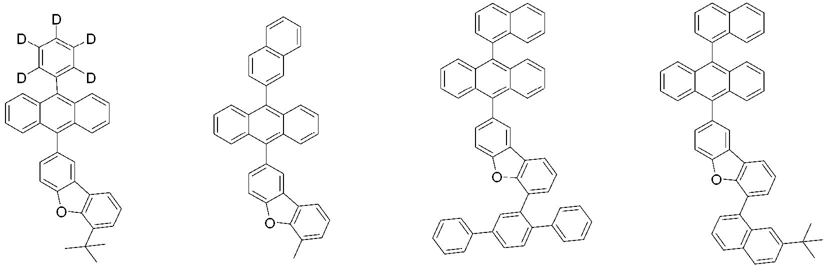

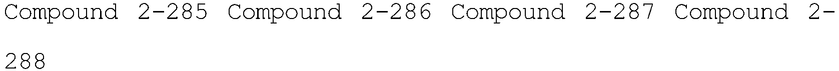

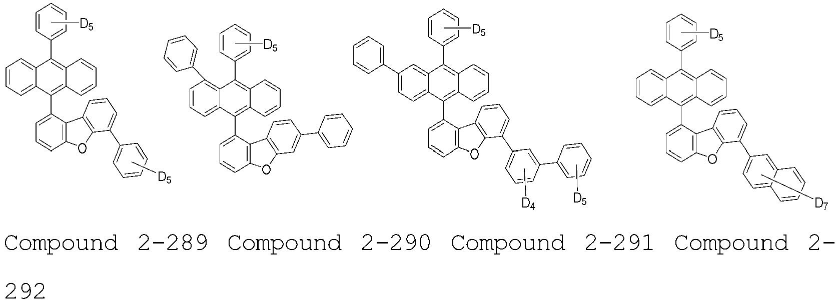

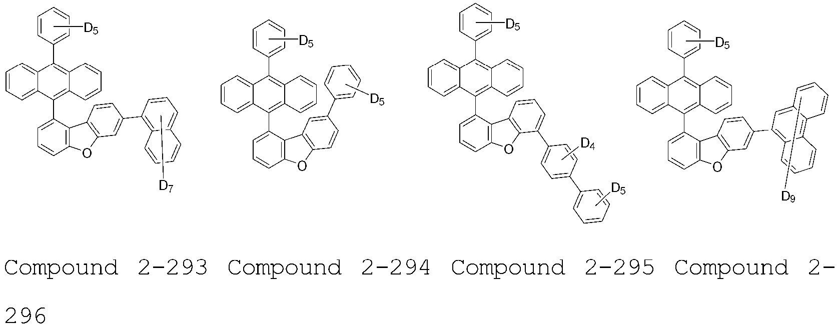

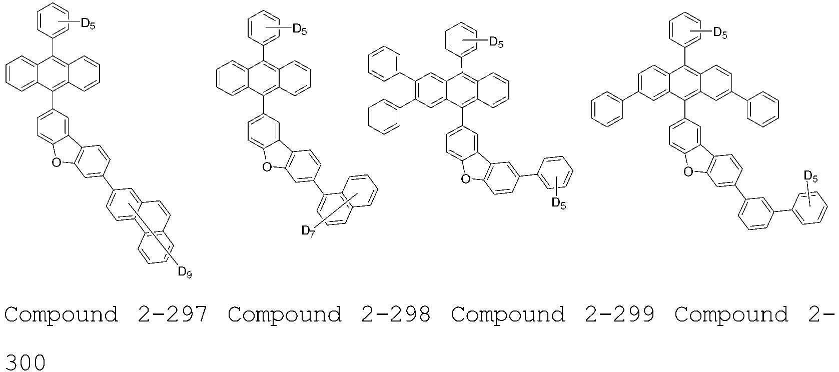

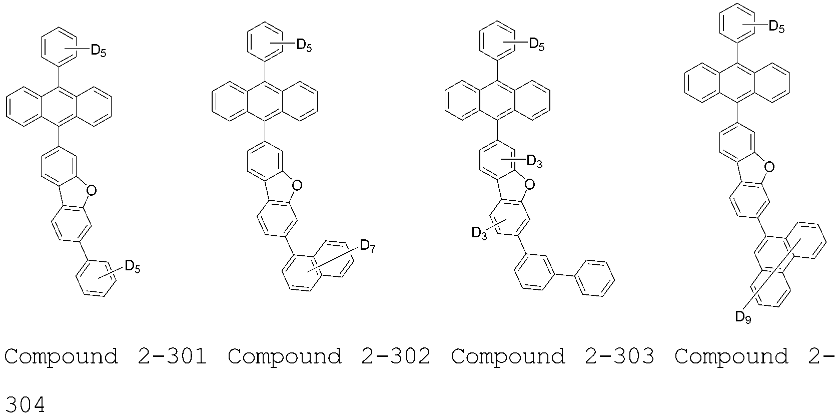

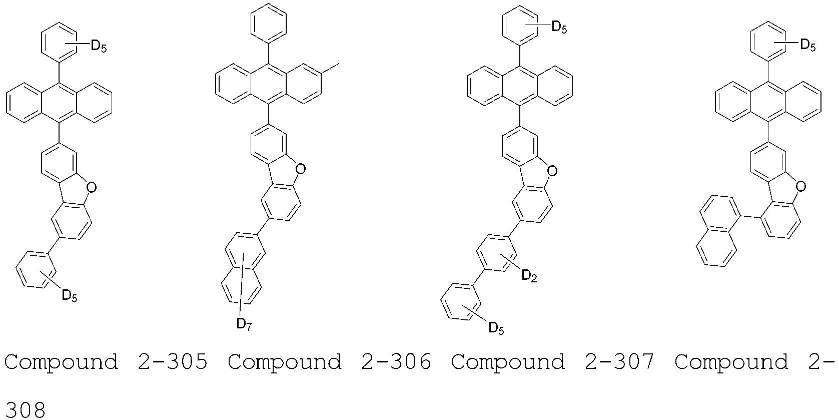

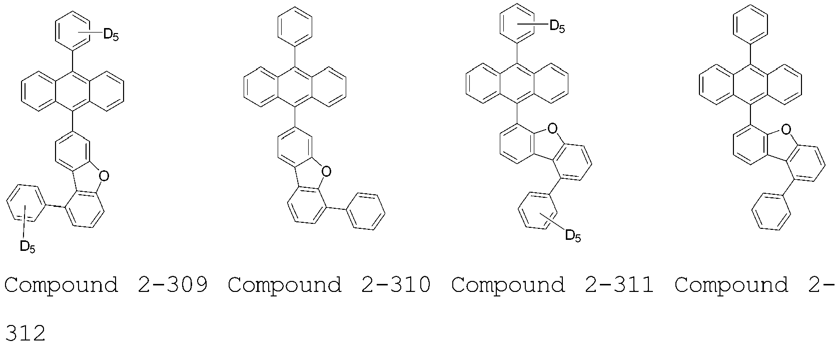

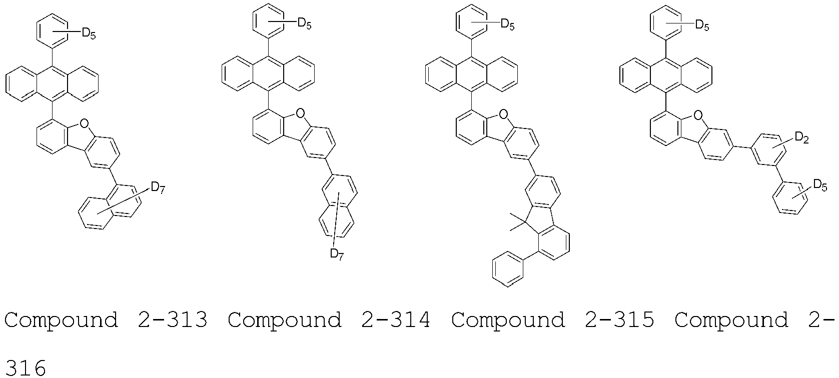

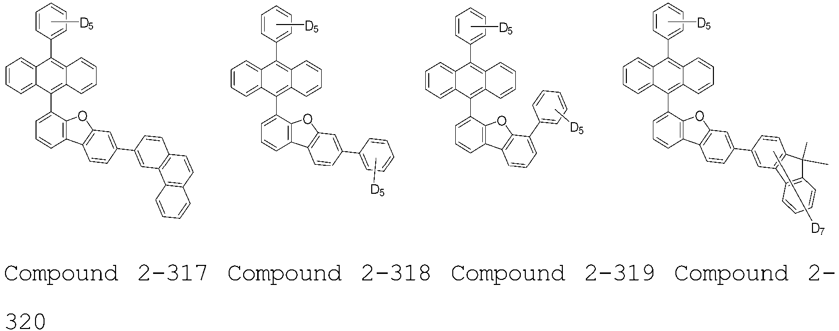

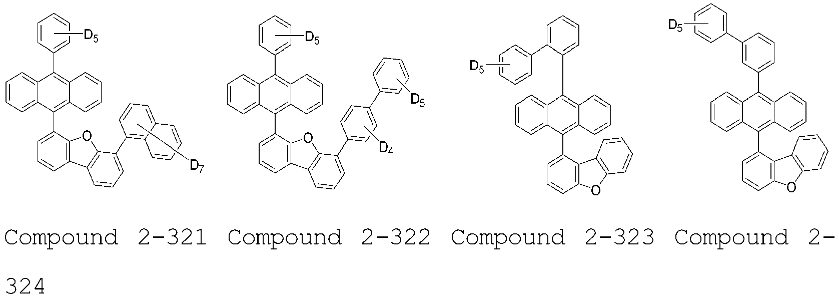

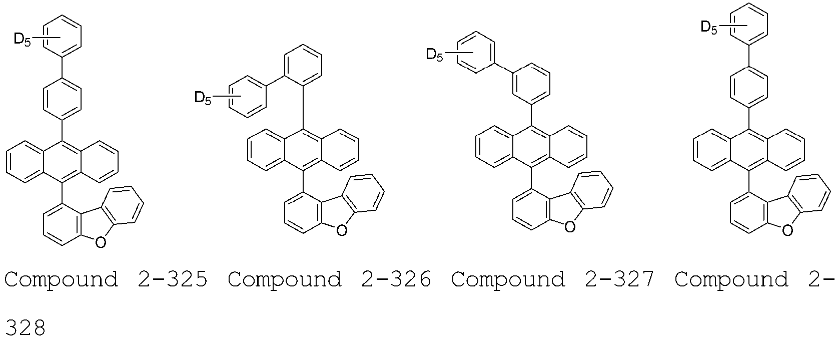

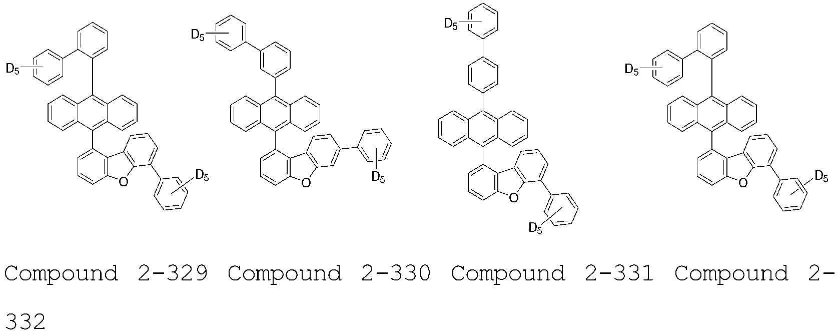

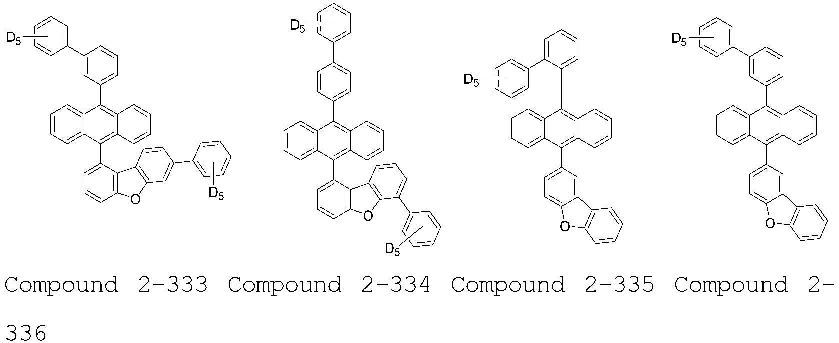

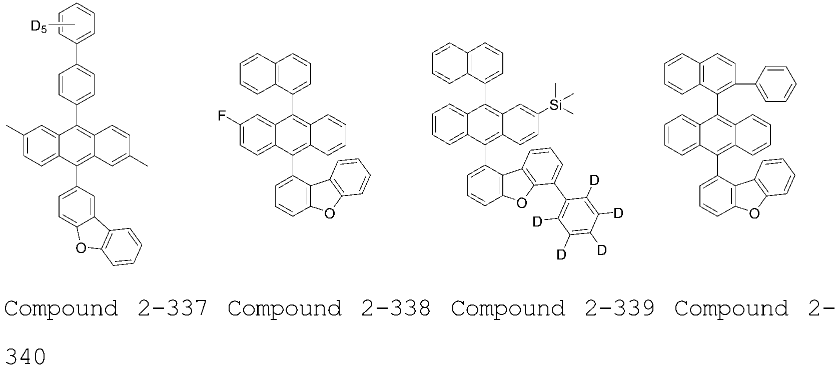

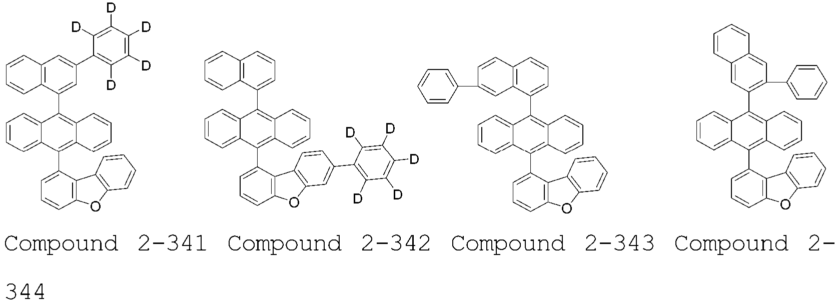

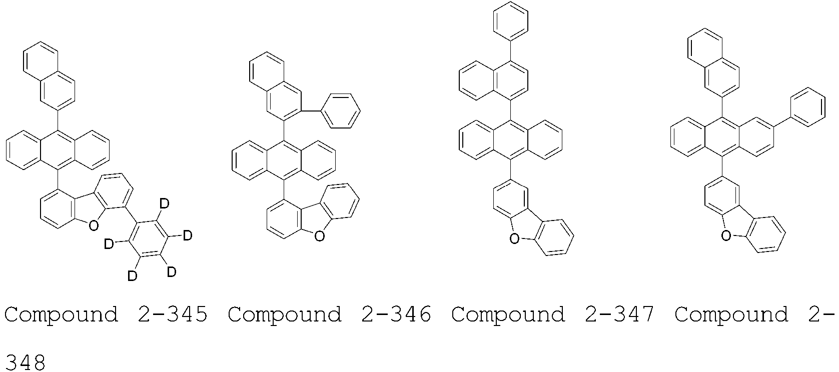

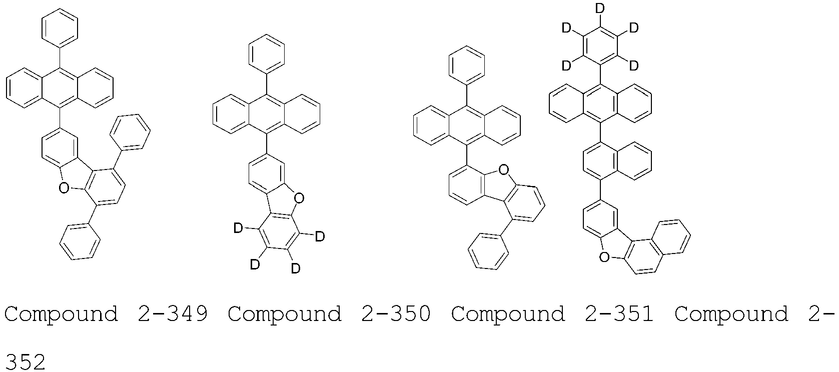

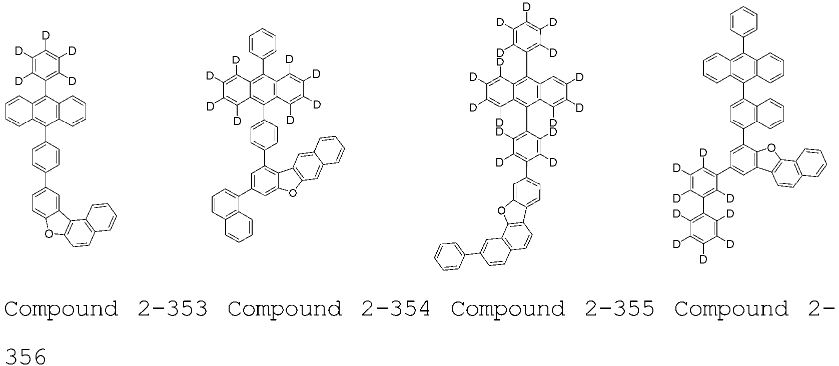

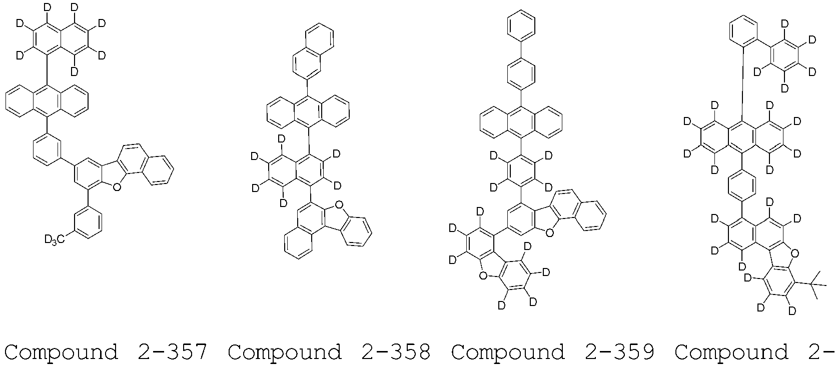

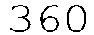

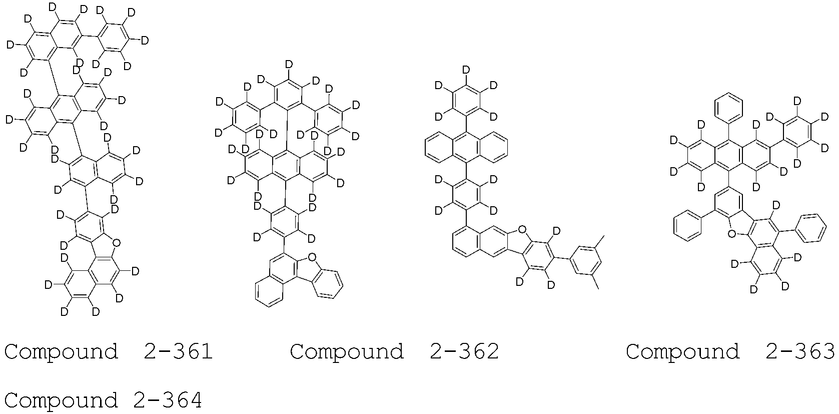

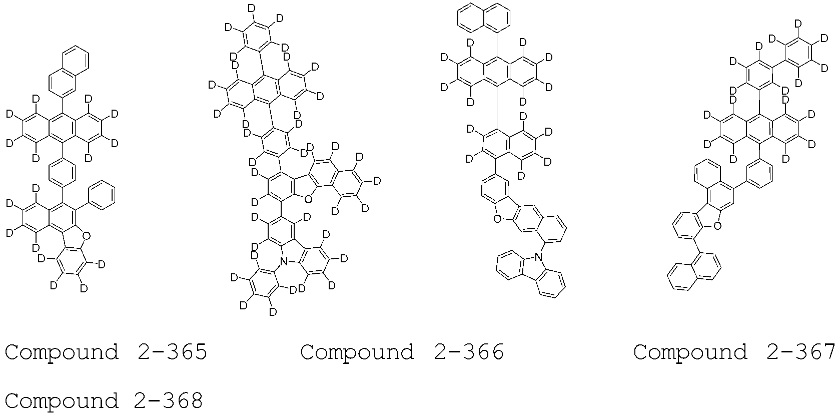

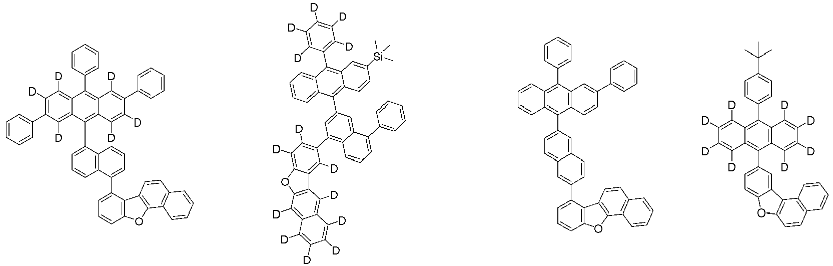

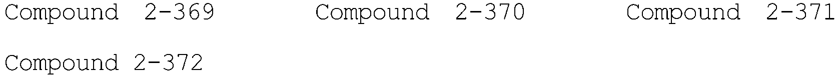

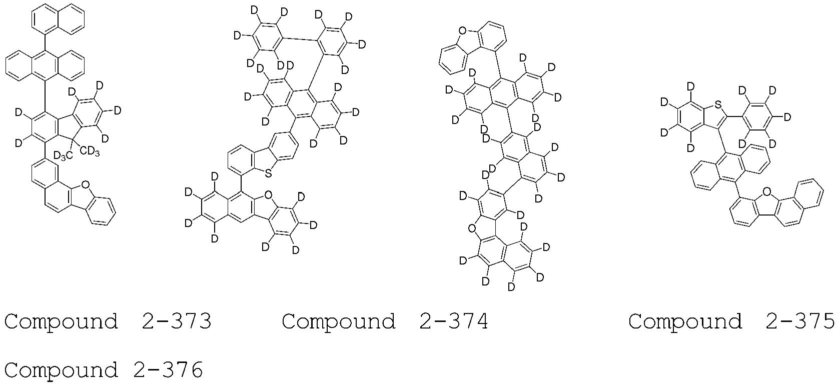

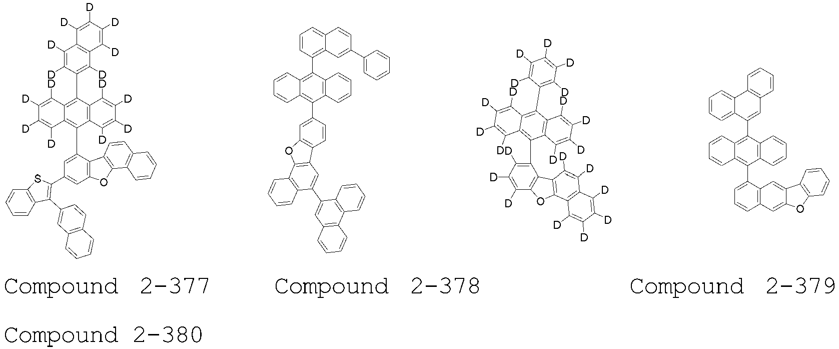

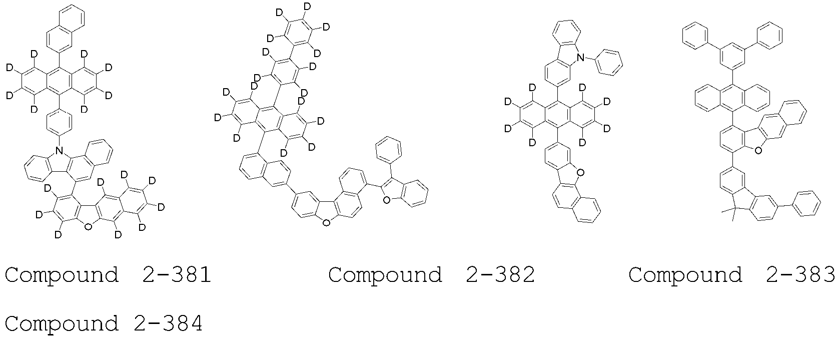

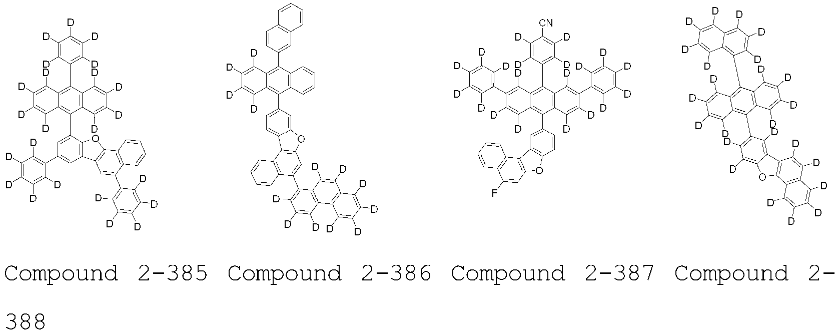

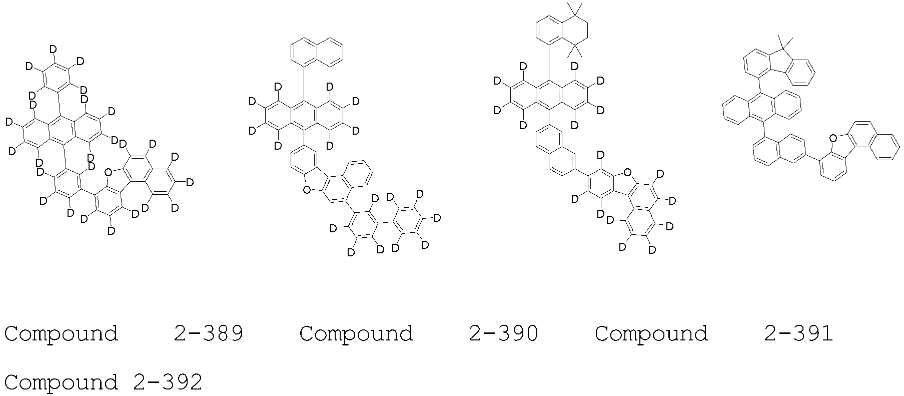

The organic layer in the organic light-emitting diode according to an embodiment of the present disclosure may further include at least one layer selected from a hole injection layer, a hole transport layer, a functional layer capable of both hole injection and hole transport, an electron blocking layer, a hole blocking layer, an electron transport layer, and an electron injection layer between the first electrode and the second electrode.

In an embodiment, the organic light-emitting diode according to the present disclosure may include at least one of a hole transport layer and a hole injection layer between the first electrode and the emission layer, and may include at least one of an electron transport layer and an electron injection layer between the emission layer and the second electrode.

In the present disclosure, the emission layer may employ a dopant material together with a host. When the emission layer contains a host and a dopant, the content of the dopant may be conventionally selected from the range of about 0.01 to about 20 parts by weight, based on about 100 parts by weight of the host, but with no limitations thereto.

In an embodiment of the organic light-emitting diode according to the present disclosure, the first emission layer may include a first host and a first dopant and the second emission layer may include a second host and a second dopant. In this regard, at least one of the first dopant in the first emission layer and the second dopant in the second emission layer may contain at least one polycyclic compound represented by the following Chemical Formula 6. Preferably, the first dopant in the first emission layer and the second dopant in the second emission layer, which may be same or different, may both contain at least one polycyclic compound represented by Chemical Formula 6: 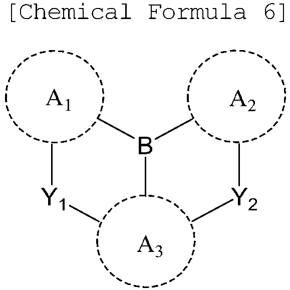 wherein,
Y₁ and Y₂, which are same or different, are each independently any one selected from O, S, NR₈₁, CR₈₂R₈₃, SiR₈₄R₈₅, and GeR₈₆R₈₇,
A₁ to A₃, which are identical to or different from each other, are each independently any one selected from a substituted or unsubstituted aromatic hydrocarbon ring of 6 to 50 carbon atoms, a substituted or unsubstituted aliphatic hydrocarbon ring of 5 to 50 carbon atoms, a substituted or unsubstituted, aliphatic hydrocarbon ring-fused aromatic hydrocarbon ring of 8 to 50 carbon atoms, a substituted or unsubstituted aromatic heterocyclic ring of 2 to 50 carbon atoms, and a substituted or unsubstituted, aliphatic hydrocarbon ring-fused aromatic heterocyclic ring of 5 to 50 carbon atoms,
R₈₁ to R₈₇, which are identical to or different from each other, are each independently a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₈₁ to R₈₇ may be connected to the A₁ to A₃ ring moieties to additionally form a mono- or polycyclic aliphatic or aromatic ring, and
a linkage may be made between R₈₂ and R₈₃, between R₈₄ and R₈₅, and between R₈₆ and R₈₇ to additionally form respective mono- or polycyclic aliphatic or aromatic rings,
wherein the term "substituted" in the expression "substituted or unsubstituted" used for the compound of Chemical Formula 6 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, a substituted or unsubstituted aliphatic heterocyclic ring-fused aryl of 6 to 30 carbon atoms, a substituted or unsubstituted aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with at least one hydrogen atom on the substituents being substitutable with a deuterium or tritium atom.

In a more preferred embodiment of the present disclosure, the A₁ to A₃ moieties, which are identical to or different from each other, may each be independently any one selected from a substituted or unsubstituted aromatic hydrocarbon ring of 6 to 30 carbon atoms and a substituted or unsubstituted aliphatic hydrocarbon ring-fused aromatic hydrocarbon ring of 8 to 50 carbon atoms.

In an embodiment, the first dopant in the first emission layer and the second dopant in the second emission layer are identical to each other and may both include at least one type of the polycyclic compounds represented by Chemical Formula 6.

In addition, when the first dopant or second dopant in the organic light-emitting diode according to the present disclosure contains at least one type of the polycyclic compound represented by chemical Formula 6, the polycyclic compound represented by Chemical Formula 6 may be a polycyclic compound represented by the following Chemical Formula 6-1 or 6-2. Preferably, the first dopant in the first emission layer and the second dopant in the second emission layer may both be a polycyclic compound represented by Chemical Formula 6-1 or 6-2: 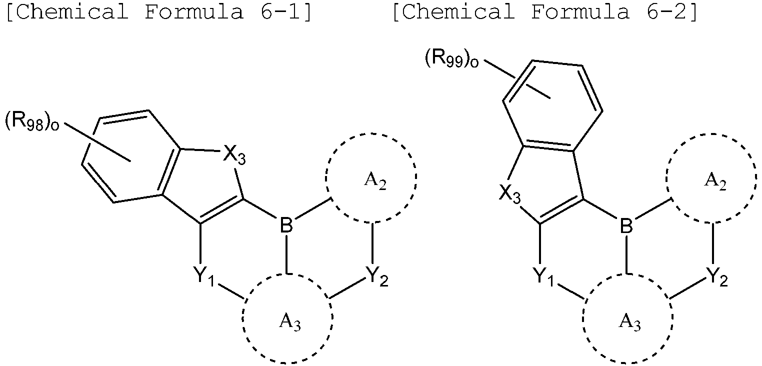 wherein,
X₃ is O or S,
Y₁ and Y₂, which are identical to or different from each other, are each independently any one selected from O, S, NR₉₁, CR₉₂R₉₃, SiR₉₄R₉₅, and GeR₉₆R₉₇,
A₂ and A₃, which are identical to or different from each other, are each independently any one selected from a substituted or unsubstituted aromatic hydrocarbon ring of 6 to 50 carbon atoms, a substituted or unsubstituted aliphatic hydrocarbon ring of 5 to 50 carbon atoms, a substituted or unsubstituted aliphatic hydrocarbon ring-fused aromatic hydrocarbon ring of 8 to 50 carbon atoms, a substituted or unsubstituted aromatic heterocyclic ring of 2 to 50 carbon atoms, and a substituted or unsubstituted aliphatic hydrocarbon ring-fused aromatic heterocyclic ring of 5 to 50 carbon atoms,
R₉₁ to R₉₈, which are identical to or different from each other, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₉₁ to R₉₇ may be connected to A₂ and A₃ ring moieties to additionally form aliphatic or aromatic mono- or polycyclic ring,
a linkage may be formed between R₉₂ and R₉₃, between R₉₄ and R₉₅, and between R₉₆ and R₉₇ to additionally form respective aliphatic or aromatic mono- or polycyclic rings,
∘ is 4, wherein the corresponding R₉₈ substituents or R₉₉ substituents are identical to or different from each other, and
adjacent R₉₈ substituents or R₉₉ substituents may be connected to additionally form an aliphatic or aromatic mono- or polycyclic ring,
wherein the term "substituted" in the expression "substituted or unsubstituted" used for the compound of Chemical Formulas 6-1 and 6-2 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, a substituted or unsubstituted aliphatic heterocyclic ring-fused aryl of 6 to 30 carbon atoms, a substituted or unsubstituted aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with at least one hydrogen atom on the substituents being substitutable with a deuterium or tritium atom.

In an embodiment, concrete examples of the compound reprenseted by Chemical Formula 6 include, but are not limited to:

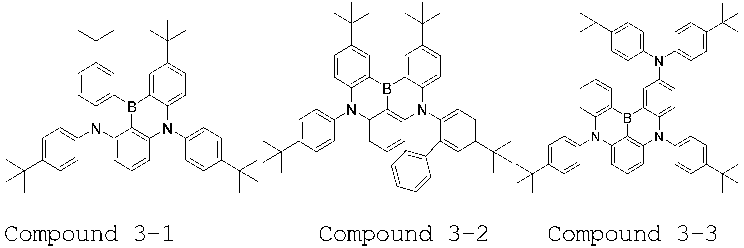

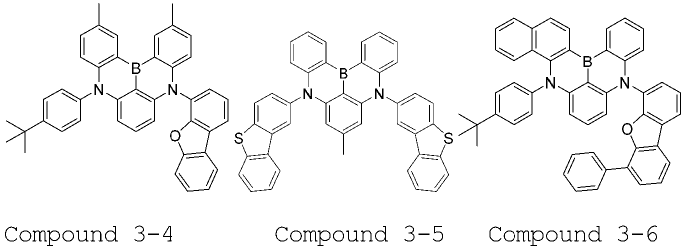

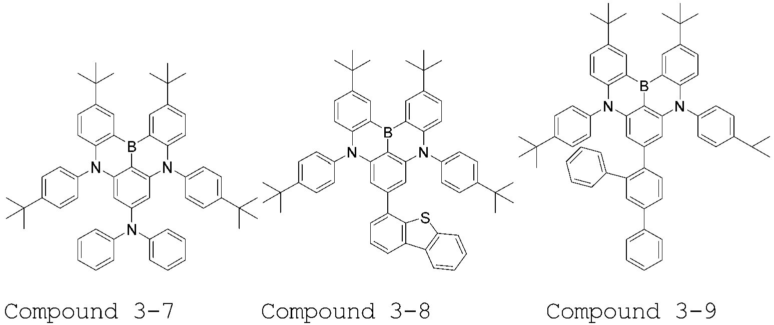

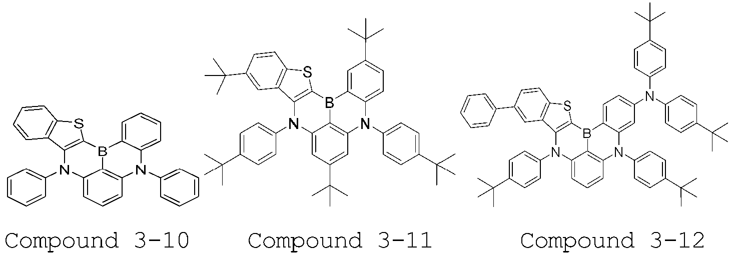

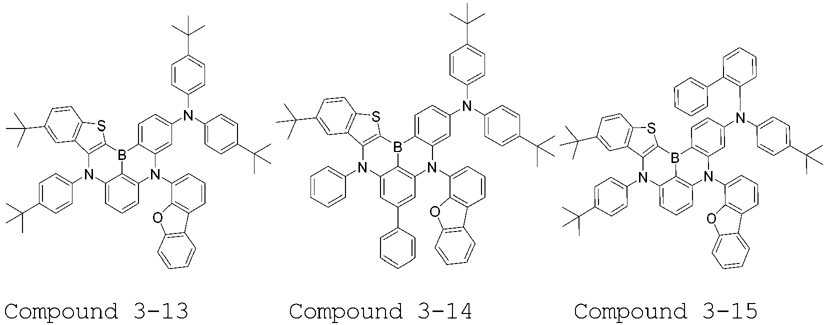

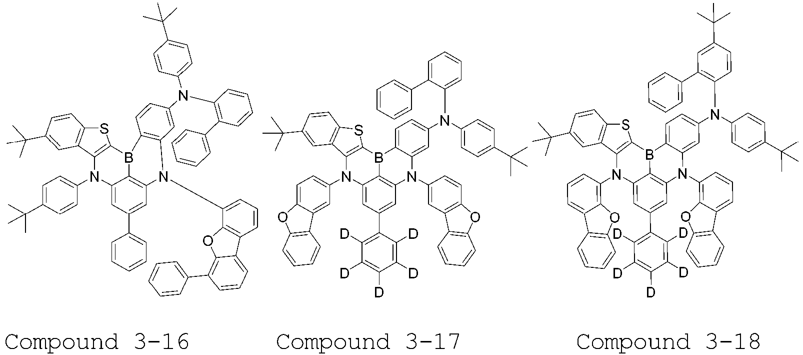

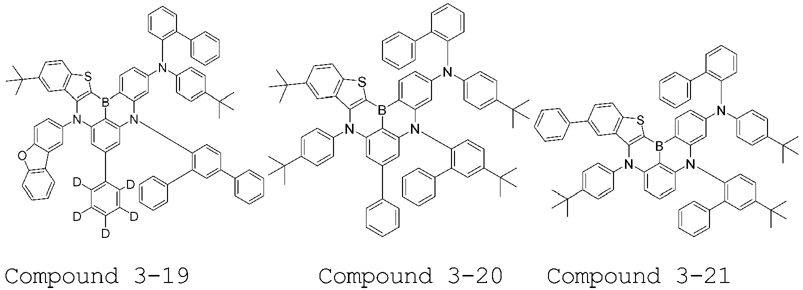

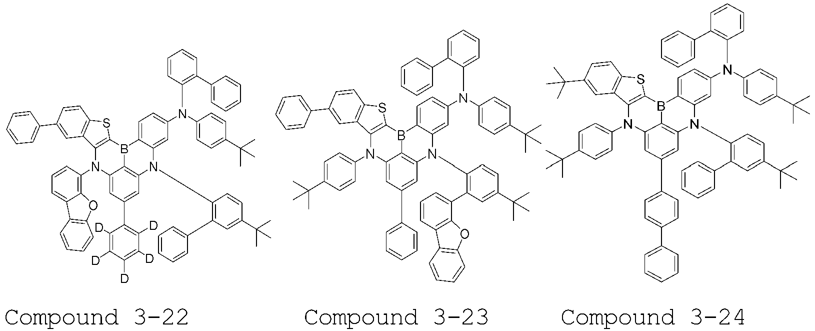

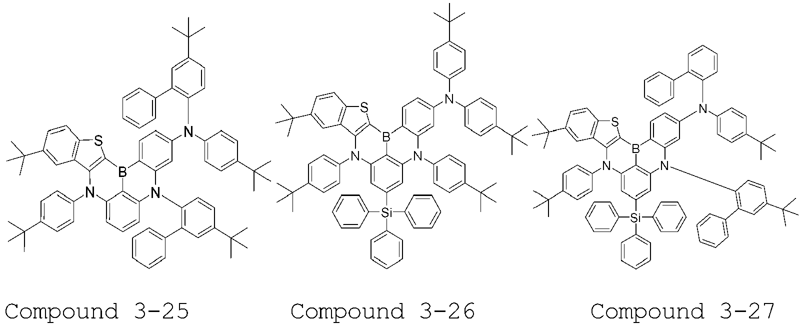

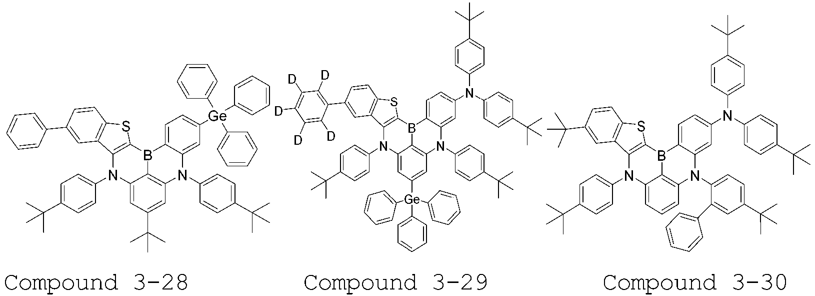

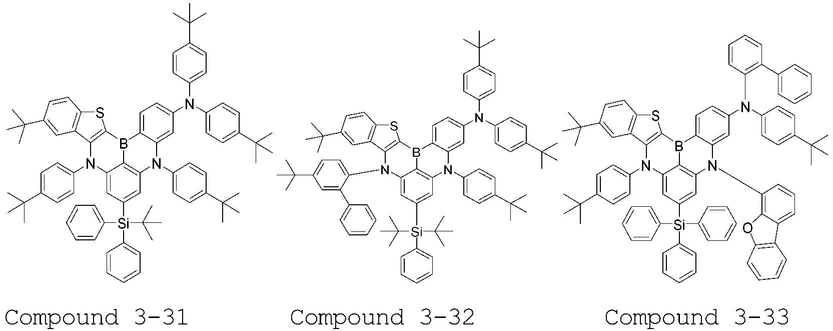

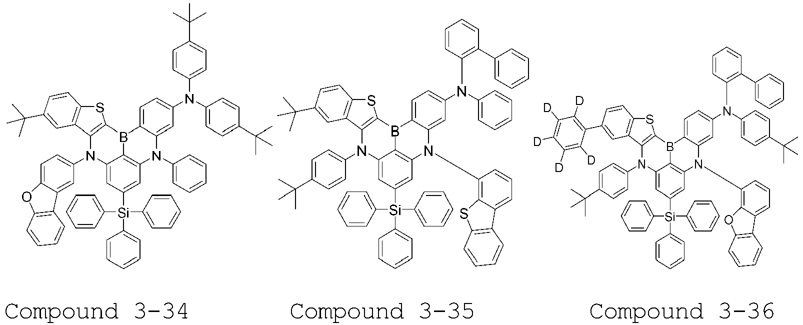

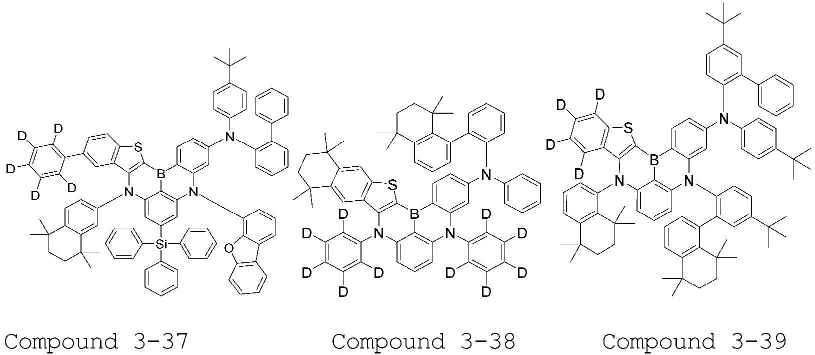

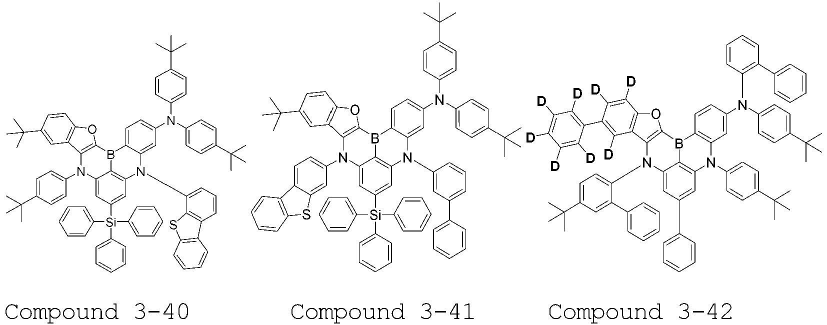

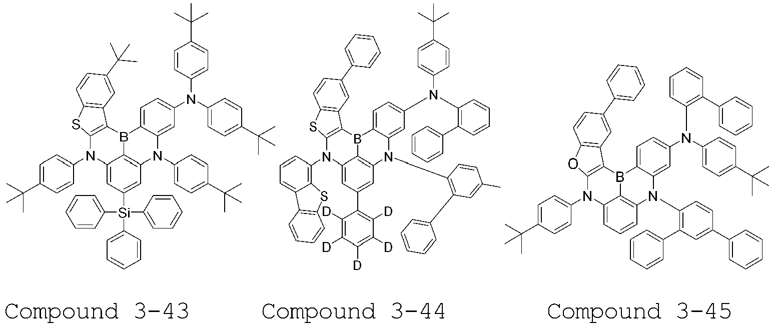

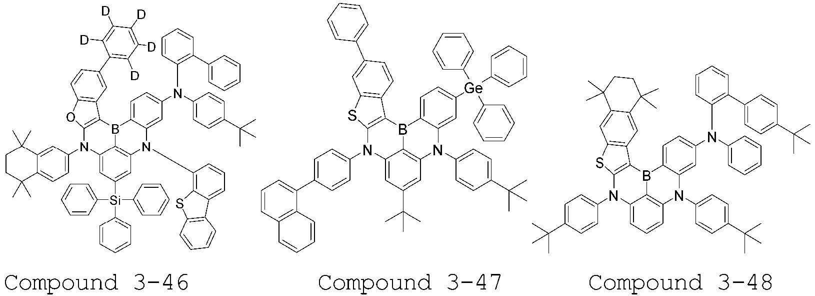

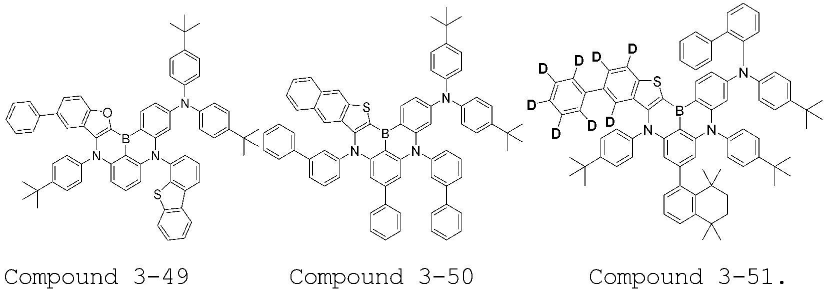

Below, with reference to the drawing, a description will be given of the organic light-emitting diode of the present disclosure.

FIGURE is a schematic view illustrating the structure of an organic light-emitting diode according to an embodiment of the present disclosure.

As shown in FIGURE, the organic light-emitting diode according to an embodiment of the present disclosure comprises an anode (20), a hole transport layer (40), a first emission layer (50'), a second emission layer (50), an electron transport layer (60), and a cathode (80), wherein the anode and the cathode serve as a first electrode and a second electrode, respectively, with the interposition of the hole transport layer between the anode and the light emission layer, and the electron transport layer between the emission layer and the cathode.

Furthermore, the organic light-emitting diode according to an embodiment of the present disclosure may include a hole injection layer (30) between the anode (20) and the hole transport layer (40), and an electron injection layer (70) between the electron transport layer (60) and the cathode (80).

Reference is made to FIGURE with regard to the organic light-emitting diode of the present disclosure and the fabrication thereof.

First, a substrate (10) is coated with an anode material to form an anode (20). So long as it is used in a typical organic electroluminescence (EL) device, any substrate may be used as the substrate (10). Preferable is an organic substrate or transparent plastic substrate that exhibits excellent transparency, surface smoothness, ease of handling, and waterproofness. As the anode material, indium tin oxide (ITO), indium zinc oxide (IZO), tin oxide (SnO₂), or zinc oxide (ZnO), which are transparent and superior in terms of conductivity, may be used.

A hole injection layer material is applied on the anode (20) by thermal deposition in a vacuum or by spin coating to form a hole injection layer (30). Subsequently, thermal deposition in a vacuum or by spin coating may also be conducted to form a hole transport layer (40) with a hole transport layer material on the hole injection layer (30).

So long as it is typically used in the art, any material may be selected for the hole injection layer (30) without particular limitations thereto. Examples include, but are not limited to, 2-TNATA [4,4',4"-tris(2-naphthylphenyl-phenylamino)-triphenylamine], NPD[N,N'-di(1-naphthyl)-N,N'-diphenylbenzidine)], TPD[N,N'-diphenyl-N,N'-bis(3-methylphenyl)-1,1'-biphenyl-4,4'-diamine], DNTPD[N,N'-diphenyl-N,N'-bis-[4-(phenyl-m-tolyl-amino)-phenyl]-biphenyl-4,4'-diamine], HAT-CN[1,4,5,8,9,11-Hexaazatriphenylenehexacarbonitrile], etc.

Any material that is typically used in the art may be selected for the hole transport layer (40) without particular limitations thereto. Examples include, but are not limited to, N,N'-bis(3-methylphenyl)-N,N'-diphenyl-[1,1-biphenyl]-4,4'-diamine(TPD) or N,N'-di(naphthalen-1-yl)-N,N'-diphenylbenzidine (a-NPD), N-[[1,1'-biphenyl]-4-yl]-9,9-dimethyl-N-[4-(9-phenyl-9H-carbazol-3-yl)phenyl]-9H-fluorene-2-amine(BCFN), and the like.

In an embodiment of the present disclosure, an electron blocking layer may be additionally disposed on the hole transport layer. Functioning to prevent the electrons injected from the electron injection layer from entering the hole transport layer through the emission layer, the electron blocking layer is adapted to increase the life span and luminous efficiency of the diode. The electron blocking layer may be formed at a suitable position between the emission layer and the hole injection layer and particularly between the emission layer and the hole transport layer.

Next, the first emission layer (50') and the second emission layer (50) may each be deposited or co-deposited on the hole transport layer (40) by deposition in a vacuum or by spin coating.

Here, the compound represented by any one of Chemical Formulas 1 to 4; or the compound represented by Chemical Formula 5 may be used as a host material in the first or second emission layer of the organic light-emitting layer while the compound represented by Chemical Formula 6 may be used as a dopant material in the first or second emission layer of the organic light-emitting diode. The materials constituting these layers are as previously described.

In addition, as a method for forming the emission layer, a thin film may be formed by depositing (codepositing) a plurality or one type of first host material and a dopant material from different deposition sources. Alternatively, the plurality of hosts (for example, the first host material and the second host material) may be mixed in advance, and the mixed hosts may be co-deposited with the dopant using a different deposition source.

Herein, when each host is separately co-deposited from different deposition sources, there is a disadvantage in that the manufacturing process becomes complicated. However, since the thin film is formed without the respective materials contacting each other in advance, deterioration in device characteristics caused by interactions between different compounds, which may occur when the compounds are premixed in a single deposition source, can be prevented. On the other hand, when the hosts are premixed in advance, there is an advantage in that the manufacturing apparatus or manufacturing process can be simplified.

According to a specific embodiment of the present disclosure, the thickness of the first emission layer (50') and the second emission layer (50) may range from 30 Å to 200 Å and preferably from 50 Å to 150 Å.

The electron transport layer (60) may be deposited on the emitting layer by vacuum deposition or spin coating.

In the present disclosure, the electron transport layer (60) functions to stably transport electrons injected from the electron-injecting electrode (cathode), and known electron transport materials may be used. Examples of such known materials include quinoline derivatives, particularly tris(8-quinolinolato)aluminum (Alq₃), Liq, TAZ, BAlq, beryllium bis(benzoquinolin-10-olate) (Bebq₂), Compound 201, Compound 202, BCP, and oxadiazole derivatives such as PBD, BMD, and BND, but are not limited thereto:

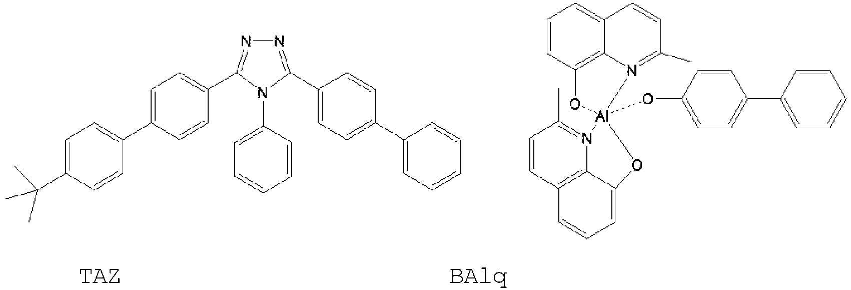

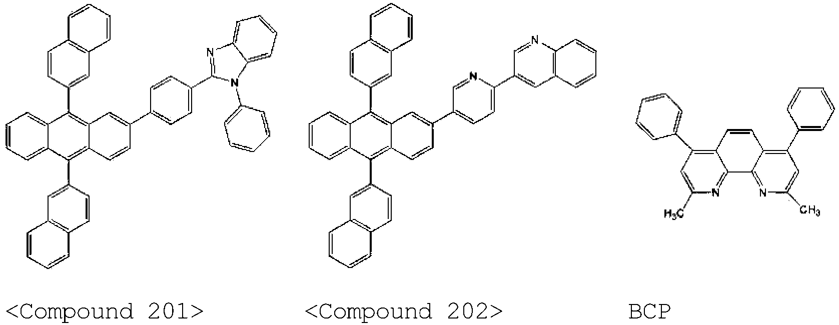

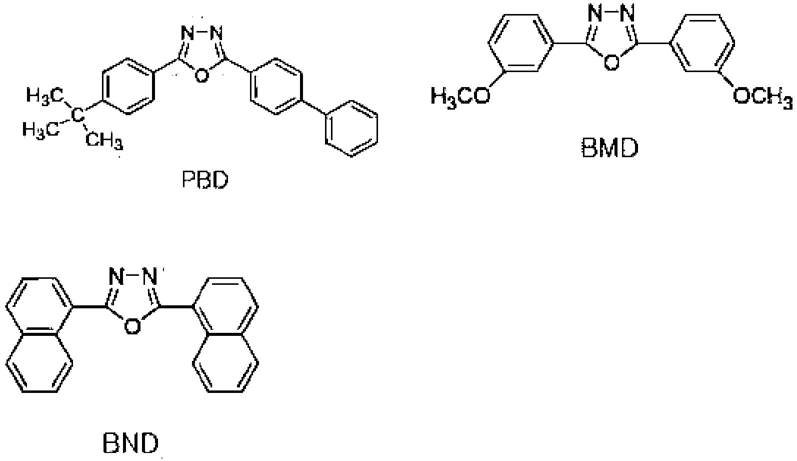

In addition, after formation of the electron transport layer, an electron injection layer (EIL) that functions to facilitate electron injection from the cathode may be formed on the electron transport layer. The material for the EIL is not particularly limited.

Any material that is conventionally used in the art can be available for the electron injection layer (70) without particular limitations. Examples include CsF, NaF, LiF, Li₂O, and BaO. Deposition conditions for the electron injection layer may vary, depending on compounds used, but may be generally selected from condition scopes that are almost the same as for the formation of hole injection layers.

The electron injection layer (70) may range in thickness from about 1 Å to about 100 Å, and particularly from about 3 Å to about 90 Å. Given the thickness range for the electron injection layer, the diode can exhibit satisfactory electron injection properties without actually elevating a driving voltage.

In order to facilitate electron injection, the cathode (80) may be made of a material having a small work function, such as metal or metal alloy such as lithium (Li), magnesium (Mg), calcium (Ca), an alloy aluminum (Al) thereof, aluminum-lithium (Al-Li), magnesium-indium (Mg-In), and magnesium-silver (Mg-Ag). Alternatively, ITO or IZO may be employed to form a transparent cathode for an organic light-emitting diode.

Moreover, the organic light-emitting diode of the present disclosure may further comprise a emission layer containing a blue, green, or red luminescent material that emits radiations in the wavelength range of 380 nm to 800 nm. That is, the emission layer in the present disclosure has a multi-layer structure wherein the blue, green, or red luminescent material may be a fluorescent material or a phosphorescent material.

Furthermore, at least one selected from among the layers may be deposited using a single-molecule deposition process or a solution process.

Here, the deposition process is a process by which a material is vaporized in a vacuum or at a low pressure and deposited to form a layer, and the solution process is a method in which a material is dissolved in a solvent and applied for the formation of a thin film by means of inkjet printing, roll-to-roll coating, screen printing, spray coating, dip coating, spin coating, etc.

Also, the organic light-emitting diode of the present disclosure may be applied to a device selected from among flat display devices; flexible display devices; stretchable display devices; monochrome or grayscale flat illumination devices; monochrome or grayscale flexible illumination devices; vehicle or aircraft display devices, and display devices for virtual or augmented reality.

### (EXAMPLES)

### SYNTHESIS EXAMPLE 1. Synthesis of [Compound 1-1]

### Synthesis Example 1-1. Synthesis of A-1

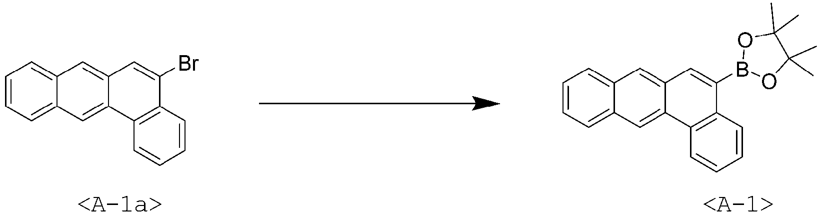

In a round-bottom flask, <A-1a> (50 g), bis(pinacolate)diboron (72.3 g), [1,1'-bis(diphenylphosphino)ferrocene]dichloropalladium(II) (6.6 g), potassium acetate (47.9 g), and toluene (500 mL) were stirred together under reflux. After completion of the reaction, the reaction mixture was subjected to extraction with water. The organic layer thus obtained was concentrated in a vacuum, followed by purification through column chromatography to afford Compound <A-1>. (50.5 g, 87.6%)

### Synthesis Example 1-2. Synthesis of A-2

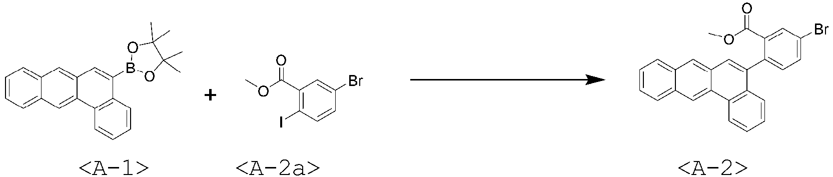

In a round-bottom flask, <A-1> (50 g), <A-2a> (48.1 g), Pd[(PPh)₃]₄ (3.3 g), K₂CO₃ (39 g), toluene (250 mL), ethanol (150 mL), and distilled water (100 mL) were stirred together for 24 hours under reflux. After completion of the reaction, the reaction mixture was subjected to extraction with water and ethyl acetate. The organic layer thus obtained was concentrated in a vacuum, followed by purification through column chromatography to afford Compound <A-2>. (45.7g, 73.6%)

### Synthesis Example 1-3. Synthesis of A-3

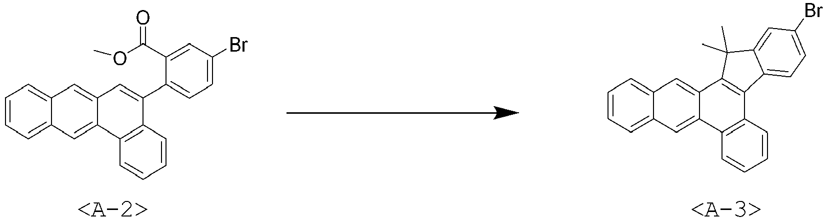

In a round-bottom flask, <A-2> (60 g) and THF (600 mL) was stirred together at -10°C for 30 minutes under a nitrogen atmosphere. Subsequent to slow addition of drops of methylmagnesium bromide (3M) (160 mL), the mixture was heated to room temperature and then stirred at 80°C for 2 hours under reflux. The temperature was lowered to -10°C, and quenching was performed with water and 2N HCl before extraction. The organic layer thus obtained was concentrated in a vacuum and dissolved in DCM (600 mL) and then, BF₃ was slowly added at -10°C thereto in a dropwise manner. After completion of the reaction, the reaction was subjected to extraction with water, and the organic layer thus formed was concentrated in a vacuum, followed by purification through column chromatography to afford Compound <A-3>. (46.7 g, 81.14%)

### Synthesis Example 1-4. Synthesis of [Compound 1-1]

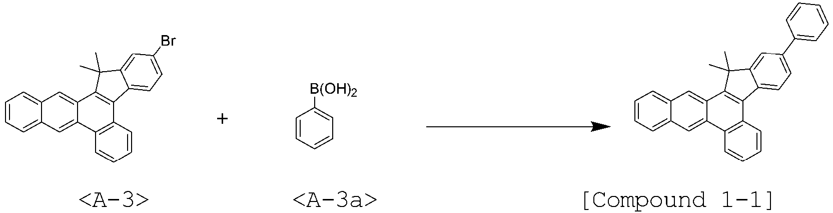

The same synthesis procedure as in Synthesis Example 1-2, with the exception of using <A-3a> and <A-3> instead of <A-1> and <A-2a>, respectively, was carried out to afford [Compound 1-1]. (yield 37.6%)
MS (MALDI-TOF) : m/z 420.19 [M⁺]

### SYNTHESIS EXAMPLE 2. Synthesis of [Compound 1-2]

### Synthesis Example 2-1. Synthesis of [Compound 1-2]

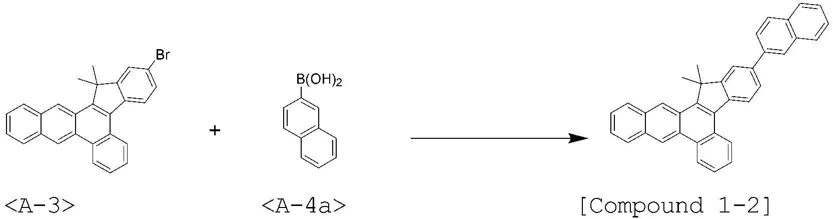

The same synthesis procedure as in Synthesis Example 1-2, with the exception of using <A-4a> and <A-3> instead of <A-1> and <A-2a>, respectively, was carried out to afford [Compound 1-2]. (yield 44%)
MS (MALDI-TOF) : m/z 470.20 [M⁺]

### SYNTHESIS EXAMPLE 3. Synthesis of [Compound 1-3]

### Synthesis Example 3-1. Synthesis of [Compound 1-3]

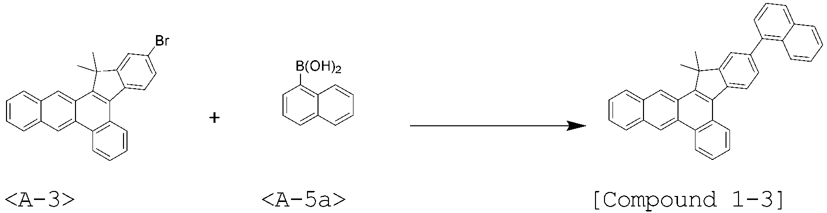

The same synthesis procedure as in Synthesis Example 1-2, with the exception of using <A-5a> and <A-3> instead of <A-1> and <A-2a>, respectively, was carried out to afford [Compound 1-3]. (yield 44%)
MS (MALDI-TOF) : m/z 470.20 [M⁺]

### SYNTHESIS EXAMPLE 4. Synthesis of [Compound 1-4]

### Synthesis Example 4-1. Synthesis of [Compound 1-4]

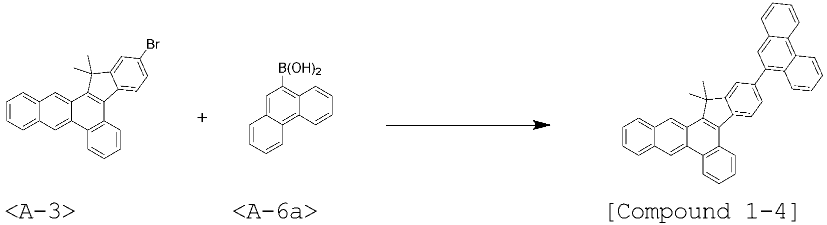

The same synthesis procedure as in Synthesis Example 1-2, with the exception of using <A-6a> and <A-3> instead of <A-1> and <A-2a>, respectively, was carried out to afford [Compound 1-4]. (yield 44%)
MS (MALDI-TOF) : m/z 520.22 [M⁺]

### SYNTHESIS EXAMPLE 5. Synthesis of [Compound 1-5]

### Synthesis Example 5-1. Synthesis of [Compound 1-5]

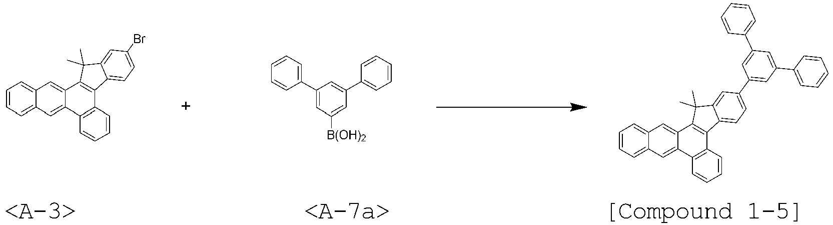

The same synthesis procedure as in Synthesis Example 1-2, with the exception of using <A-7a> and <A-3> instead of <A-1> and <A-2a>, respectively, was carried out to afford [Compound 1-5]. (yield 44%)
MS (MALDI-TOF) : m/z 572.25 [M⁺]

### SYNTHESIS EXAMPLE 6. Synthesis of [Compound 1-7]

### Synthesis Example 6-1. Synthesis of [Compound 1-7]

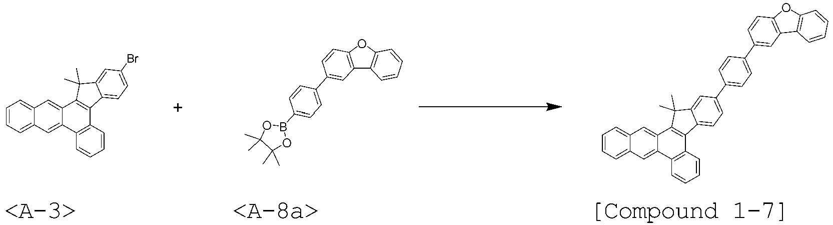

The same synthesis procedure as in Synthesis Example 1-2, with the exception of using <A-8a> and <A-3> instead of <A-1> and <A-2a>, respectively, was carried out to afford [Compound 1-7]. (yield 56.7%)
MS (MALDI-TOF) : m/z 586.23 [M⁺]

### SYNTHESIS EXAMPLE 7. Synthesis of [Compound 1-8]

### Synthesis Example 7-1. Synthesis of [Compound 1-8]

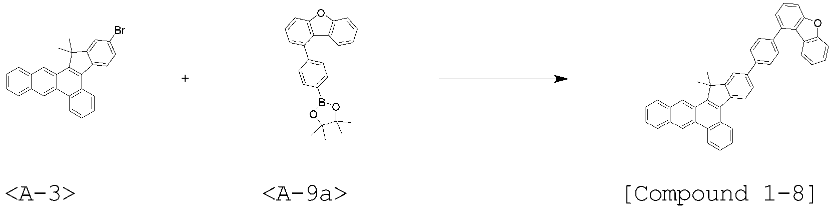

The same synthesis procedure as in Synthesis Example 1-2, with the exception of using <A-9a> and <A-3> instead of <A-1> and <A-2a>, respectively, was carried out to afford [Compound 1-8]. (yield 60.4%)
MS (MALDI-TOF) : m/z 586.23 [M⁺]

### SYNTHESIS EXAMPLE 8. Synthesis of [Compound 1-9]

### Synthesis Example 8-1. Synthesis of B-1

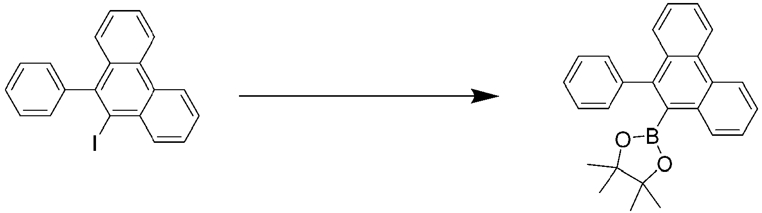

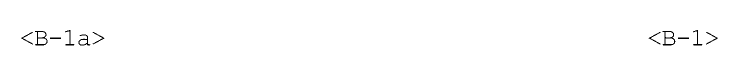

The same synthesis procedure as in Synthesis Example 1-1, with the exception of using <B-1a> instead of <A-1a>, was carried out to afford <B-1>. (yield 79.8%)

### Synthesis Example 8-2. Synthesis of [Compound 1-9]

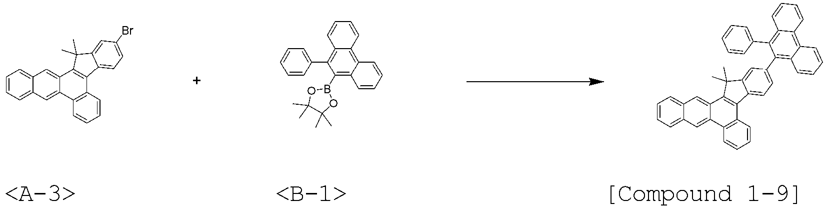

The same synthesis procedure as in Synthesis Example 1-2, with the exception of using <B-1> and <A-3> instead of <A-1> and <A-2a>, respectively, was carried out to afford [Compound 1-9]. (yield 54.7%)
MS (MALDI-TOF) : m/z 596.25 [M⁺]

### SYNTHESIS EXAMPLE 9. Synthesis of [Compound 1-10]

### Synthesis Example 9-1. Synthesis of B-2

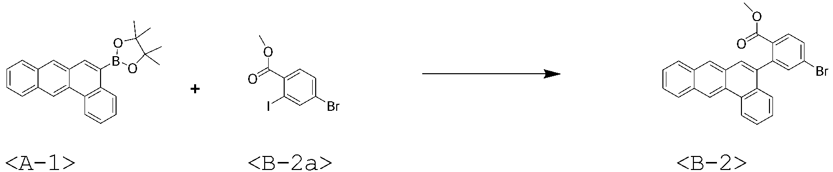

The same synthesis procedure as in Synthesis Example 1-2, with the exception of using <B-2a> instead of <A-2a>, was carried out to afford <B-2>. (yield 85.4%)

### Synthesis Example 9-2. Synthesis of B-3

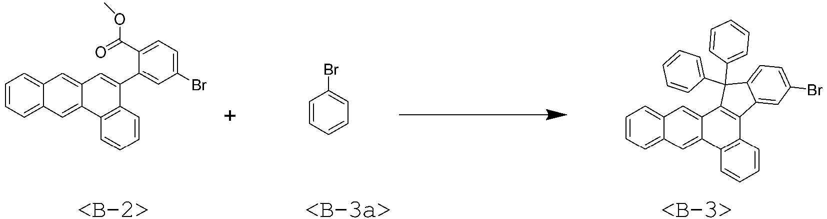

In a round-bottom flask, Compound <B-3a> (50 g) and THF were stirred together at -78°C while n-BuLi was slowly added in a dropwise manner. After 2 hours, Compound <B-2> (56.2g) was added to the flask the temperature of which was then elevated to room temperature. After completion of the reaction, the reaction mixture was subjected to extraction with water and the organic layer thus obtained was concentrated. Acetic acid (1000 mL) was added to the concentrate and stirred while HCl (5mL) was dropwise added. Recrystallization in MeOH was followed by filter drying to afford Compound <B-3>. (48.7 g, 69.8%)

### Synthesis Example 9-3. Synthesis of [Compound 1-10]

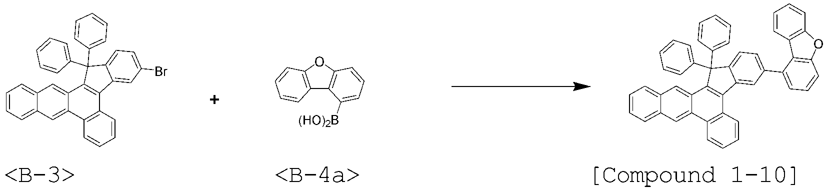

The same synthesis procedure as in Synthesis Example 1-2, with the exception of using <B-4a> and <B-3> instead of <A-1> and <A-2a>, respectively, was carried out to afford [Compound 1-10]. (yield 45.6%)
MS (MALDI-TOF) : m/z 634.23 [M⁺]

### SYNTHESIS EXAMPLE 10. Synthesis of [Compound 1-11]

### Synthesis Example 10-1. Synthesis of [Compound 1-11]

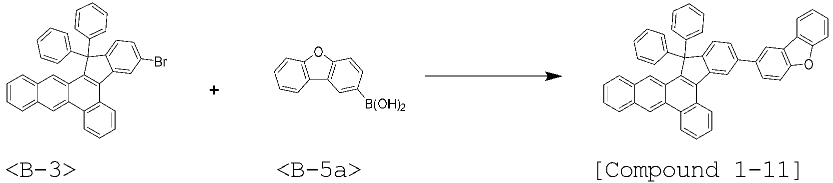

The same synthesis procedure as in Synthesis Example 1-2, with the exception of using <B-5a> and <B-3> instead of <A-1> and <A-2a>, respectively, was carried out to afford [Compound 1-11]. (yield 45.6%)
MS (MALDI-TOF) : m/z 634.23 [M⁺]

### SYNTHESIS EXAMPLE 11. Synthesis of [Compound 1-12]

### Synthesis Example 11-1. Synthesis of [Compound 1-12]

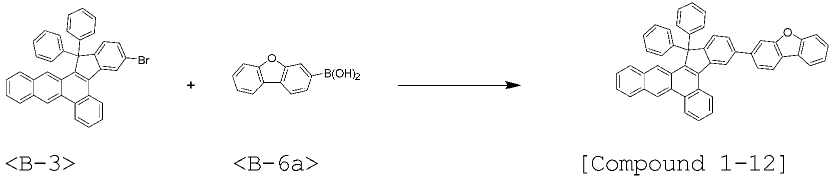

The same synthesis procedure as in Synthesis Example 1-2, with the exception of using <B-6a> and <B-3> instead of <A-1> and <A-2a>, respectively, was carried out to afford [Compound 1-12]. (yield 51.2%)
MS (MALDI-TOF) : m/z 634.23 [M⁺]

### SYNTHESIS EXAMPLE 12. Synthesis of [Compound 1-13]

### Synthesis Example 12-1. Synthesis of [Compound 1-13]

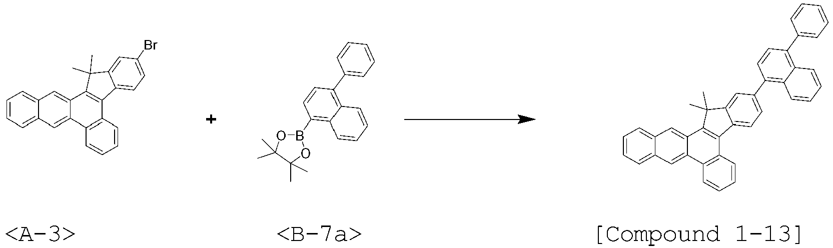

The same synthesis procedure as in Synthesis Example 1-2, with the exception of using <B-7a> and <A-3> instead of <A-1> and <A-2a>, respectively, was carried out to afford [Compound 1-13]. (yield 60.3%)
MS (MALDI-TOF) : m/z 546.23 [M⁺]

### SYNTHESIS EXAMPLE 13. Synthesis of [Compound 1-14]

### Synthesis Example 13-1. Synthesis of [Compound 1-14]

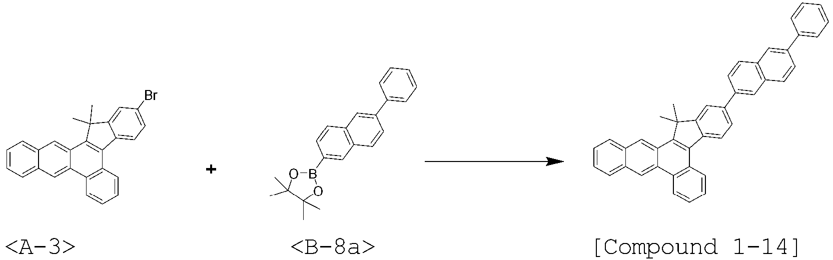

The same synthesis procedure as in Synthesis Example 1-2, with the exception of using <B-8a> and <A-3> instead of <A-1> and <A-2a>, respectively, was carried out to afford [Compound 1-14]. (yield 55.4%)
MS (MALDI-TOF) : m/z 546.23 [M⁺]

### SYNTHESIS EXAMPLE 14. Synthesis of [Compound 1-15]

### Synthesis Example 14-1. Synthesis of [Compound 1-15]

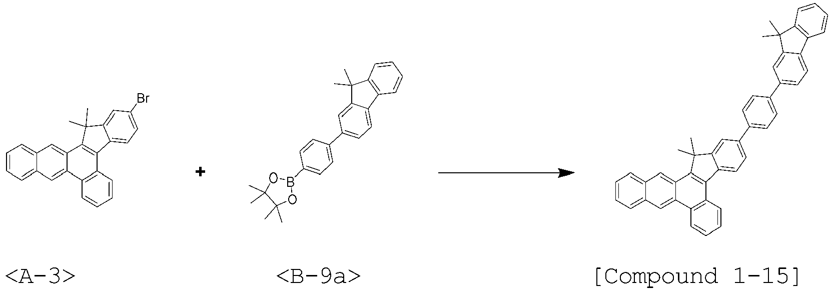

The same synthesis procedure as in Synthesis Example 1-2, with the exception of using <B-9a> and <A-3> instead of <A-1> and <A-2a>, respectively, was carried out to afford [Compound 1-15]. (yield 47.1%)
MS (MALDI-TOF) : m/z 612.28 [M⁺]

### SYNTHESIS EXAMPLE 15. Synthesis of [Compound 1-16]

### Synthesis Example 15-1. Synthesis of [Compound 1-16]

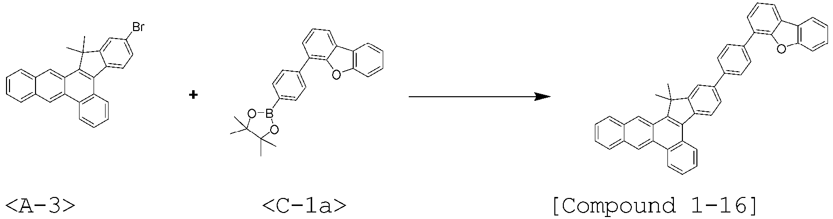

The same synthesis procedure as in Synthesis Example 1-2, with the exception of using <C-1a> and <A-3> instead of <A-1> and <A-2a>, respectively, was carried out to afford [Compound 1-16]. (yield 42.3%)
MS (MALDI-TOF) : m/z 586.23 [M⁺]

### SYNTHESIS EXAMPLE 16. Synthesis of [Compound 1-17]

### Synthesis Example 16-1. Synthesis of [Compound 1-17]

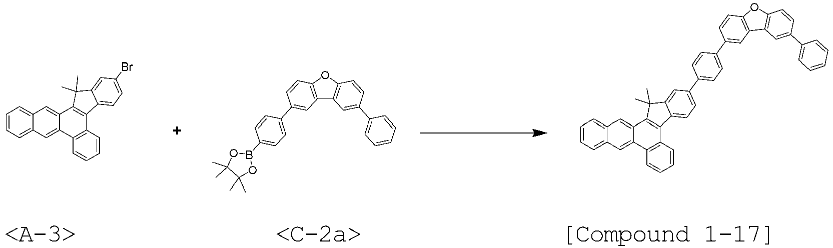

The same synthesis procedure as in Synthesis Example 1-2, with the exception of using <C-2a> and <A-3> instead of <A-1> and <A-2a>, respectively, was carried out to afford [Compound 1-17]. (yield 42.4%)
MS (MALDI-TOF) : m/z 662.26 [M⁺]

### SYNTHESIS EXAMPLE 17. Synthesis of [Compound 1-18]

### Synthesis Example 17-1. Synthesis of [Compound 1-18]

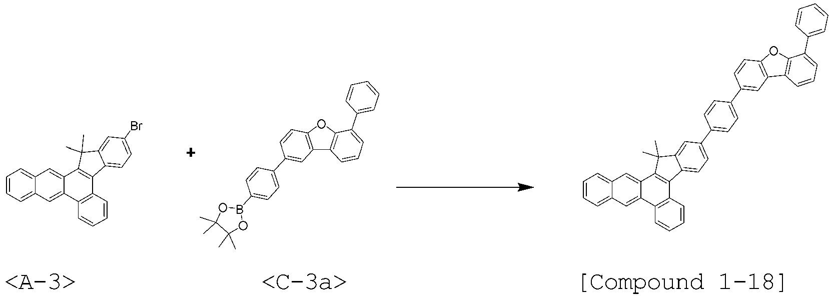

The same synthesis procedure as in Synthesis Example 1-2, with the exception of using <C-3a> and <A-3> instead of <A-1> and <A-2a>, respectively, was carried out to afford [Compound 1-18]. (yield 42.4%)
MS (MALDI-TOF) : m/z 662.26 [M⁺]

### SYNTHESIS EXAMPLE 18. Synthesis of [Compound 1-20]

### Synthesis Example 18-1. Synthesis of [Compound 1-20]

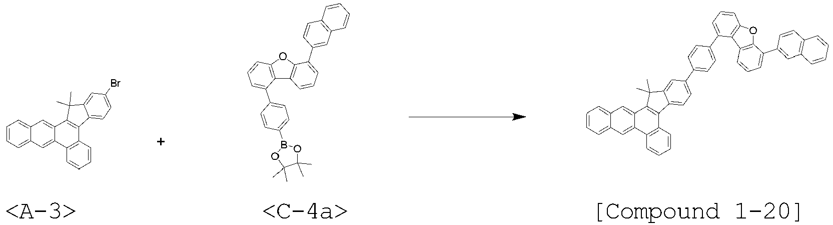

The same synthesis procedure as in Synthesis Example 1-2, with the exception of using <C-4a> and <A-3> instead of <A-1> and <A-2a>, respectively, was carried out to afford [Compound 1-20]. (yield 65.6%)
MS (MALDI-TOF) : m/z 712.28 [M⁺]

### SYNTHESIS EXAMPLE 19. Synthesis of [Compound 1-25]

### Synthesis Example 19-1. Synthesis of C-5

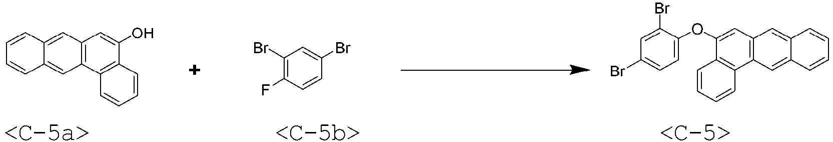

In a round-bottom flask, Compound <C-5a> (100 g), K₃PO₄ (173.8 g), Compound <C-5b> (102.9 g), and DMF (1000 mL) were stirred together at 90°C for 24 hours under reflux. After completion of the reaction, the reaction mixture was subjected to extraction with water and ethyl acetate. The organic layer thus obtained was concentrated and purified through column chromatography to afford Compound <C-5>. (140.6 g, 79.2%)

### Synthesis Example 19-2. Synthesis of C-6

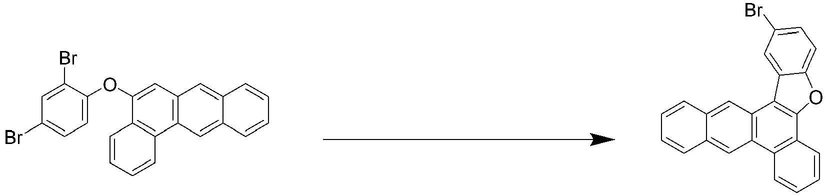

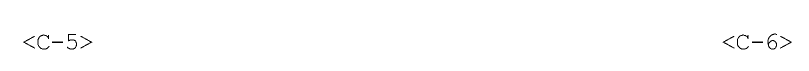

In a round-bottom flask, Compound <C-5> (60 g), triphenylphosphine (2.9 g), palladium acetate (0.9 g), DBU (42.1 g), and DMF (600 mL) were stirred together at 110°C for 24 hours under reflux. The reaction mixture was subjected to extraction with water and ethylacetate, and the organic layer thus obtained was concentrated and then purified through column chromatography to afford Compound <C-6>. (30.7g, 62.9%)

### Synthesis Example 19-3. Synthesis of C-7

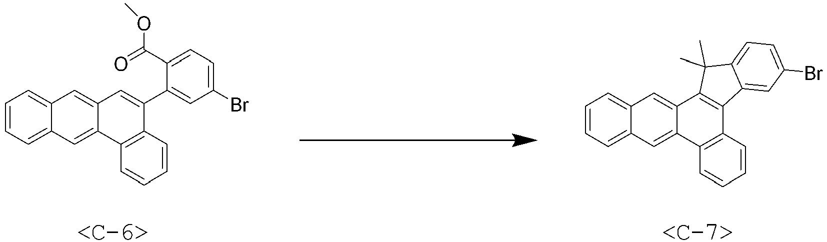

The same synthesis procedure as in Synthesis Example 1-3, with the exception of using <C-6> instead of <A-2>, was carried out to afford <C-7>. (yield 64.5%)

### Synthesis Example 19-4. Synthesis of C-8

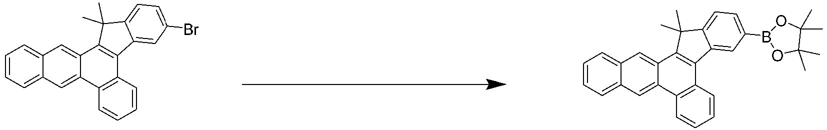

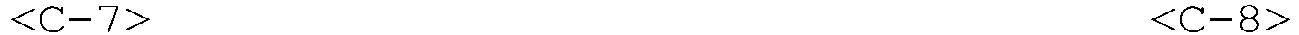

The same synthesis procedure as in Synthesis Example 1-1, with the exception of using <C-7> instead of <A-1a>, was carried out to afford <C-8>. (yield 75.4%)

### Synthesis Example 19-5. Synthesis of C-9

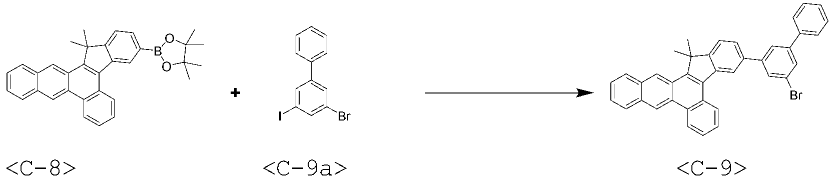

The same synthesis procedure as in Synthesis Example 1-2, with the exception of using <C-8> and <C-9a> instead of <A-1> and <A-2a>, respectively, was carried out to afford <C-9>. (yield 65.4%)

### Synthesis Example 19-6. Synthesis of D-1

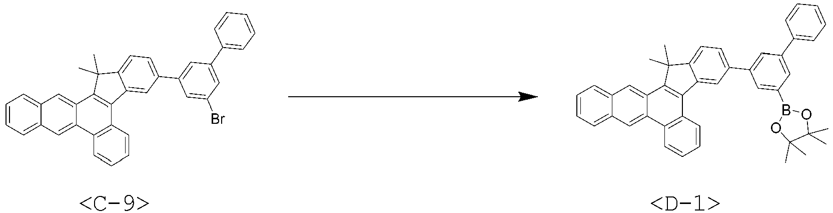

The same synthesis procedure as in Synthesis Example 1-1, with the exception of using <C-9> instead of <A-1a>, was carried out to afford <D-1>. (yield 78.1%)

### Synthesis Example 19-7. Synthesis of [Compound 1-25]

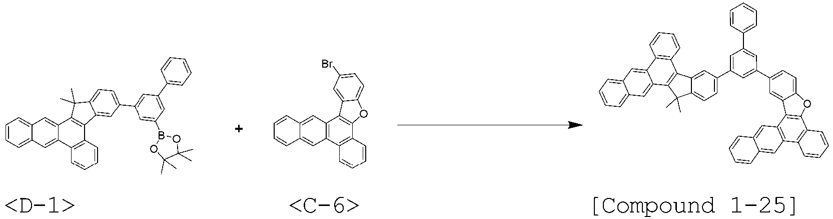

The same synthesis procedure as in Synthesis Example 1-2, with the exception of using <D-1> and <C-6> instead of <A-1> and <A-2a>, respectively, was carried out to afford [Compound 1-25]. (yield 65.4%)
MS (MALDI-TOF) : m/z 812.31 [M⁺]

### SYNTHESIS EXAMPLE 20. Synthesis of [Compound 1-26]

### Synthesis Example 20-1. Synthesis of D-2

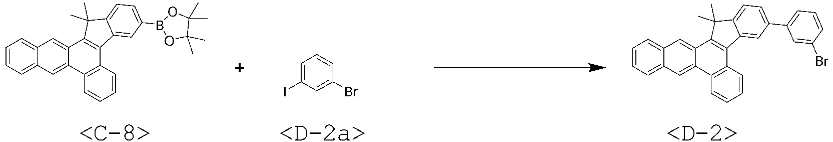

The same synthesis procedure as in Synthesis Example 1-2, with the exception of using <C-8> and <D-2a> instead of <A-1> and <A-2a>, respectively, was carried out to afford <D-2>. (yield 80.1%)

### Synthesis Example 20-2. Synthesis of D-3

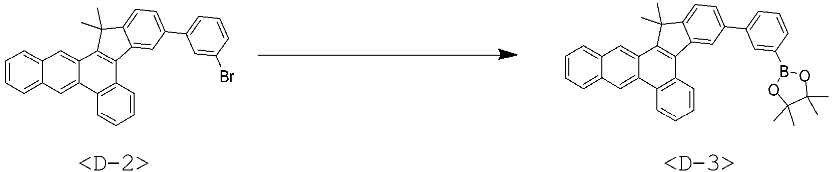

The same synthesis procedure as in Synthesis Example 1-1, with the exception of using <D-2> instead of <A-1a>, was carried out to afford <D-3>. (yield 77.3%)

### Synthesis Example 20-3. Synthesis of [Compound 1-26]

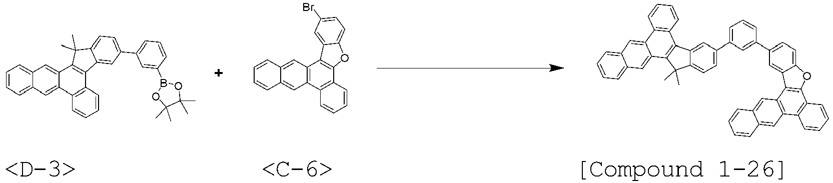

The same synthesis procedure as in Synthesis Example 1-2, with the exception of using <D-3> and <C-6> instead of <A-1> and <A-2a>, respectively, was carried out to afford [Compound 1-26]. (yield 67%)
MS (MALDI-TOF) : m/z 736.28 [M⁺]

### SYNTHESIS EXAMPLE 21. Synthesis of [Compound 1-34]

### Synthesis Example 21-1. Synthesis of D-4

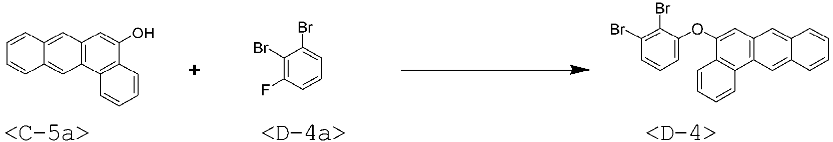

The same synthesis procedure as in Synthesis Example 19-1, with the exception of using <D-4a> instead of <C-5b>, was carried out to afford <D-4>. (yield 75.3%)

### Synthesis Example 21-2. Synthesis of D-5

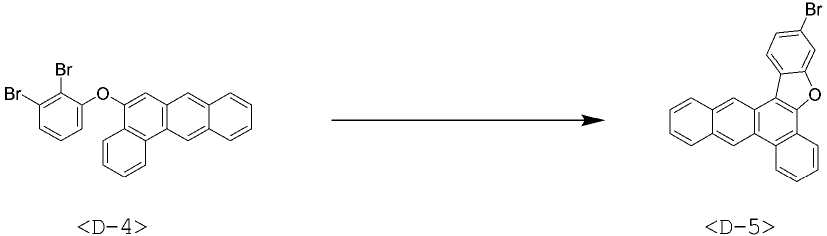

The same synthesis procedure as in Synthesis Example 19-2, with the exception of using <D-4> instead of <C-5>, was carried out to afford <D-5>. (yield 61.5%)

### Synthesis Example 21-3. Synthesis of [Compound 1-34]

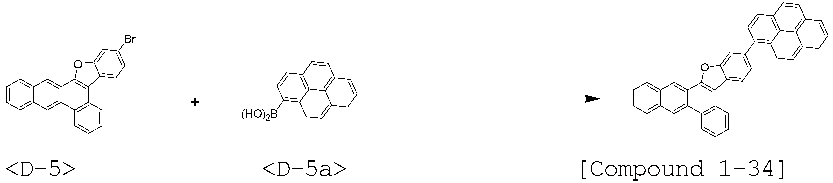

The same synthesis procedure as in Synthesis Example 1-2, with the exception of using <D-5a> and <D-5> instead of <A-1> and <A-2a>, respectively, was carried out to afford [Compound 1-34]. (yield 52.5%)
MS (MALDI-TOF) : m/z 520.18 [M⁺]

### SYNTHESIS EXAMPLE 22. Synthesis of [Compound 1-37]

### Synthesis Example 22-1. Synthesis of D-6

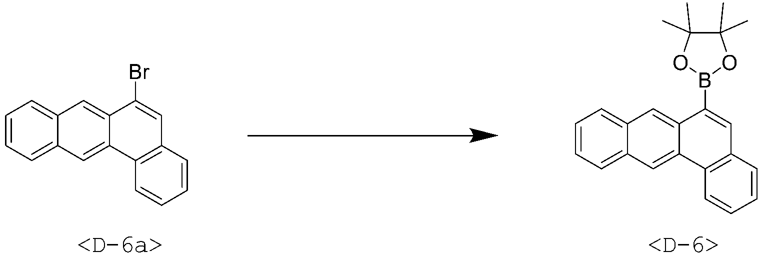

The same synthesis procedure as in Synthesis Example 1-1, with the exception of using <D-6a> instead of <A-1a>, was carried out to afford <D-6>. (yield 75.8%)

### Synthesis Example 22-2. Synthesis of D-7

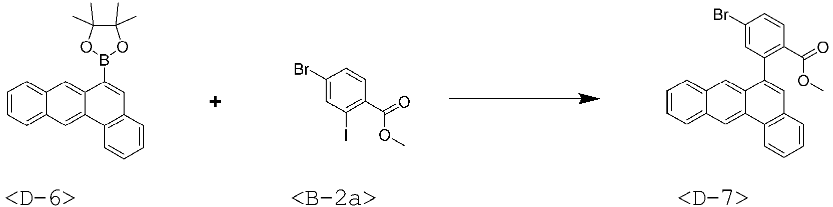

The same synthesis procedure as in Synthesis Example 1-2, with the exception of using <D-6> and <B-2a> instead of <A-1> and <A-2a>, was carried out to afford <D-7>. (yield 76.7%)

### Synthesis Example 22-3. Synthesis of D-8

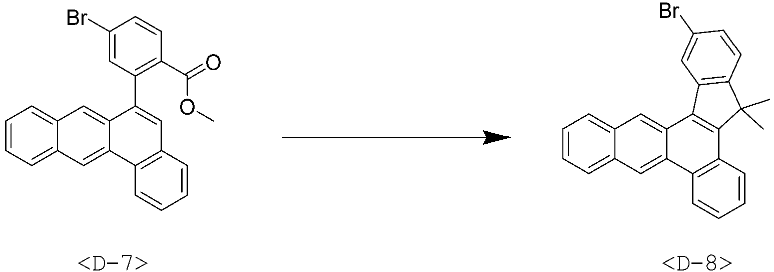

The same synthesis procedure as in Synthesis Example 1-3, with the exception of using <D-7> instead of <A-2>, was carried out to afford <D-8>. (yield 73.7%)

### Synthesis Example 22-4. Synthesis of [Compound 1-37]

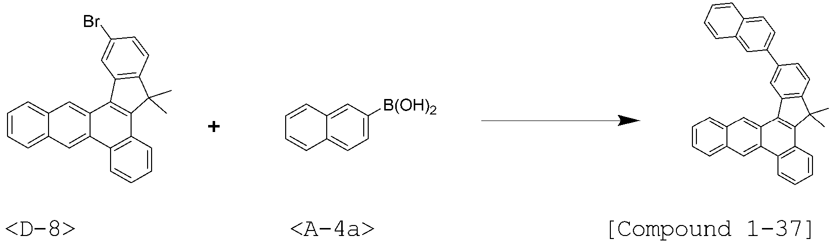

The same synthesis procedure as in Synthesis Example 1-2, with the exception of using <A-4a> and <D-8> instead of <A-1> and <A-2a>, respectively, was carried out to afford [Compound 1-37]. (yield 50.2%)
MS (MALDI-TOF) : m/z 470.20 [M⁺]

### SYNTHESIS EXAMPLE 23. Synthesis of [Compound 1-38]

### Synthesis Example 23-1. Synthesis of [Compound 1-38]

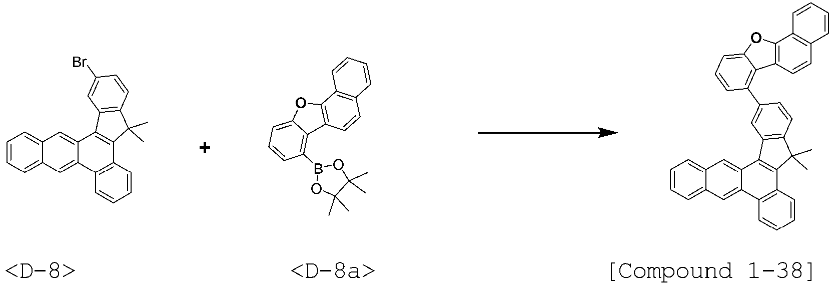

The same synthesis procedure as in Synthesis Example 1-2, with the exception of using <D-8a> and <D-8> instead of <A-1> and <A-2a>, respectively, was carried out to afford [Compound 1-38]. (yield 37.4%)
MS (MALDI-TOF) : m/z 560.21 [M⁺]

### SYNTHESIS EXAMPLE 24. Synthesis of [Compound 1-42]

### Synthesis Example 24-1. Synthesis of D-9

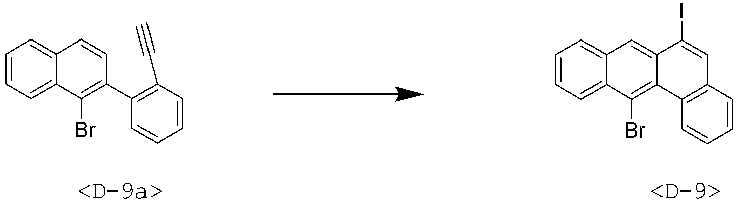

In a round-bottom flask, Compound <D-9a> (50 g) and DCM (500 mL) were stirred together. The mixture was chilled to -78°C before drops of iodochloride (31.7 g) were slowly added. After one hour, extraction with water was conducted, and the organic layer thus formed was concentrated and purified through column chromatography to afford Compound <D-9>. (60.8g, 86.3%)

### Synthesis Example 24-2. Synthesis of E-1

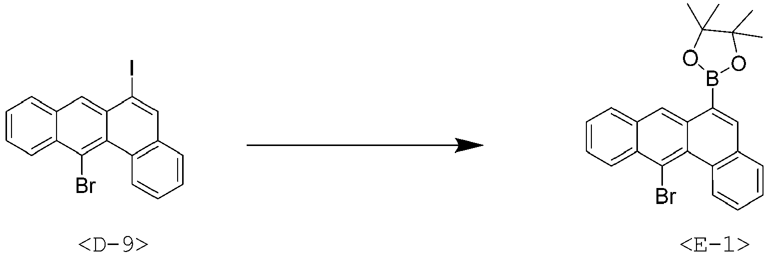

The same synthesis procedure as in Synthesis Example 1-1, with the exception of using <D-9> instead of <A-1a>, was carried out to afford <E-1>. (yield 76.7%)

### Synthesis Example 24-3. Synthesis of E-2

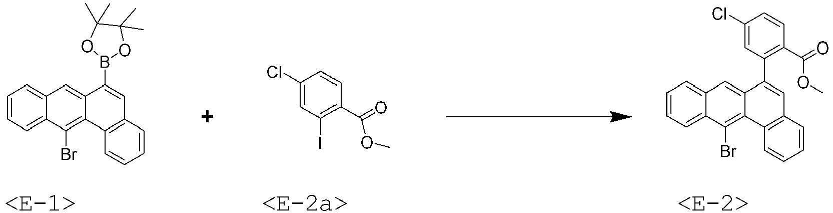

The same synthesis procedure as in Synthesis Example 1-2, with the exception of using <E-1> and <E-2a> instead of <A-1> and <A-2a>, respectively, was carried out to afford <E-2>. (yield 75.1%)

### Synthesis Example 24-4. Synthesis of E-3

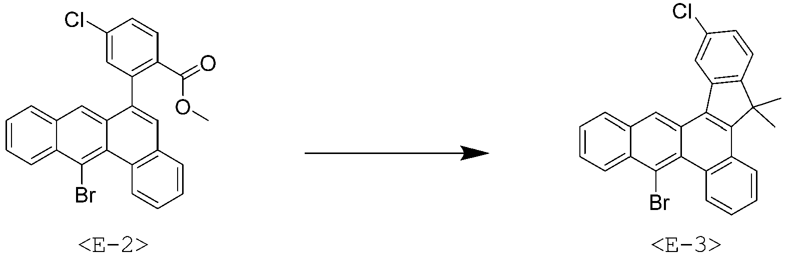

The same synthesis procedure as in Synthesis Example 1-3, with the exception of using <E-2> instead of <A-2>, was carried out to afford <E-3>. (yield 50.5%)

### Synthesis Example 24-5. Synthesis of E-4

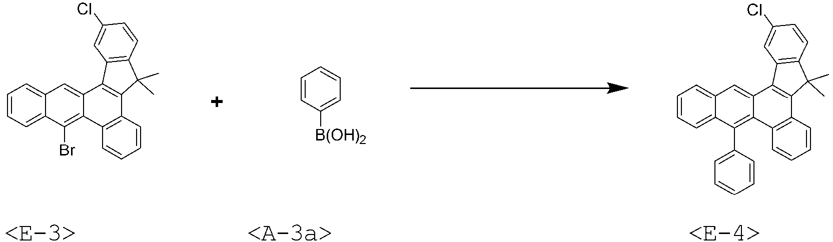

The same synthesis procedure as in Synthesis Example 1-2, with the exception of using <A-3a> and <E-3> instead of <A-1> and <A-2a>, respectively, was carried out to afford <E-4>. (yield 55.7%)

### Synthesis Example 24-6. Synthesis of [Compound 1-42]

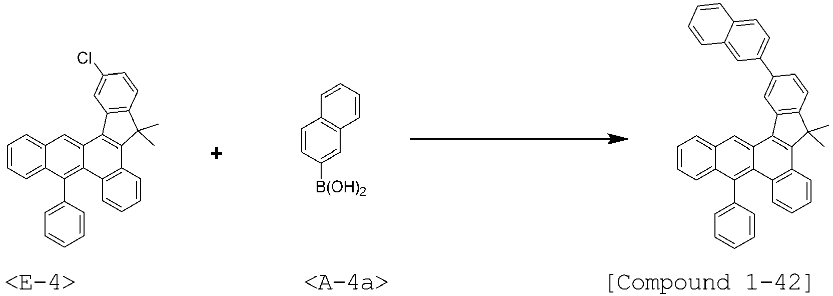

The same synthesis procedure as in Synthesis Example 1-2, with the exception of using <A-4a> and <E-4> instead of <A-1> and <A-2a>, respectively, was carried out to afford [Compound 1-42]. (yield 43.3%)
MS (MALDI-TOF) : m/z 546.23 [M⁺]

### SYNTHESIS EXAMPLE 25. Synthesis of [Compound 1-44]

### Synthesis Example 25-1. Synthesis of E-5

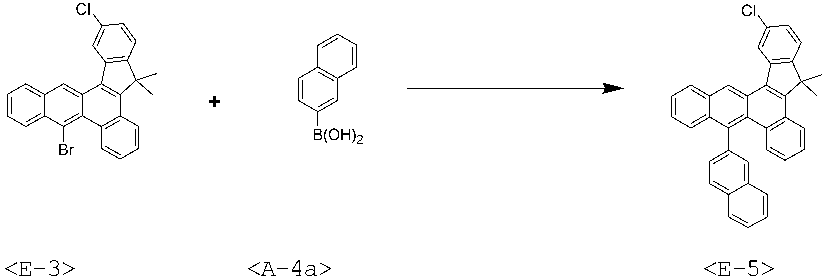

The same synthesis procedure as in Synthesis Example 1-2, with the exception of using <A-4a> and <E-3> instead of <A-1> and <A-2a>, respectively, was carried out to afford <E-5>. (yield 51.2%)

### Synthesis Example 25-2. Synthesis of [Compound 1-44]

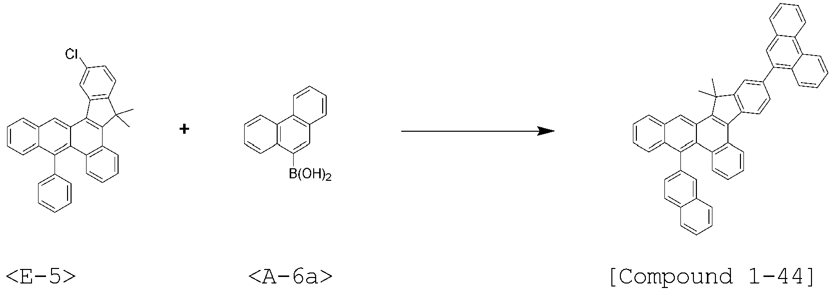

The same synthesis procedure as in Synthesis Example 1-2, with the exception of using <A-6a> and <E-5> instead of <A-1> and <A-2a>, respectively, was carried out to afford [Compound 1-44]. (yield 37.2%)
MS (MALDI-TOF) : m/z 646.27 [M⁺]

### SYNTHESIS EXAMPLE 26. Synthesis of [Compound 1-70]

### Synthesis Example 26-1. Synthesis of [Compound 1-70]

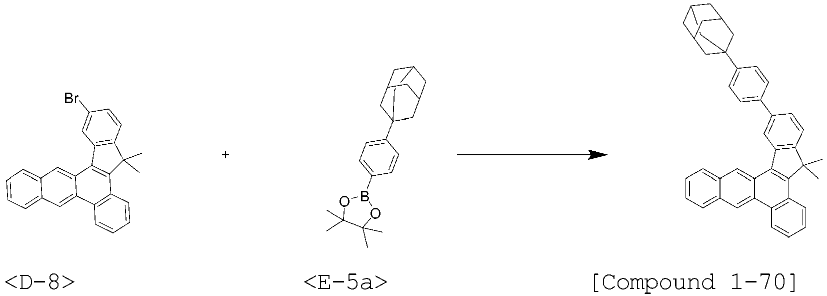

The same synthesis procedure as in Synthesis Example 1-2, with the exception of using <E-5a> and <D-8> instead of <A-1> and <A-2a>, respectively, was carried out to afford [Compound 1-70]. (yield 51.2%)
MS (MALDI-TOF) : m/z 554.30 [M⁺]

### SYNTHESIS EXAMPLE 27. Synthesis of [Compound 1-71]

### Synthesis Example 27-1. Synthesis of [Compound 1-71]

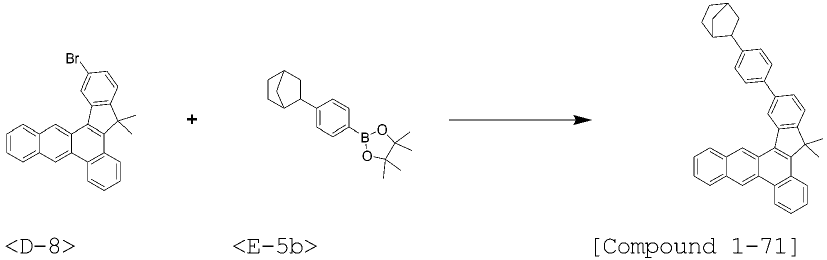

The same synthesis procedure as in Synthesis Example 1-2, with the exception of using <E-5b> and <D-8> instead of <A-1> and <A-2a>, respectively, was carried out to afford [Compound 1-71]. (yield 40.3%)
MS (MALDI-TOF) : m/z 514.27 [M⁺]

### SYNTHESIS EXAMPLE 28. Synthesis of [Compound 1-72]

### Synthesis Example 28-1. Synthesis of [Compound 1-72]

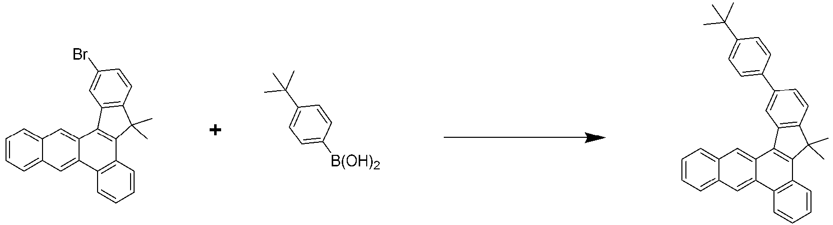

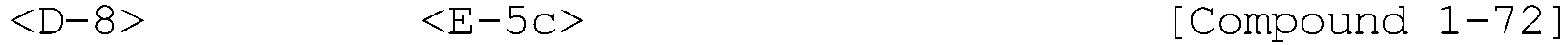

The same synthesis procedure as in Synthesis Example 1-2, with the exception of using <E-5c> and <D-8> instead of <A-1> and <A-2a>, respectively, was carried out to afford [Compound 1-72]. (yield 44.3%)
MS (MALDI-TOF) : m/z 476.25 [M⁺]

### SYNTHESIS EXAMPLE 29. Synthesis of [Compound 1-145]

### Synthesis Example 29-1. Synthesis of E-6

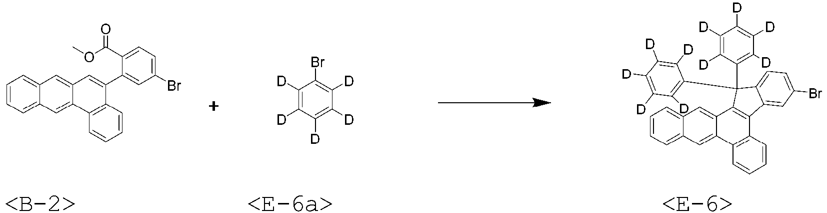

The same synthesis procedure as in Synthesis Example 9-2, with the exception of using <E-6a> instead of <B-3a>, was carried out to afford <E-6>. (yield 93.7%)

### Synthesis Example 29-2. Synthesis of [Compound 1-145]

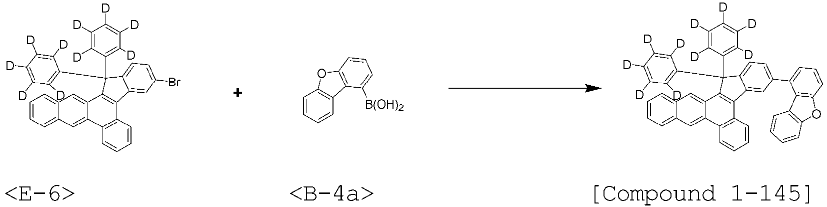

The same synthesis procedure as in Synthesis Example 1-2, with the exception of using <B-4a> and <E-6> instead of <A-1> and <A-2a>, respectively, was carried out to afford [Compound 1-145]. (yield 65.3%)
MS (MALDI-TOF) : m/z 644.29 [M⁺]

### SYNTHESIS EXAMPLE 30. Synthesis of [Compound 1-146]

### Synthesis Example 30-1. Synthesis of [Compound 1-146]

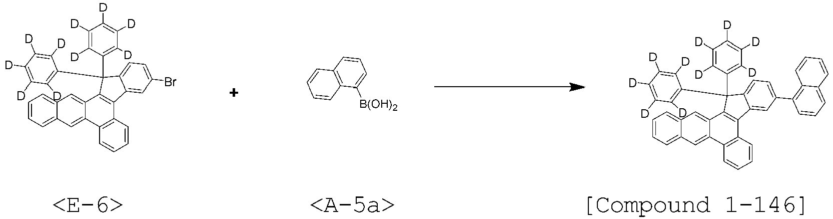

The same synthesis procedure as in Synthesis Example 1-2, with the exception of using <A-5a> and <E-6> instead of <A-1> and <A-2a>, respectively, was carried out to afford [Compound 1-146]. (yield 55.6%)
MS (MALDI-TOF) : m/z 604.30 [M⁺]

### SYNTHESIS EXAMPLE 31. Synthesis of [Compound 1-157]

### Synthesis Example 31-1. Synthesis of E-7

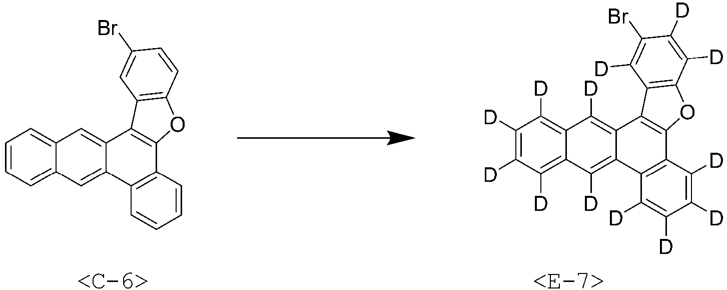

In a round-bottom flask, Compound <C-6> (10 g) and DCB (100 mL) were stirred together at room temperature to complete dissolution. Then, bezene-d6 (100 mL) was added and stirred at 10°C for 30 minutes and drops of trifluoromethanesulfonic acid (7.6 g) were slowly added while being stirred at 10°C for 6 hours. Thereafter, heavy water (200 mL) was added before stirring for 30 minutes. The organic layer thus formed was concentrated and purified through column chromatography to afford Compound <E-7>. (9.3g, 35.6%)

### Synthesis Example 31-2. Synthesis of [Compound 1-157]

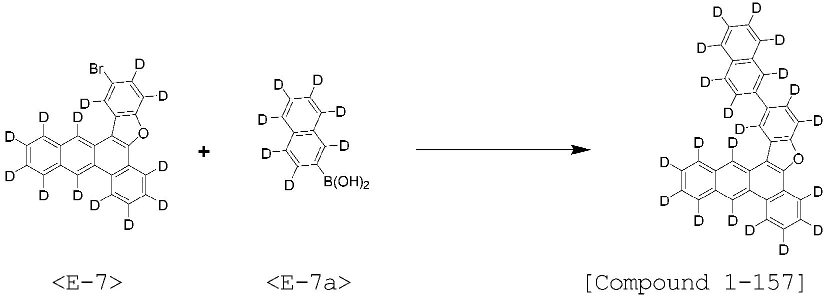

The same synthesis procedure as in Synthesis Example 1-2, with the exception of using <E-7a> and <E-7> instead of <A-1> and <A-2a>, respectively, was carried out to afford [Compound 1-157]. (yield 55.6%)
MS (MALDI-TOF) : m/z 464.28 [M⁺]

### SYNTHESIS EXAMPLE 32. Synthesis of [Compound 1-174]

### Synthesis Example 32-1 : Synthesis of [Compound 1-174]

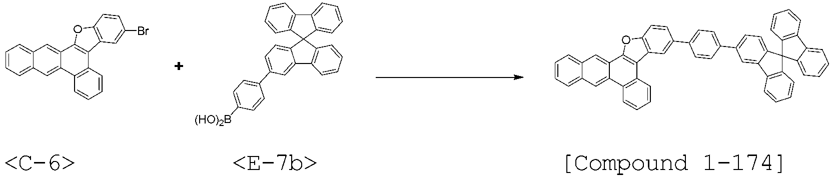

The same synthesis procedure as in Synthesis Example 1-2, with the exception of using <E-7b> and <C-6> instead of <A-1> and <A-2a>, respectively, was carried out to afford [Compound 1-174]. (yield 50.3%)
MS (MALDI-TOF) : m/z 708.25 [M⁺]

### SYNTHESIS EXAMPLE 33. Synthesis of [Compound 1-175]

### Synthesis Example 33-1 : Synthesis of [Compound 1-175]

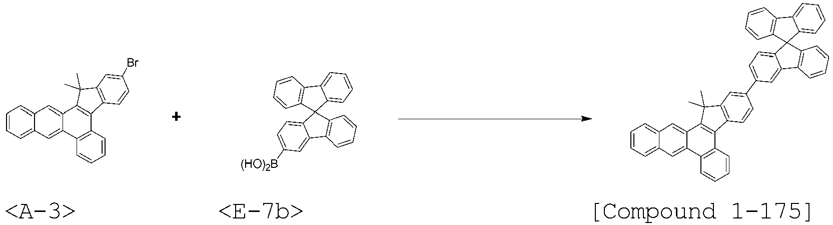

The same synthesis procedure as in Synthesis Example 1-2, with the exception of using <E-7b> and <A-3> instead of <A-1> and <A-2a>, respectively, was carried out to afford [Compound 1-175]. (yield 45.7%)
MS (MALDI-TOF) : m/z 658.27 [M⁺]

### SYNTHESIS EXAMPLE 34. Synthesis of [Compound 1-178]

### Synthesis Example 34-1 : Synthesis of [Compound 1-178]

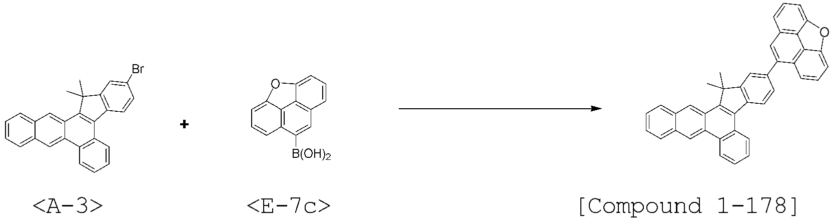

The same synthesis procedure as in Synthesis Example 1-2, with the exception of using <E-7c> and <A-3> instead of <A-1> and <A-2a>, respectively, was carried out to afford [Compound 1-178]. (yield 48.4%)
MS (MALDI-TOF) : m/z 534.20 [M⁺]

### EXAMPLES 1 TO 82 : Fabrication of Organic Light-Emitting Diodes Including 1^{st} and 2^{nd} Emission Layers

An ITO glass substrate was patterned to have a translucent area of 2 mm x 2 mm and cleansed. The ITO glass was mounted in a vacuum chamber that was then set to have a base pressure of 1x10⁻⁷ torr. On the ITO glass substrate, a hole injection layer was formed (100 Å) with the electron acceptor of the following Structural Formula [Acceptor-1] and [Chemical Formula F] such that the deposition ratio is [Acceptor-1] : [Chemical Formula F] = 2 : 98. As a hole transport layer, [Chemical Formula F] was deposited (550 Å), followed by forming an electron blocking layer with [Chemical Formula G] (50 Å). Then, a first emission layer and a second emission layer were sequentially formed as emission layers, wherein the first emission layer was established by forming a film (50 Å) from a combination of a compound represented by any one of Chemical Formulas 1 to 4 according to the present disclosure and a polycyclic compound (1 wt%) selected from the following [BD-1] to [BD-3] and the second emission layer was established by forming a film (150 Å) from a combination of an anthracene compound represented by the [BH-1] or [BH-2] and a polycyclic compound (1 wt%) selected from the following [BD-1] to [BD-3]. Thereafter, films were formed from [Chemical Formula H] for a hole barrier layer (50 Å), from a mixture of 1:1 of Chemical Formulas [E-1] and [E-2] for an electron transport layer (250 Å), from [E-2] for an electron injection layer (10 Å), and from Al (1000 Å) for a cathode in the order, thereby fabricating an organic light-emitting diode. The organic light-emitting diodes thus obtained were measured at 0.4 mA for luminescence properties:

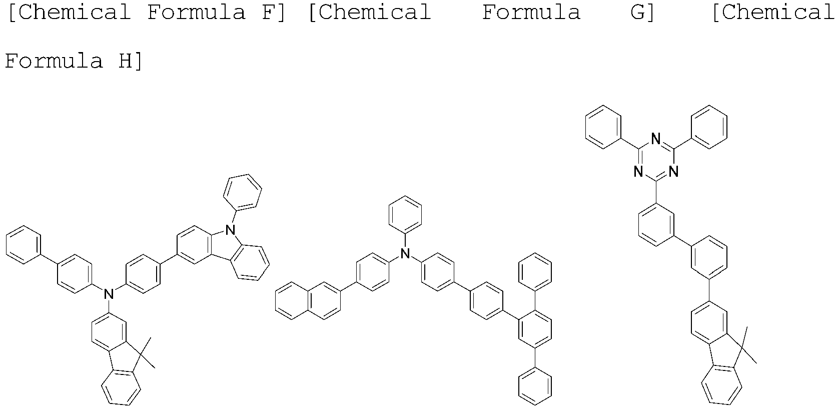

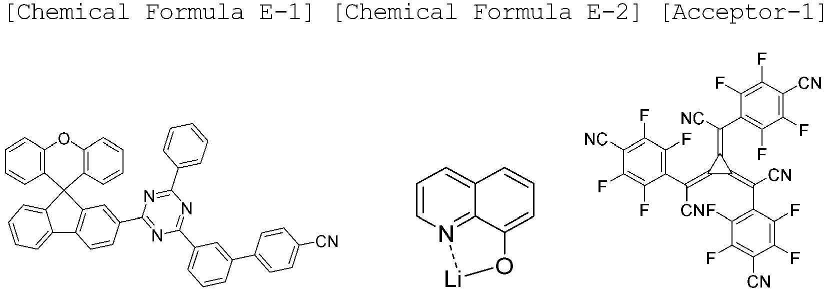

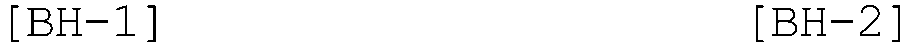

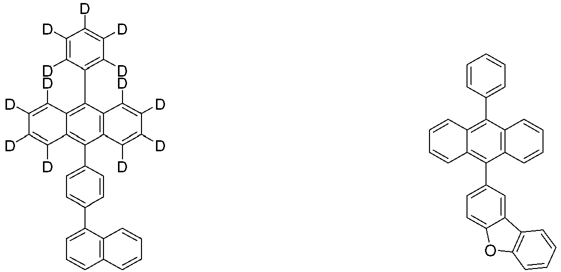

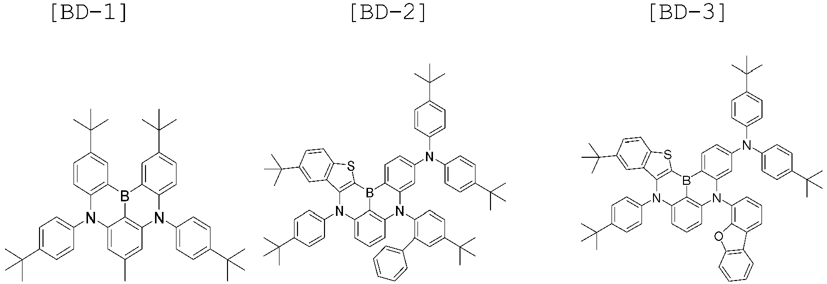

### COMPARATIVE EXAMPLES 1 TO 18

Organic light-emitting diodes for the Comparative Examples were fabricated in the same manner as in the Examples, with the exception of using the following [RH-1] to [RH-4], instead of the compounds according to the present disclosure, as hosts in the first and second emission layers. The OLEDs thus obtained were measured at 0.4 mA for luminescence properties. Here, the structures of [RH-1] to [RH-3] are as follows:

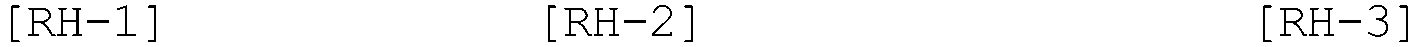

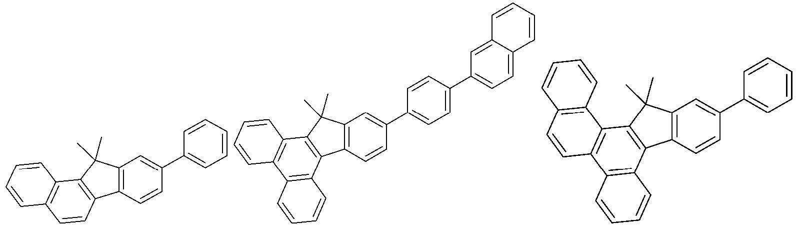

**[TABLE 1]**

| Ex. No. | 1^{st} Emission layer | | 2^{nd} Emission layer | | Driving volt. (V) | External quantum efficiency (EQE, %) | Lifetime (LT97, hr ) |
|---|---|---|---|---|---|---|---|
| | Host | Dopant | Host | Dopant | | | |
| Ex. 1 | 1-1 | BD-1 | BH-1 | BD-1 | 3.0 | 12.1 | 350 |
| Ex. 2 | 1-2 | BD-1 | BH-1 | BD-1 | 3.0 | 12.2 | 355 |
| Ex. 3 | 1-4 | BD-1 | BH-1 | BD-1 | 3.1 | 12.3 | 340 |
| Ex. 4 | 1-5 | BD-1 | BH-1 | BD-1 | 3.0 | 12.3 | 338 |
| Ex. 5 | 1-7 | BD-1 | BH-1 | BD-1 | 3.1 | 12.4 | 347 |
| Ex. 6 | 1-8 | BD-1 | BH-1 | BD-1 | 3.1 | 12.5 | 346 |
| Ex. 7 | 1-9 | BD-1 | BH-1 | BD-1 | 3.1 | 12.4 | 345 |
| Ex. 8 | 1-10 | BD-1 | BH-1 | BD-1 | 3.2 | 12.7 | 340 |
| Ex. 9 | 1-11 | BD-1 | BH-1 | BD-1 | 3.2 | 12.3 | 337 |
| Ex. 10 | 1-16 | BD-1 | BH-1 | BD-1 | 3.1 | 12.3 | 341 |
| Ex. 11 | 1-17 | BD-1 | BH-1 | BD-1 | 3.2 | 12.4 | 330 |
| Ex. 12 | 1-18 | BD-1 | BH-1 | BD-1 | 3.1 | 12.4 | 339 |
| Ex. 13 | 1-25 | BD-1 | BH-1 | BD-1 | 3.3 | 12.6 | 327 |
| Ex. 14 | 1-26 | BD-1 | BH-1 | BD-1 | 3.3 | 12.7 | 325 |
| Ex. 15 | 1-27 | BD-1 | BH-1 | BD-1 | 3.3 | 12.5 | 330 |
| Ex. 16 | 1-34 | BD-1 | BH-1 | BD-1 | 3.2 | 12.7 | 335 |
| Ex. 17 | 1-37 | BD-1 | BH-1 | BD-1 | 3.0 | 12.3 | 346 |
| Ex. 18 | 1-38 | BD-1 | BH-1 | BD-1 | 3.1 | 12.7 | 350 |
| Ex. 19 | 1-40 | BD-1 | BH-1 | BD-1 | 3.1 | 12.8 | 340 |
| Ex. 20 | 1-41 | BD-1 | BH-1 | BD-1 | 3.1 | 12.8 | 347 |
| Ex. 21 | 1-42 | BD-1 | BH-1 | BD-1 | 3.1 | 12.9 | 346 |
| Ex. 22 | 1-44 | BD-1 | BH-1 | BD-1 | 3.2 | 13.0 | 345 |
| Ex. 23 | 1-70 | BD-1 | BH-1 | BD-1 | 3.0 | 12.5 | 337 |
| Ex. 24 | 1-72 | BD-1 | BH-1 | BD-1 | 3.0 | 12.3 | 350 |
| Ex. 25 | 1-145 | BD-1 | BH-1 | BD-1 | 3.2 | 13.1 | 360 |
| Ex. 26 | 1-54 | BD-1 | BH-1 | BD-1 | 3.3 | 13.3 | 365 |
| Ex. 27 | 1-157 | BD-1 | BH-1 | BD-1 | 3.1 | 13.2 | 370 |
| Ex. 28 | 1-174 | BD-1 | BH-1 | BD-1 | 3.1 | 13.2 | 365 |
| Ex. 29 | 1-2 | BD-1 | BH-2 | BD-1 | 3.0 | 12.3 | 365 |
| Ex. 30 | 1-4 | BD-1 | BH-2 | BD-1 | 3.1 | 12.6 | 361 |
| Ex. 31 | 1-8 | BD-1 | BH-2 | BD-1 | 3.1 | 12.9 | 360 |
| Ex. 32 | 1-10 | BD-1 | BH-2 | BD-1 | 3.2 | 13.0 | 358 |
| Ex. 33 | 1-12 | BD-1 | BH-2 | BD-1 | 3.2 | 12.9 | 355 |
| Ex. 34 | 1-18 | BD-1 | BH-2 | BD-1 | 3.3 | 12.7 | 360 |
| Ex. 35 | 1-40 | BD-1 | BH-2 | BD-1 | 3.2 | 12.9 | 355 |
| Ex. 36 | 1-42 | BD-1 | BH-2 | BD-1 | 3.1 | 13.0 | 360 |
| Ex. 37 | 1-44 | BD-1 | BH-2 | BD-1 | 3.2 | 13.0 | 362 |
| Ex. 38 | 1-70 | BD-1 | BH-2 | BD-1 | 3.1 | 12.4 | 367 |
| Ex. 39 | 1-146 | BD-1 | BH-2 | BD-1 | 3.2 | 13.0 | 368 |
| Ex. 40 | 1-145 | BD-1 | BH-2 | BD-1 | 3.2 | 13.0 | 373 |
| Ex. 41 | 1-154 | BD-1 | BH-2 | BD-1 | 3.2 | 13.0 | 378 |
| Ex. 42 | 1-157 | BD-1 | BH-2 | BD-1 | 3.1 | 12.9 | 380 |
| Ex. 43 | 1-174 | BD-1 | BH-2 | BD-1 | 3.2 | 13.1 | 370 |
| Ex. 44 | 1-3 | BD-2 | BH-1 | BD-2 | 3.0 | 12.5 | 350 |
| Ex. 45 | 1-12 | BD-2 | BH-1 | BD-2 | 3.2 | 12.9 | 342 |
| Ex. 46 | 1-13 | BD-2 | BH-1 | BD-2 | 3.1 | 12.6 | 348 |
| Ex. 47 | 1-14 | BD-2 | BH-1 | BD-2 | 3.2 | 12.7 | 356 |
| Ex. 48 | 1-15 | BD-2 | BH-1 | BD-2 | 3.1 | 12.3 | 335 |
| Ex. 49 | 1-19 | BD-2 | BH-1 | BD-2 | 3.0 | 12.9 | 340 |
| Ex. 50 | 1-20 | BD-2 | BH-1 | BD-2 | 3.2 | 12.8 | 341 |
| Ex. 51 | 1-21 | BD-2 | BH-1 | BD-2 | 3.2 | 12.8 | 340 |
| Ex. 52 | 1-31 | BD-2 | BH-1 | BD-2 | 3.2 | 13.0 | 335 |
| Ex. 53 | 1-32 | BD-2 | BH-1 | BD-2 | 3.1 | 13.1 | 342 |
| Ex. 54 | 1-33 | BD-2 | BH-1 | BD-2 | 3.1 | 12.8 | 345 |
| Ex. 55 | 1-71 | BD-2 | BH-1 | BD-2 | 3.2 | 12.5 | 348 |
| Ex. 56 | 1-72 | BD-2 | BH-1 | BD-2 | 3.0 | 12.4 | 346 |
| Ex. 57 | 1-146 | BD-2 | BH-1 | BD-2 | 3.0 | 13.1 | 352 |
| Ex. 58 | 1-1 | BD-2 | BH-2 | BD-2 | 3.1 | 12.6 | 361 |
| Ex. 59 | 1-5 | BD-2 | BH-2 | BD-2 | 3.1 | 12.7 | 365 |
| Ex. 60 | 1-9 | BD-2 | BH-2 | BD-2 | 3.2 | 12.9 | 363 |
| Ex. 61 | 1-10 | BD-2 | BH-2 | BD-2 | 3.3 | 13.1 | 356 |
| Ex. 62 | 1-11 | BD-2 | BH-2 | BD-2 | 3.3 | 13.0 | 358 |
| Ex. 63 | 1-25 | BD-2 | BH-2 | BD-2 | 3.2 | 12.8 | 348 |
| Ex. 64 | 1-26 | BD-2 | BH-2 | BD-2 | 3.2 | 12.8 | 345 |
| Ex. 65 | 1-27 | BD-2 | BH-2 | BD-2 | 3.2 | 12.7 | 336 |
| Ex. 66 | 1-37 | BD-2 | BH-2 | BD-2 | 3.1 | 12.6 | 360 |
| Ex. 67 | 1-38 | BD-2 | BH-2 | BD-2 | 3.2 | 12.9 | 355 |
| Ex. 68 | 1-40 | BD-2 | BH-2 | BD-2 | 3.2 | 12.5 | 355 |
| Ex. 69 | 1-41 | BD-2 | BH-2 | BD-2 | 3.1 | 12.7 | 345 |
| Ex. 70 | 1-42 | BD-2 | BH-2 | BD-2 | 3.2 | 12.8 | 345 |
| Ex. 71 | 1-70 | BD-2 | BH-2 | BD-2 | 3.1 | 12.6 | 348 |
| Ex. 72 | 1-4 | BD-3 | BH-1 | BD-3 | 3.1 | 12.6 | 360 |
| Ex. 73 | 1-8 | BD-3 | BH-1 | BD-3 | 3.1 | 12.9 | 355 |
| Ex. 74 | 1-10 | BD-3 | BH-1 | BD-3 | 3.2 | 13.1 | 345 |
| Ex. 75 | 1-12 | BD-3 | BH-1 | BD-3 | 3.2 | 13.0 | 348 |
| Ex. 76 | 1-145 | BD-3 | BH-1 | BD-3 | 3.2 | 13.1 | 380 |
| Ex. 77 | 1-4 | BD-3 | BH-2 | BD-3 | 3.1 | 12.9 | 375 |
| Ex. 78 | 1-10 | BD-3 | BH-2 | BD-3 | 3.2 | 13.1 | 360 |
| Ex. 79 | 1-145 | BD-3 | BH-2 | BD-3 | 3.2 | 13.1 | 375 |
| Ex. 80 | 1-154 | BD-3 | BH-2 | BD-3 | 3.2 | 13.0 | 396 |
| Ex. 81 | 1-157 | BD-3 | BH-2 | BD-3 | 3.1 | 13.1 | 420 |
| Ex. 82 | 1-178 | BD-3 | BH-2 | BD-3 | 3.1 | 13.1 | 380 |
| C. Ex. 1 | RH-1 | BD-1 | BH-1 | BD-1 | 3.5 | 11.5 | 130 |
| C. Ex. 2 | RH-2 | BD-1 | BH-1 | BD-1 | 3.4 | 12.0 | 123 |
| C. Ex. 3 | RH-3 | BD-1 | BH-1 | BD-1 | 3.4 | 11.0 | 120 |
| C. Ex. 4 | RH-1 | BD-1 | BH-2 | BD-1 | 3.3 | 9.1 | 150 |
| C. Ex. 5 | RH-2 | BD-1 | BH-2 | BD-1 | 3.4 | 9.5 | 110 |
| C. Ex. 6 | RH-3 | BD-1 | BH-2 | BD-1 | 3.4 | 9.8 | 140 |
| C. Ex. 7 | RH-1 | BD-2 | BH-1 | BD-2 | 3.5 | 9.8 | 140 |
| C. Ex. 8 | RH-2 | BD-2 | BH-1 | BD-2 | 3.4 | 9.6 | 150 |
| C. Ex. 9 | RH-3 | BD-2 | BH-1 | BD-2 | 3.4 | 9.9 | 110 |
| C. Ex. 10 | RH-1 | BD-2 | BH-2 | BD-2 | 3.3 | 10.0 | 180 |
| C. Ex. 11 | RH-2 | BD-2 | BH-2 | BD-2 | 3.4 | 10.4 | 200 |
| C. Ex. 12 | RH-3 | BD-2 | BH-2 | BD-2 | 3.4 | 10.3 | 200 |
| C. Ex. 13 | RH-1 | BD-3 | BH-1 | BD-3 | 3.5 | 9.7 | 110 |
| C. Ex. 14 | RH-2 | BD-3 | BH-1 | BD-3 | 3.3 | 10.0 | 130 |
| C. Ex. 15 | RH-3 | BD-3 | BH-1 | BD-3 | 3.3 | 19.8 | 120 |
| C. Ex. 16 | RH-1 | BD-3 | BH-2 | BD-3 | 3.3 | 10.1 | 140 |
| C. Ex. 17 | RH-2 | BD-3 | BH-2 | BD-3 | 3.2 | 10.4 | 150 |
| C. Ex. 18 | RH-3 | BD-3 | BH-2 | BD-3 | 3.3 | 10.5 | 110 |

As shown in Table 1, it was confirmed that the organic light-emitting diodes employing the compounds according to the present disclosure in the first emission layer and the second emission layer exhibited high efficiency and long lifetime characteristics, with improved luminous efficiency and device lifetime at lower driving voltages, in comparison to the OLEDs using comparative compounds according to conventional technologies (Comparative Examples 1 to 18).

In addition, when compared to the OLEDs (Comparative Examples 11 to 16) using single emission layers having the same total thickness as the combined thicknesses of the first and second emission layers of the OLEDs according to the present disclosure, rather than a two-layered emission structure, it was confirmed that the OLEDs of the present disclosure exhibited significantly superior luminous efficiency and lifetime characteristics in all aspects. This is presumed to be due to the efficient energy transfer enabled by the formation of excitons in the first light-emitting and their subsequent transfer to the second emission layer, where triplet-triplet fusion occurs, in the multi-layered emission structure of the present disclosure, thereby providing high efficiency and long lifetime.

In contrast, in the case of a device employing a single emission layer, as in the comparative examples, all emission processes must occur within a single layer, which reduces the probability of triplet-triplet fusion. This, in turn, is presumed to lead to device degradation, resulting in reduced efficiency and lifetime. Therefore, it was confirmed that it is easier to fabricate a high-efficiency, long-lifetime OLED by using both the first and second emission layers, compared to a single emission layer.

### EXAMPLES 83 TO 110 : Fabrication of Organic Light-Emitting Diodes Including 1^{st} and 2^{nd} Emission Layers

Organic light-emitting diodes with first and second emission layers are fabricated in the same manner as in Examples 1 to 82, with the exception that either or both of the emission layers employed two compounds of the following [BH-3] to [BH-7] as a host and each of the emission layers employed the following [BD-4] as a dopant. Here, the structures of [BH-3] to [BH-7] and [BD-4] are as follows and the OLEDs thus obtained were measured at 0.4 mA for luminescence properties.

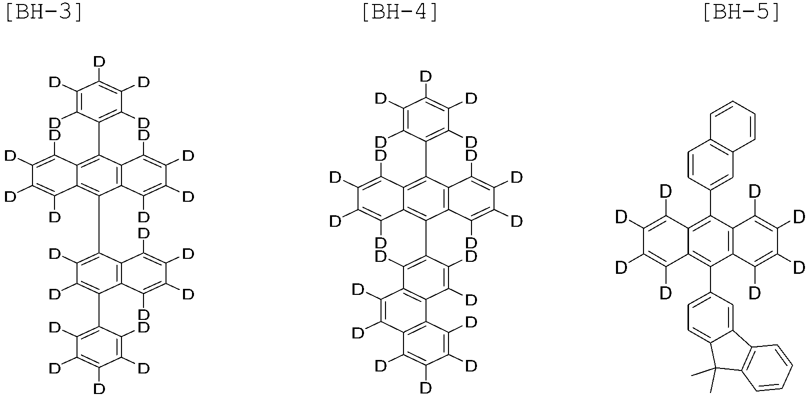

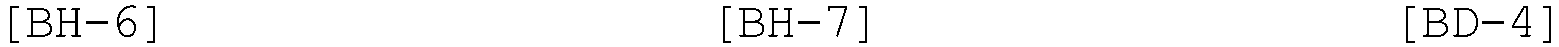

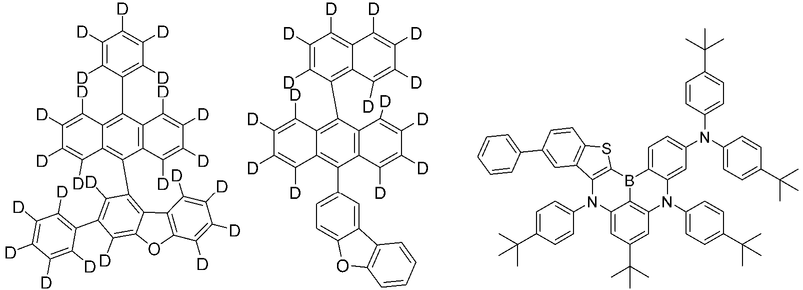

**[TABLE 2]**

| Ex. No. | 1^{st} Emission layer | | 2^{nd} Emission layer | | Driving volt. (V) | External quantum efficiency (EQE, %) | Lifetime (LT97,hr) |
|---|---|---|---|---|---|---|---|
| | Host | | Host | | | | |
| Ex. 83 | 1-1 | | BH-3 | BH-6 | 3.3 | 12.7 | 360 |
| Ex. 84 | 1-2 | | BH-3 | BH-6 | 3.3 | 12.7 | 360 |
| Ex. 85 | 1-26 | | BH-3 | BH-6 | 3.4 | 13.0 | 354 |
| Ex. 86 | 1-42 | | BH-3 | BH-6 | 3.4 | 12.8 | 350 |
| Ex. 87 | 1-70 | | BH-3 | BH-6 | 3.3 | 12.9 | 360 |
| Ex. 88 | 1-174 | | BH-3 | BH-6 | 3.3 | 13.1 | 365 |
| Ex. 89 | 1-11 | | BH-4 | BH-6 | 3.3 | 12.8 | 354 |
| Ex. 90 | 1-34 | | BH-4 | BH-6 | 3.3 | 12.9 | 355 |
| Ex. 91 | 1-157 | | BH-5 | BH-6 | 3.3 | 13.0 | 330 |
| Ex. 92 | 1-175 | | BH-5 | BH-6 | 3.3 | 13.1 | 357 |
| Ex. 93 | 1-11 | | BH-3 | BH-4 | 3.3 | 13.0 | 348 |
| Ex. 94 | 1-38 | | BH-3 | BH-5 | 3.3 | 13.1 | 359 |
| Ex. 95 | 1-42 | | BH-3 | BH-7 | 3.4 | 12.9 | 355 |
| Ex. 96 | 1-38 | | BH-4 | BH-7 | 3.2 | 13.0 | 360 |
| Ex. 97 | 1-70 | | BH-5 | BH-7 | 3.3 | 13.2 | 353 |
| Ex. 98 | 1-178 | | BH-6 | BH-7 | 3.4 | 12.8 | 350 |
| Ex. 99 | 1-1 | 1-70 | BH-3 | | 3.2 | 12.7 | 365 |
| Ex. 100 | 1-2 | 1-175 | BH-4 | | 3.4 | 13.0 | 350 |
| Ex. 101 | 1-5 | 1-11 | BH-5 | | 3.4 | 12.9 | 346 |
| Ex. 102 | 1-1 | 1-174 | BH-6 | | 3.2 | 13.1 | 363 |
| Ex. 103 | 1-42 | 1-175 | BH-7 | | 3.4 | 12.9 | 350 |
| Ex. 104 | 1-5 | 1-34 | BH-3 | BH-4 | 3.3 | 13.0 | 360 |
| Ex. 105 | 1-11 | 1-38 | BH-3 | BH-5 | 3.4 | 13.2 | 348 |
| Ex. 106 | 1-42 | 1-70 | BH-4 | BH-6 | 3.4 | 12.9 | 346 |
| Ex. 107 | 1-157 | 1-174 | BH-5 | BH-7 | 3.4 | 13.1 | 365 |
| Ex. 108 | 1-13 | 1-175 | BH-3 | BH-6 | 3.3 | 13.1 | 345 |
| Ex. 109 | 1-70 | 1-174 | BH-5 | BH-6 | 3.3 | 13.2 | 368 |
| Ex. 110 | 1-174 | 1-175 | BH-6 | BH-7 | 3.3 | 13.2 | 340 |

As shown in Table 2, it was confirmed that the organic light-emitting diodes comprising two or more different compounds as a host in at least one of the first emission layer and the second emission layer of the organic light-emitting diodes according to the present disclosure exhibited superior characteristics in terms of low driving voltage, luminous efficiency, and device lifetime.

## Claims

1. A fused cyclic compound represented by any one of the following Chemical Formulas 1 to 4: 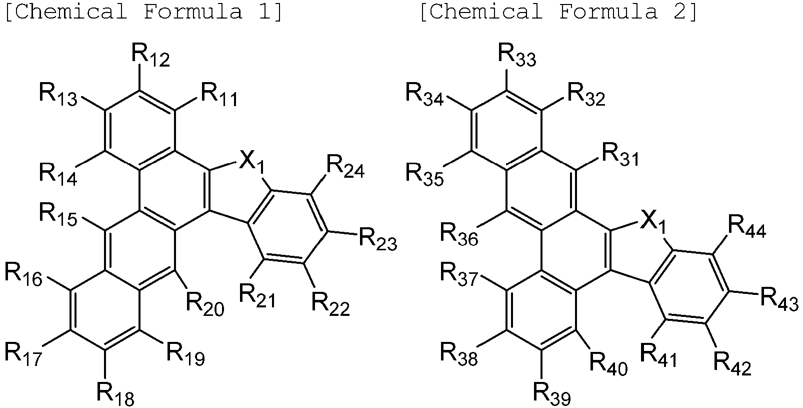 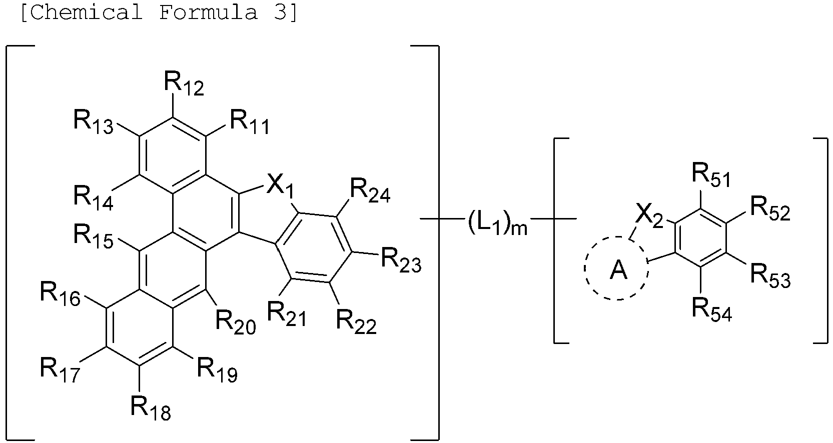 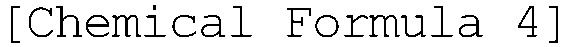 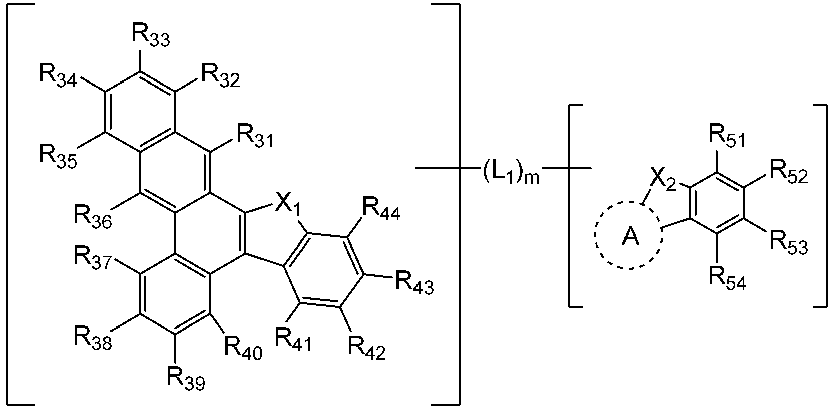 wherein,
X₁ and X₂, which are same or different, are each independently any one of O, S, and CR₁R₂,
R₁, R₂, R₁₁ to R₂₄, R₃₁ to R₄₄, R₅₁ to R₅₄, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₁ and R₂ may be linked to each other to additionally form a mono- or polycyclic aliphatic or aromatic ring,
adjacent substituents in each of the R₁₁ to R₂₄ and the R₃₁ to R₄₄ may be linked to each other to additionally form a mono- or polycyclic aliphatic or aromatic ring,
in Chemical Formulas 3 and 4,
the ring moiety A is a substituted or unsubstituted hydrocarbon ring of 6 to 30 carbon atoms,
linkers L₁, which are same or different, are each independently any one selected from a single bond, a substituted or unsubstituted arylene of 6 to 30 carbon atoms, a substituted or unsubstituted heteroarylene of 3 to 30 carbon atoms, and a substituted or unsubstituted, aliphatic hydrocarbon ring-fused arylene of 8 to 30 carbon atoms,
m is an integer of 1 to 3 wherein when m is 2 or more, the corresponding L₁ are same or different,
any one of R₁₁ to R₂₄, and any one of R₅₁ to R₅₄ and the substituents bonded to the carbon atoms as ring members of the ring moiety A in Chemical Formula 3 are each a single bond to L₁,
any one of R₃₁ to R₄₄, and any one of R₅₁ to R₅₄ and the substituents bonded to the carbon atoms as ring members of the ring moiety A in Chemical Formula 4 are each a single bond to L₁,
wherein the term "substituted" in the expression "a substituted or unsubstituted" used for the compounds of Chemical Formula 1 to Chemical Formula 4 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, and a thionyl of 1 to 30 carbon atoms, with at least one hydrogen atom on the substituent being substitutable with a deuterium or tritium atom.

2. The fused cyclic compound of claim 1, wherein the substituents R₁ and R₂ in Chemical Formulas 1 to 4 are identical to or different from each other and are each independently any one selected from a substituted or unsubstituted alkyl of 1 to 14 carbon atoms, a substituted or unsubstituted halogenated alkyl of 1 to 14 carbon atoms, a substituted or unsubstituted aryl of 6 to 20 carbon atoms, and a substituted or unsubstituted aliphatic hydrocarbon ring-fused aryl of 7 to 20 carbon atoms.

3. The fused cyclic compound of claim 1, wherein the substituents R₁, R₂, R₁₁ to R₂₄, R₃₁ to R₄₄, R₅₁ to R₅₄ in Chemical Formulas 1 to 4 are identical to or different from each other and are each independently any one selected from a hydrogen atom, a deuterium atom, a substituted or unsubstituted aryl of 6 to 30 carbon atoms, a substituted or unsubstituted heteroaryl of 2 to 30 carbon atoms, a substituted or unsubstituted cycloalkyl of 3 to 30 carbon atoms, a substituted or unsubstituted aliphatic hydrocarbon ring-fused aryl of 7 to 30 carbon atoms, a substituted or unsubstituted silyl of 1 to 30 carbon atoms, and a substituted or unsubstituted germanium of 1 to 30 carbon atoms.

4. The fused cyclic compound of claim 3, wherein
at least one of R₁₁ to R₂₄ in Chemical Formula 1 is any one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms;
at least one of R₃₁ to R₄₄ in Chemical Formula 2 is any one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms;
at least one of R₁₁ to R₂₄ and R₅₁ to R₅₄ in Chemical Formula 3 is any one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms; and
at least one of R₃₁ to R₄₄ and R₅₁ to R₅₄ in Chemical Formula 4 is any one selected from a substituted or unsubstituted aryl of 6 to 30 carbon atoms, a substituted or unsubstituted heteroaryl of 2 to 30 carbon atoms, a substituted or unsubstituted aliphatic hydrocarbon ring-fused aryl of 7 to 30 carbon atoms, and a substituted or unsubstituted silyl of 1 to 30 carbon atoms.

5. The fused cyclic compound of claim 1, wherein m in Chemical Formulas 1 to 4 is 1.

6. The fused cyclic compound of claim 1, wherein the A ring moiety in Chemical Formulas 3 and 4 is a substituted or unsubstituted aromatic hydrocarbon ring of 18 to 24 carbon atoms.

7. The fused cyclic compound of claim 1, wherein the fused cyclic compound represented by any one of Chemical Formulas 1 to 4 contains at least one deuterium atom.

8. The fused cyclic compound of claim 1, wherein the compound represented by any one of Chemical Formulas 1 to 4 is any one selected from the compounds represented by the following Compound 1-1 to Compound 1-180:

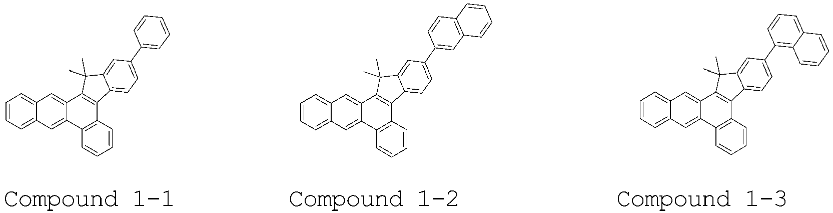

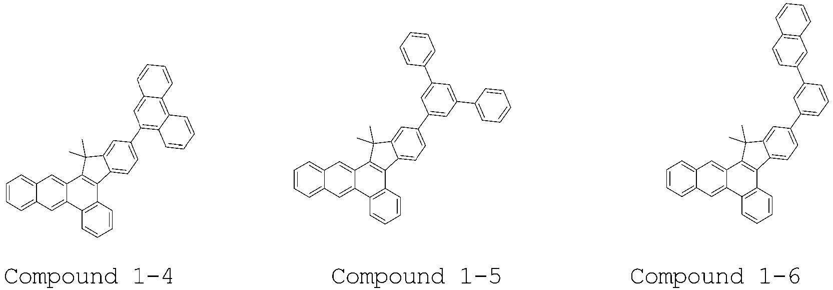

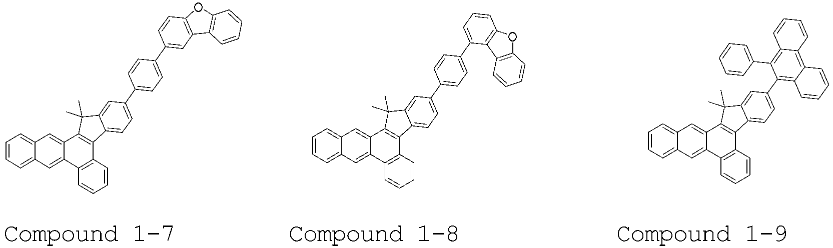

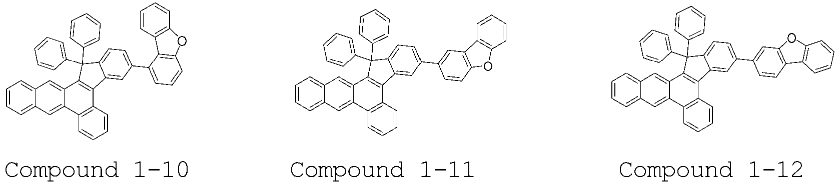

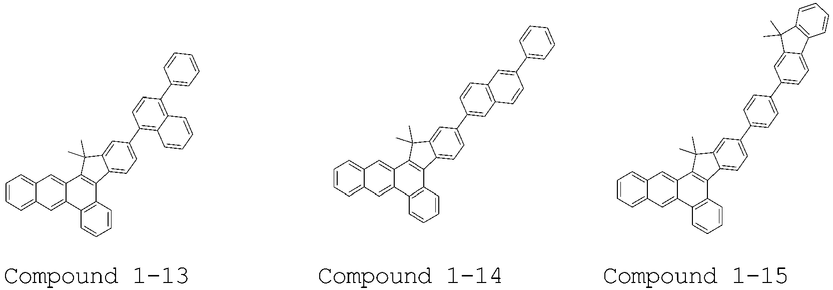

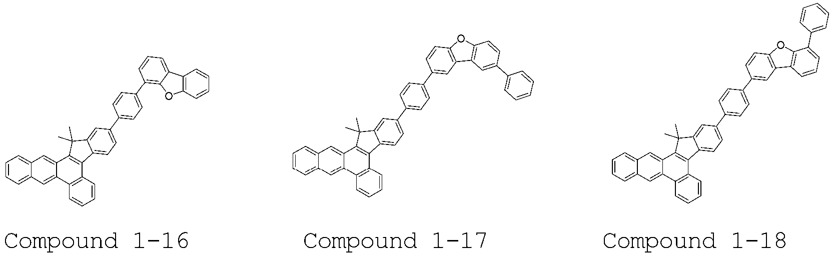

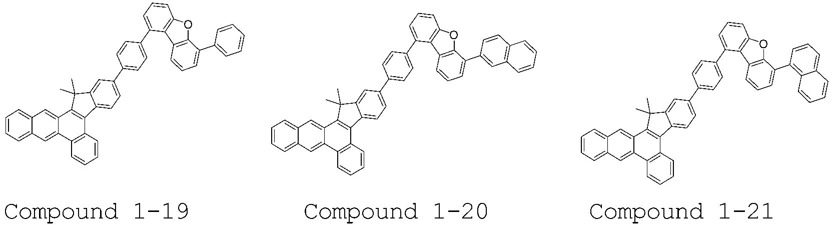

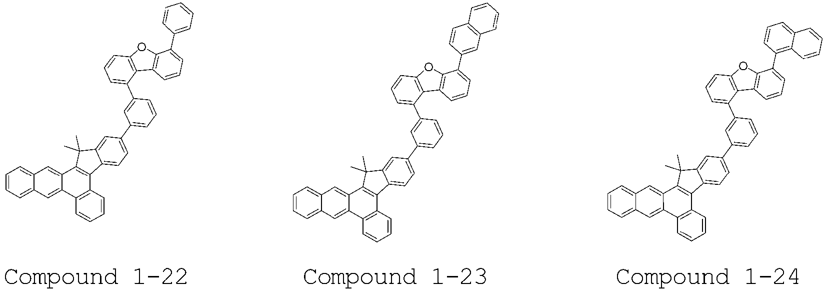

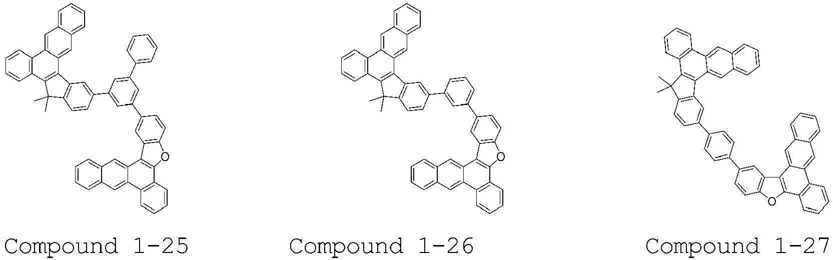

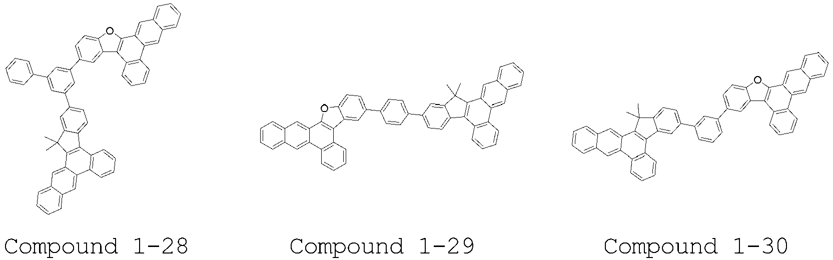

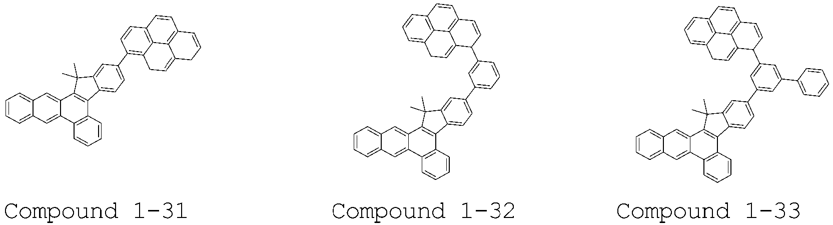

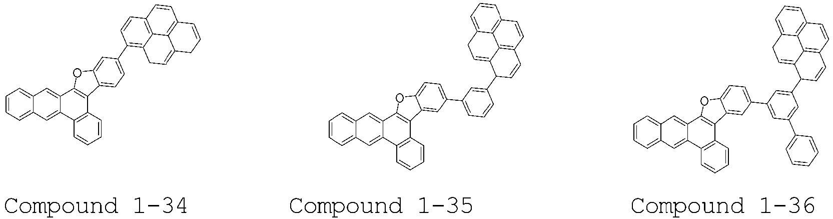

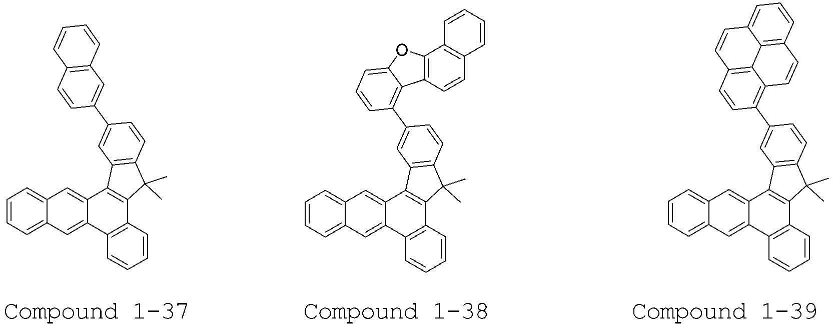

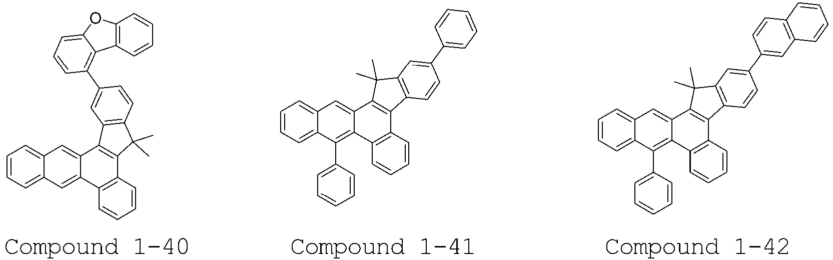

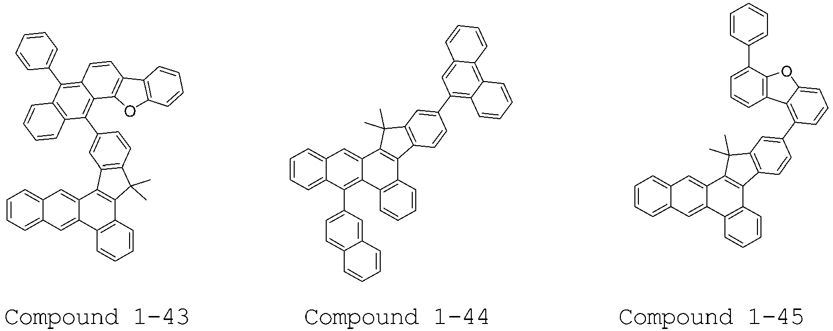

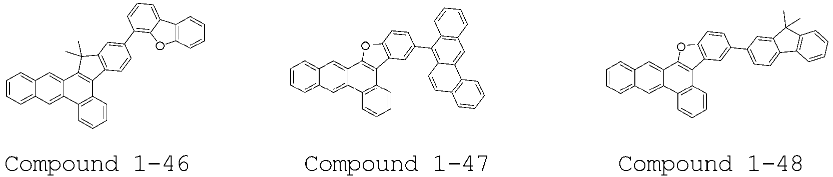

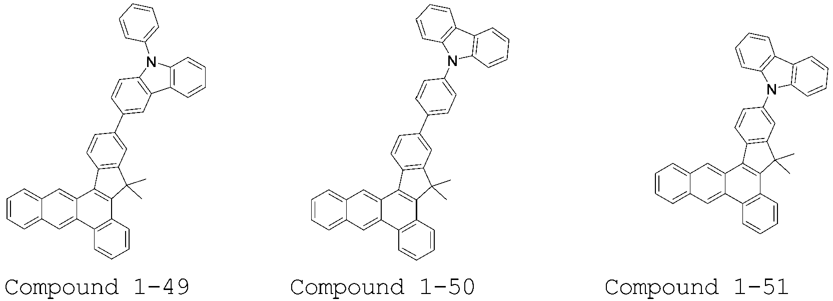

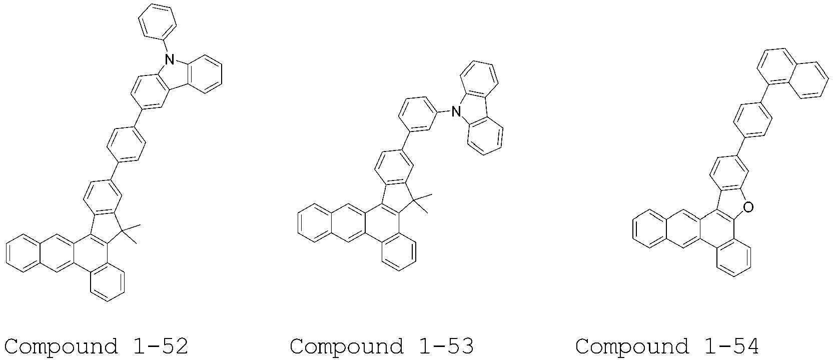

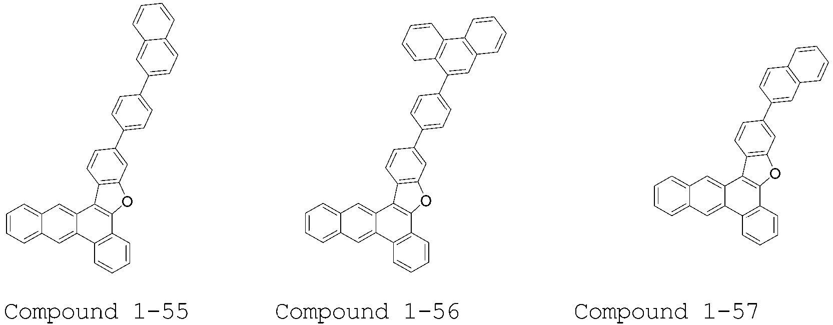

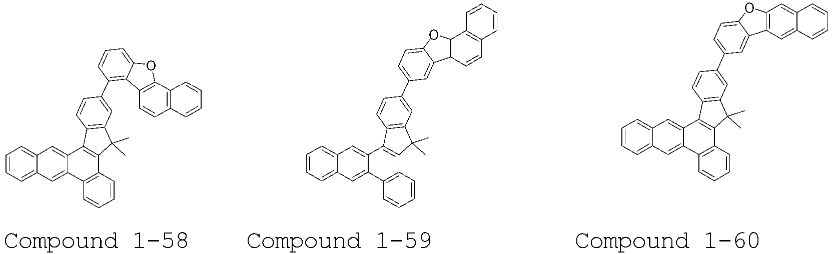

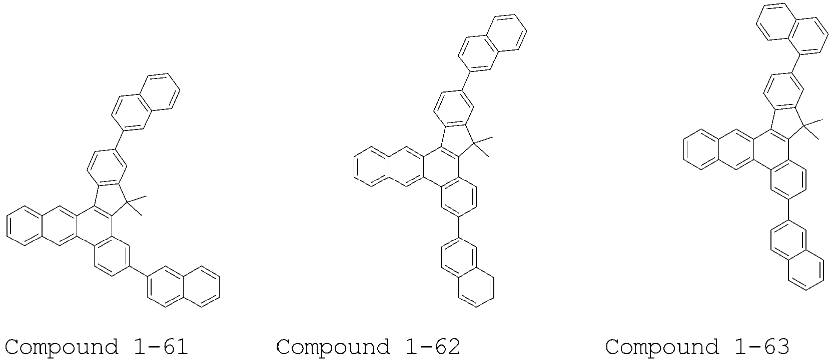

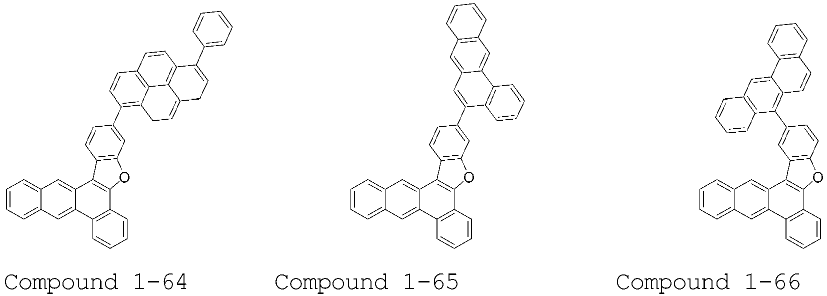

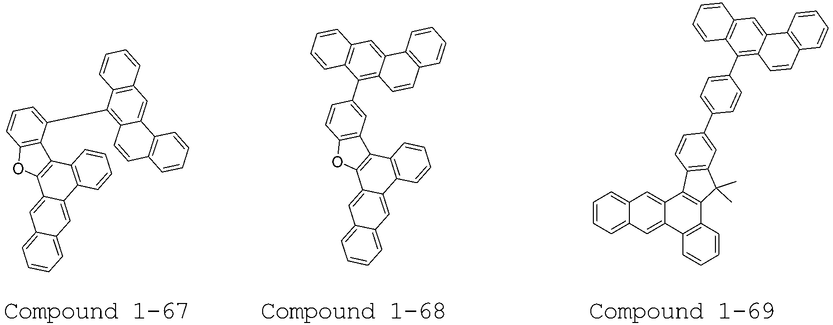

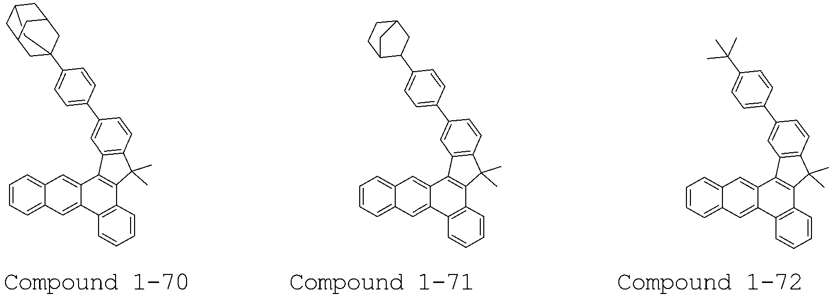

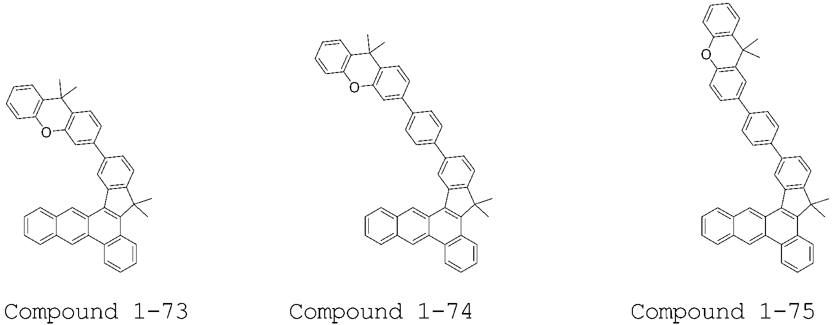

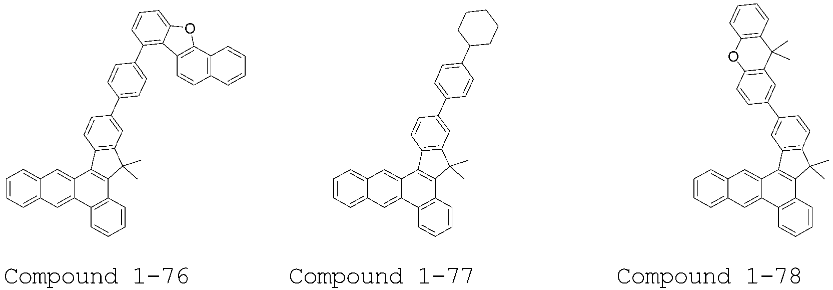

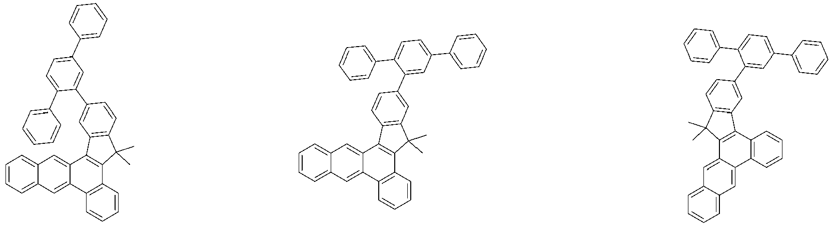

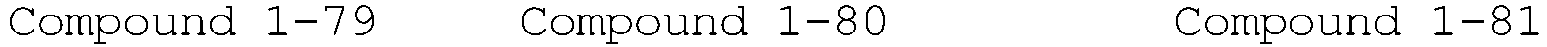

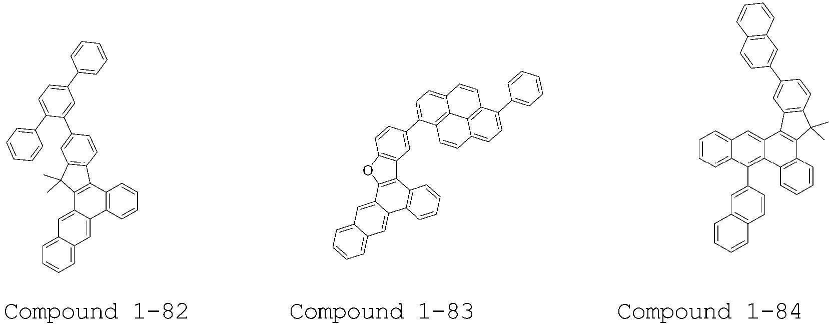

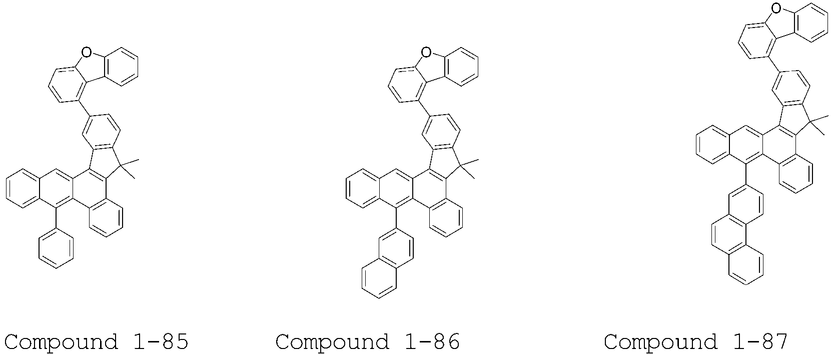

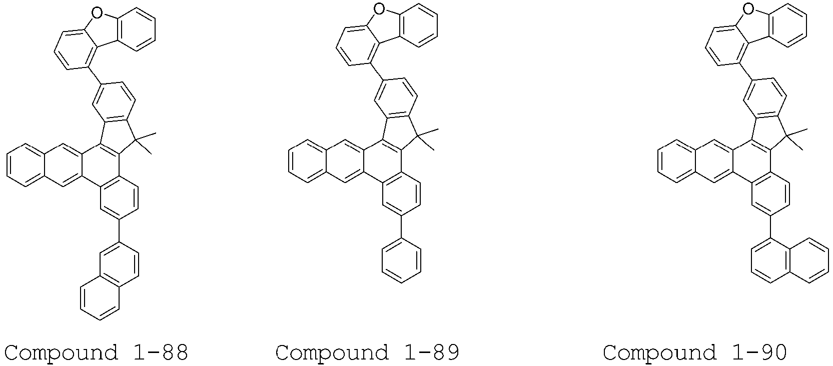

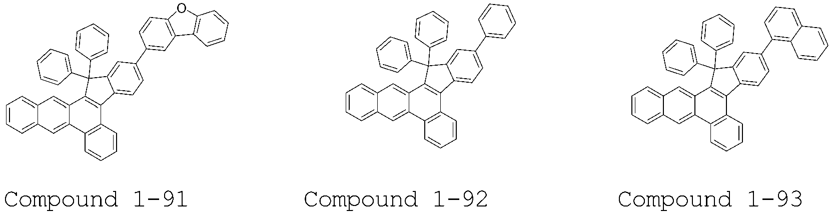

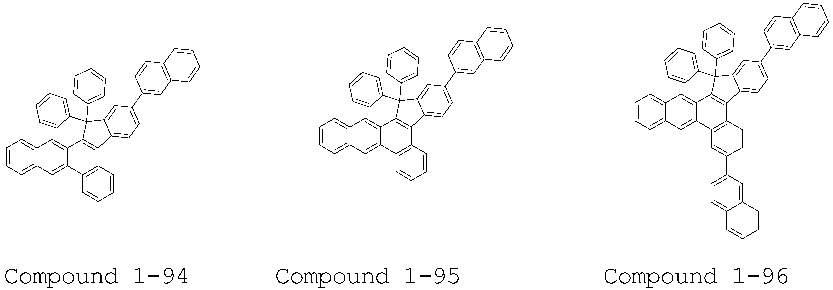

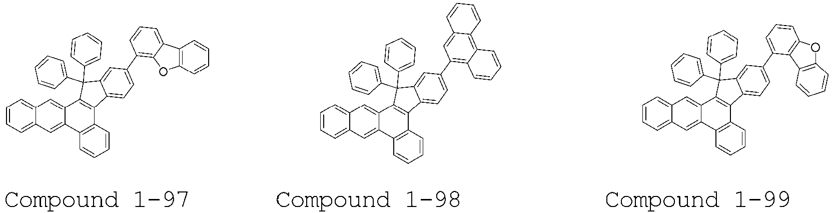

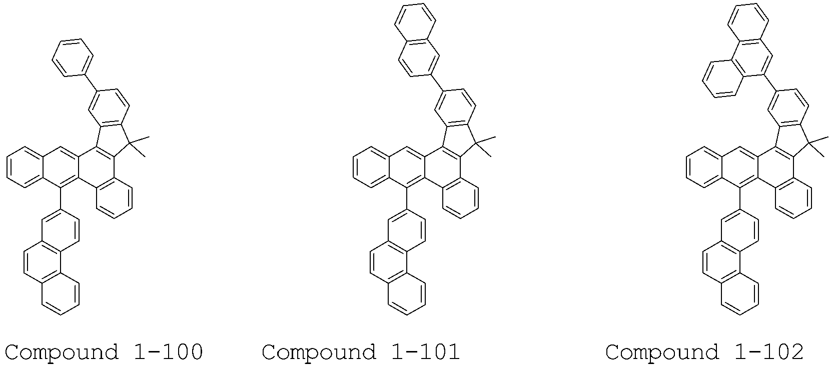

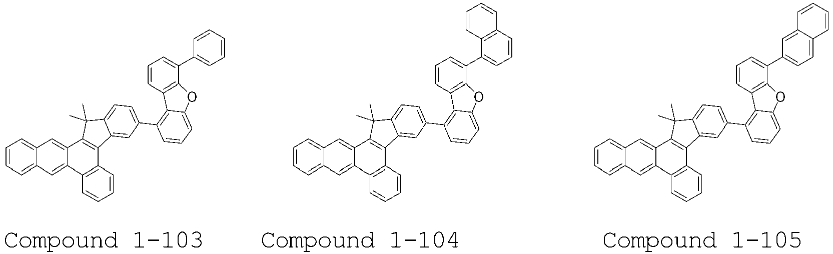

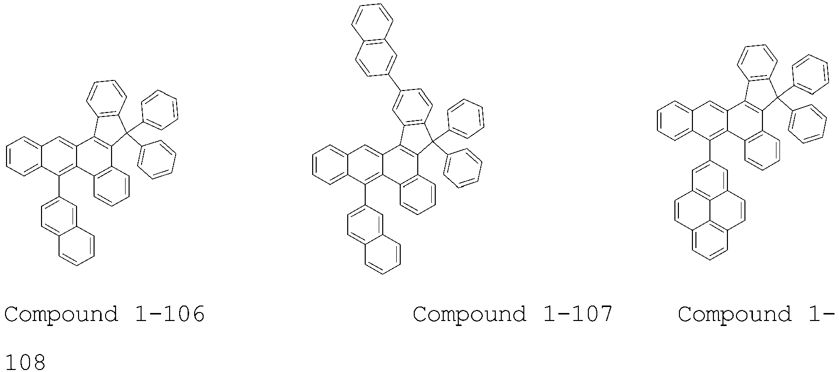

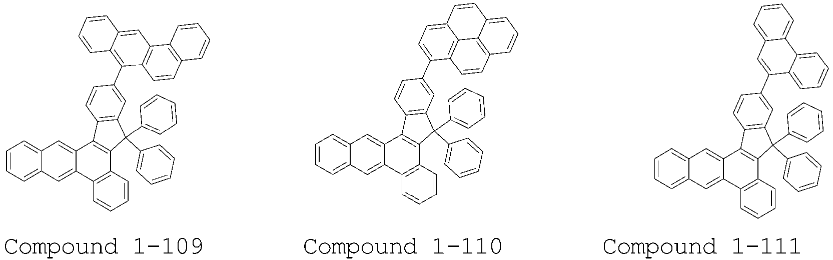

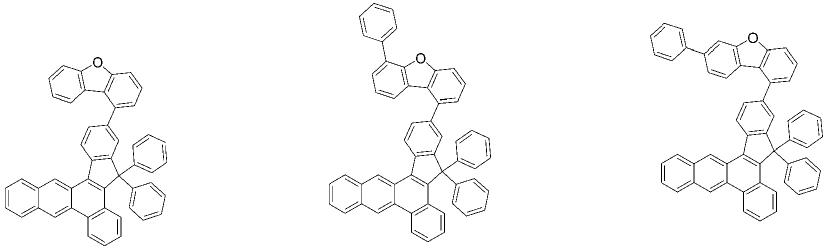

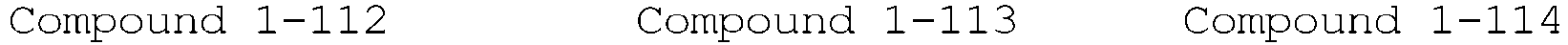

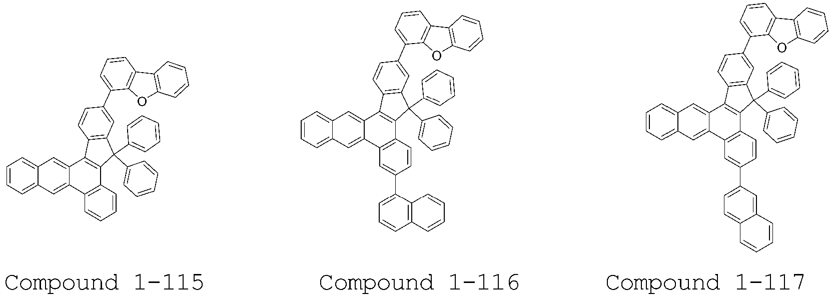

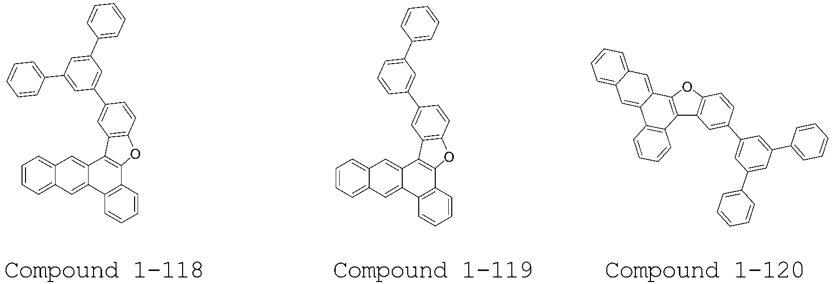

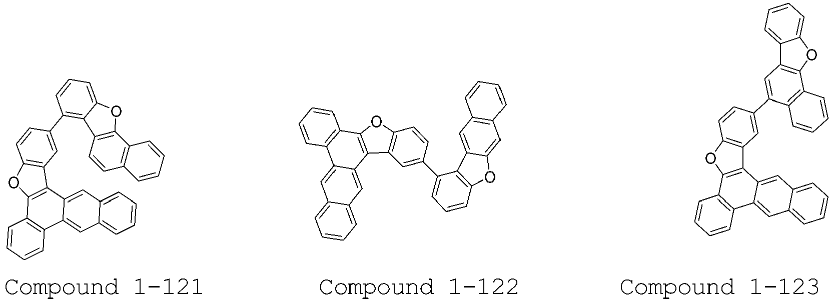

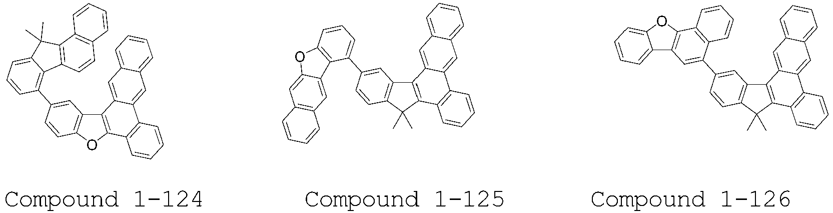

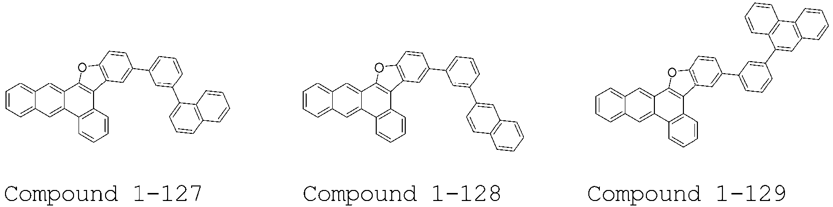

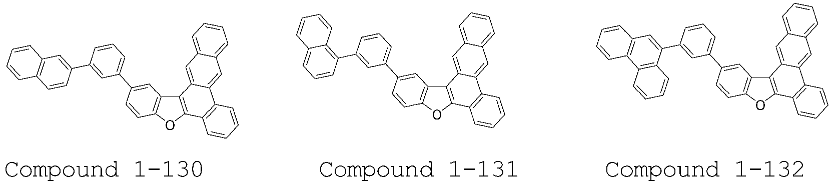

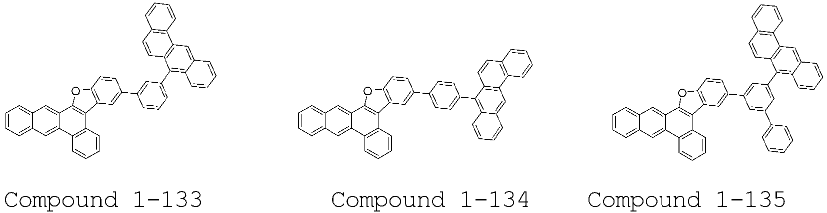

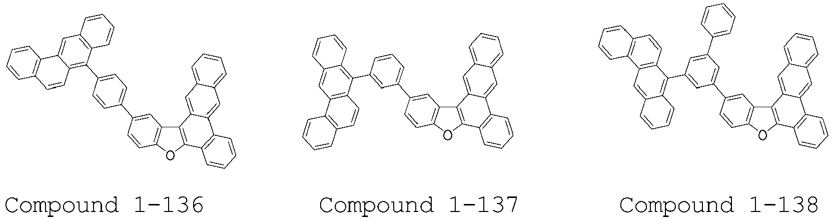

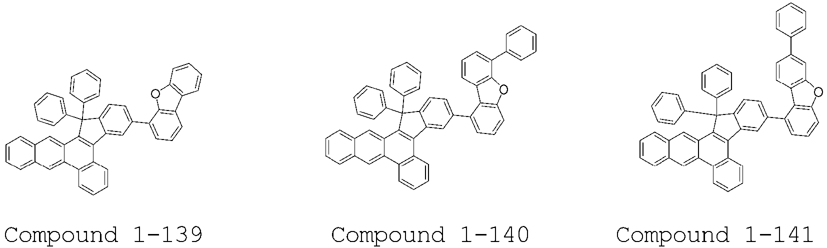

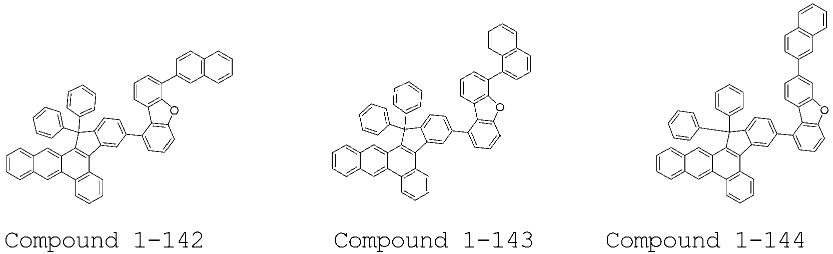

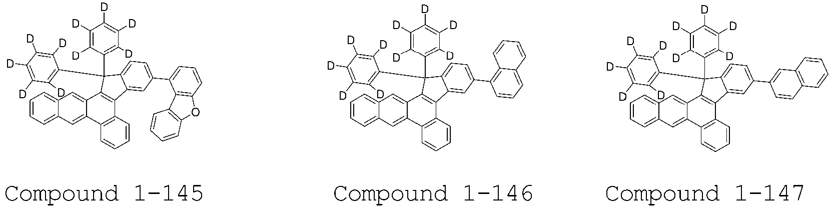

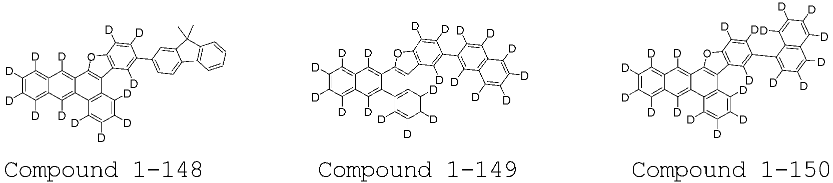

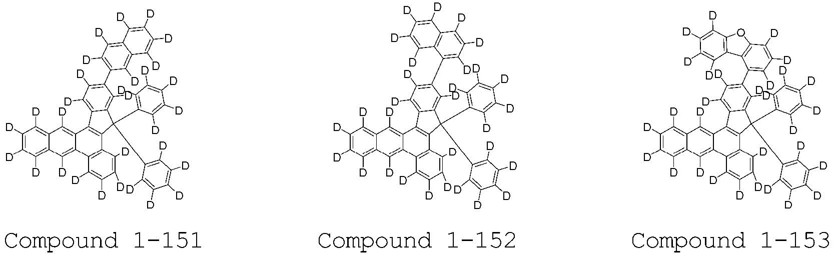

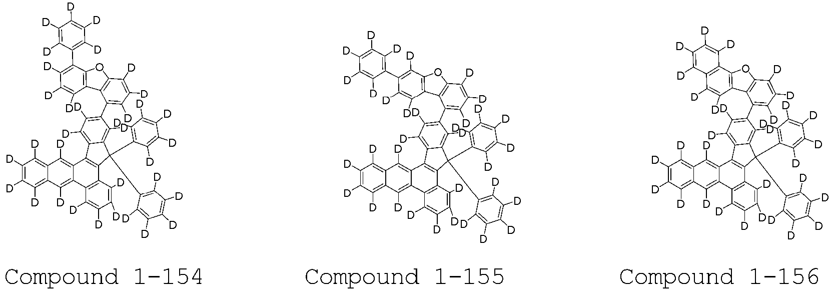

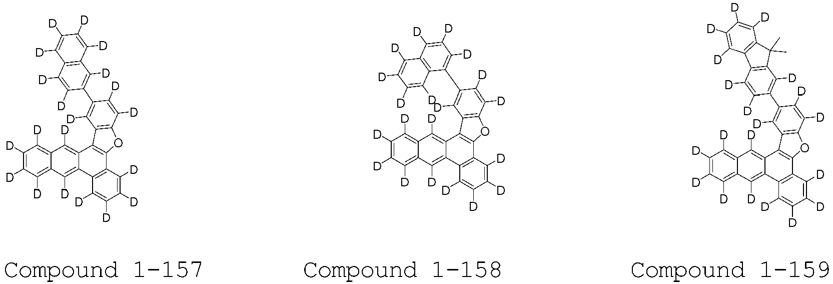

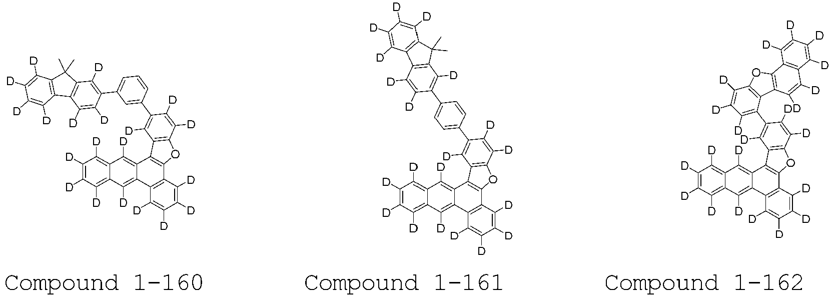

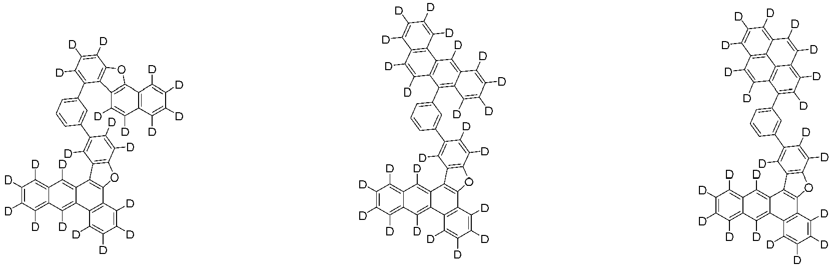

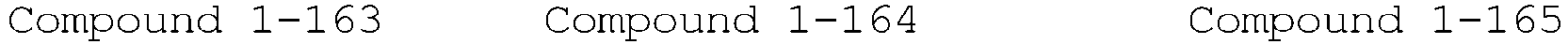

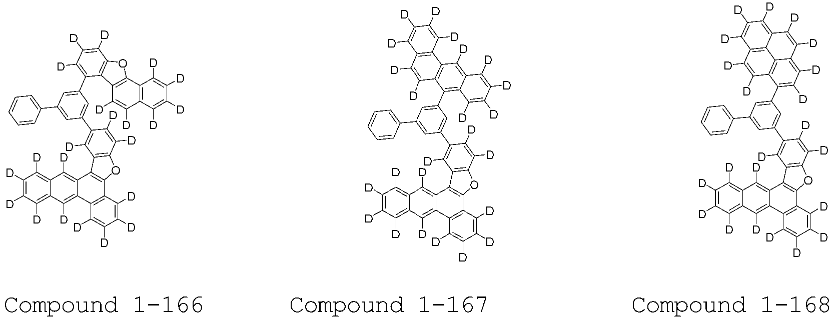

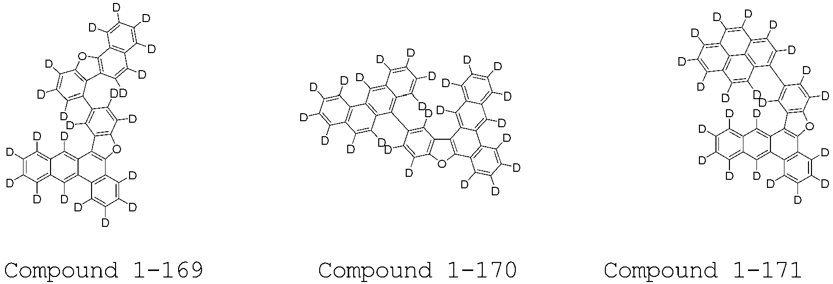

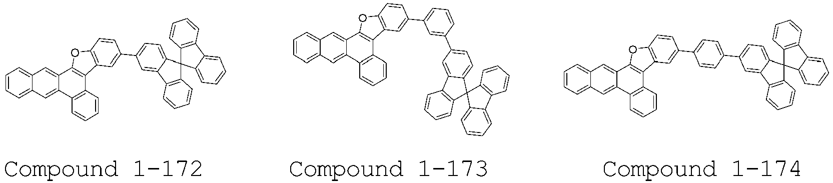

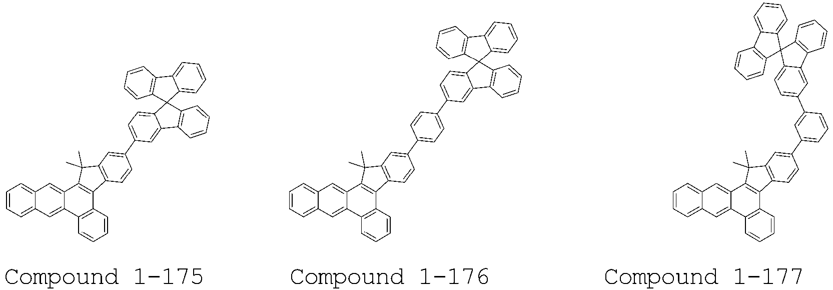

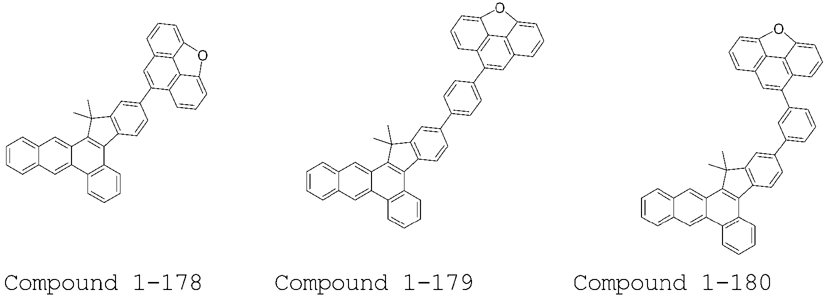

9. An organic light-emitting diode, comprising:
a first electrode;a second electrode facing the first electrode;
a first emission layer disposed between the first electrode and the second electrode and containing a first host and a first dopant; and
a second emission layer containing a second host and a second dopant,
wherein at least one of the first host and the second host comprises a fused cyclic compound represented by any one of the following Chemical Formulas 1 to 4: 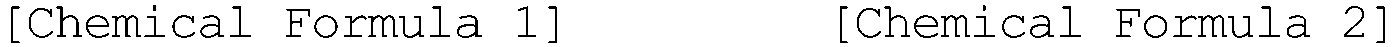 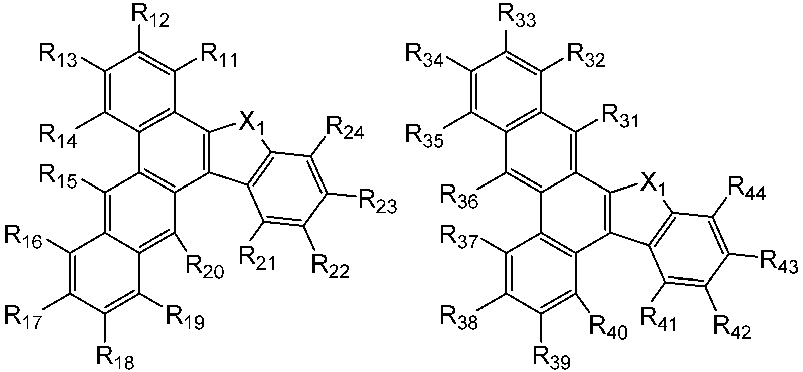 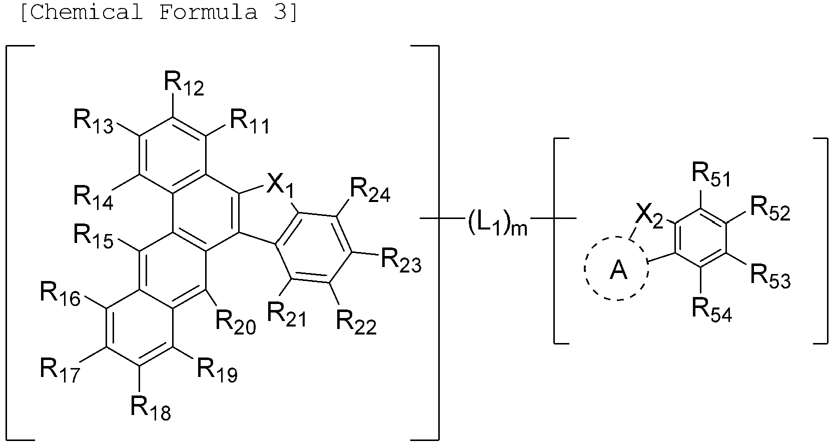 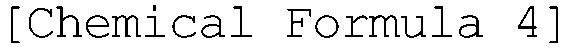 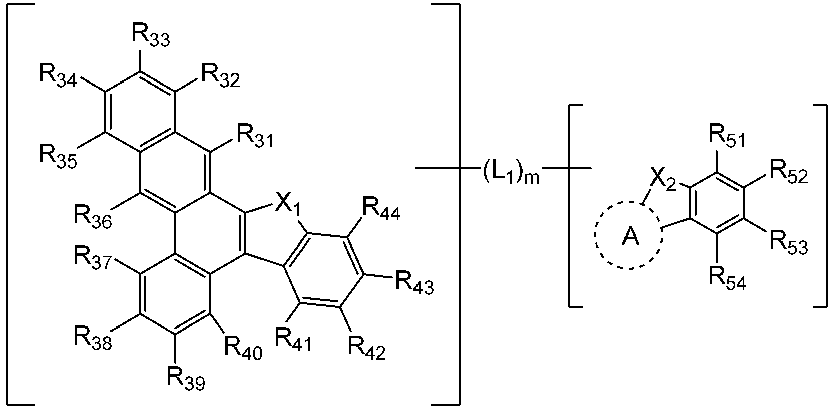
wherein,
X₁ and X₂, which are same or different, are each independently any one of O, S, and CR₁R₂,
R₁, R₂, R₁₁ to R₂₄, R₃₁ to R₄₄, R₅₁ to R₅₄, which are same or different, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
R₁ and R₂ may be linked to each other to additionally form a mono- or polycyclic aliphatic or aromatic ring,
adjacent substituents in each of the R₁₁ to R₂₄ and the R₃₁ to R₄₄ may be linked to each other to additionally form a mono- or polycyclic aliphatic or aromatic ring,
in Chemical Formulas 3 and 4,
the ring moiety A is a substituted or unsubstituted hydrocarbon ring of 6 to 30 carbon atoms,
linkers L₁, which are same or different, are each independently any one selected from a single bond, a substituted or unsubstituted arylene of 6 to 30 carbon atoms, a substituted or unsubstituted heteroarylene of 3 to 30 carbon atoms, and a substituted or unsubstituted, aliphatic hydrocarbon ring-fused arylene of 8 to 30 carbon atoms,
m is an integer of 1 to 3 wherein when m is 2 or more, the corresponding L₁ are same or different,
any one of R₁₁ to R₂₄, and any one of R₅₁ to R₅₄ and the substituents bonded to the carbon atoms as ring members of the ring moiety A in Chemical Formula 3 are each a single bond to L₁,
any one of R₃₁ to R₄₄, and any one of R₅₁ to R₅₄ and the substituents bonded to the carbon atoms as ring members of the ring moiety A in Chemical Formula 4 are each a single bond to L₁,
wherein the term "substituted" in the expression "a substituted or unsubstituted" used for the compounds of Chemical Formula 1 to Chemical Formula 4 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic hydrocarbon ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, and a thionyl of 1 to 30 carbon atoms, with at least one hydrogen atom on the substituent being substitutable with a deuterium or tritium atom.

10. The organic light-emitting diode of claim 9, wherein one of the first and second hosts within the organic light-emitting diode comprises a compound represented by any one of Chemical Formulas 1 to 4 while the other comprises an anthracene compound represented by the following Chemical Formula 5: 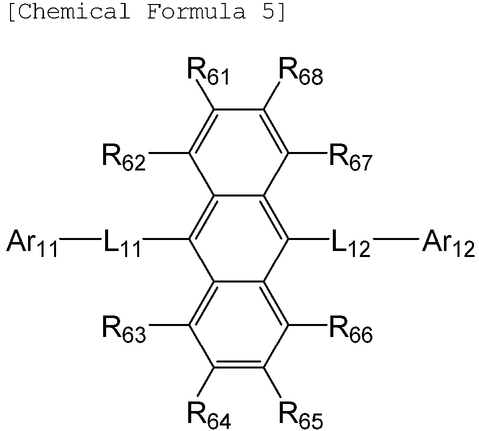 wherein,
the substituents R₆₁ to R₆₈, which are identical to or different from each other, are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
L₁₁ and L₁₂, which are identical to or different from each other, are each independently any one linker selected from a single bond, a substituted or unsubstituted arylene of 6 to 24 carbon atoms, a substituted or unsubstituted heteroarylene of 3 to 24 carbon atoms, and a substituted or unsubstituted aliphatic hydrocarbon ring-fused arylene of 8 to 24 carbon atoms,
Ar₁₁ and Ar₁₂, which are identical to or different from each other, are each independently any one selected from a substituted or unsubstituted aryl of 6 to 30 carbon atoms, a substituted or unsubstituted heteroaryl of 3 to 30 carbon atoms, a substituted or unsubstituted cycloalkyl of 3 to 30 carbon atoms, and a substituted or unsubstituted aliphatic hydrocarbon ring-fused aryl of 8 to 24 carbon atoms,
with the hydrogen atoms bonded to the carbon atoms within Chemical Formula 5 being substitutable with 0 to 60 deuterium atoms or tritium atoms,
wherein the term "substituted" in the expression "substituted or unsubstituted" used for the compound of Chemical Formula 5 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with one or more hydrogen atoms within the substituents being substitutable with deuterium atoms or tritium atoms.

11. The organic light-emitting diode of claim 10, wherein Ar₁₂ in the anthracene compound represented by Chemical Formula 5 may be a substituent represented by the following Structural Formula 1: 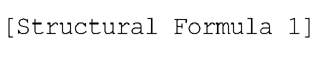 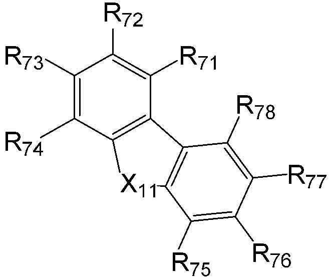 wherein,
X₁₁ is O or S,
one of R₇₁ to R₇₈ is a single bond to L₁₂ in Chemical Formula 5, with preference for R₇₁ or R₇₂ being a single bond to L₁₂,
R₇₁ to R₇₈ exclusive of the single bond to L₁₂ are identical to or different from each other and are each independently any one selected from a hydrogen atom, a deuterium atom, a tritium atom, a substituted or unsubstituted alkyl of 1 to 30 carbon atoms, a substituted or unsubstituted halogenated alkyl of 1 to 30 carbon atoms, a substituted or unsubstituted alkenyl of 2 to 30 carbon atoms, a substituted or unsubstituted alkynyl of 2 to 30 carbon atoms, a substituted or unsubstituted aryl of 6 to 50 carbon atoms, a substituted or unsubstituted cycloalkyl of 3 to 30 carbon atoms, a substituted or unsubstituted cycloalkenyl of 5 to 30 carbon atoms, a substituted or unsubstituted heterocycloalkyl of 2 to 30 carbon atoms, a substituted or unsubstituted heteroalkyl of 2 to 50 carbon atoms, a substituted or unsubstituted heteroaryl of 2 to 50 carbon atoms, a substituted or unsubstituted aromatic hydrocarbon ring-fused cycloalkyl of 7 to 30 carbon atoms, a substituted or unsubstituted aromatic heterocyclic ring-fused cycloalkyl of 5 to 30 carbon atoms, a substituted or unsubstituted aromatic ring-fused heterocycloalkyl of 6 to 30 carbon atoms, a substituted or unsubstituted aliphatic hydrocarbon ring-fused aryl of 8 to 30 carbon atoms, a substituted or unsubstituted aliphatic hydrocarbon ring-fused heteroaryl of 5 to 30 carbon atoms, a substituted or unsubstituted oxy of 1 to 30 carbon atoms, a substituted or unsubstituted thioxy of 1 to 30 carbon atoms, a substituted or unsubstituted thionyl of 1 to 30 carbon atoms, a substituted or unsubstituted amine of 0 to 40 carbon atoms, a substituted or unsubstituted silyl of 0 to 40 carbon atoms, a substituted or unsubstituted germanium of 0 to 40 carbon atoms, a thiol, a hydroxy, a nitro, a cyano, and a halogen,
adjacent substituents of R₇₁ to R₇₈ may be linked to each other to additionally form a mono- or polycyclic aliphatic or aromatic ring,
wherein the term "substituted" in the expression "substituted or unsubstituted" used for the compound of Structural Formula 1 means having at least one substituent selected from the group consisting of a deuterium atom, a tritium atom, a cyano, a halogen, a thiol, a hydroxy, a nitro, an alkyl of 1 to 30 carbon atoms, a halogenated alkyl of 1 to 30 carbon atoms, an alkenyl of 2 to 24 carbon atoms, an alkynyl of 2 to 24 carbon atoms, a cycloalkyl of 3 to 24 carbon atoms, a heteroalkyl of 1 to 24 carbon atoms, an aryl of 6 to 24 carbon atoms, an arylalkyl of 7 to 24 carbon atoms, an alkylaryl of 7 to 24 carbon atoms, a heteroaryl of 2 to 24 carbon atoms, a heteroarylalkyl of 3 to 24 carbon atoms, an alkylheteroaryl of 3 to 24 carbon atoms, an aromatic hydrocarbon ring-fused cycloalkyl of 7 to 30 carbon atoms, an aromatic heterocyclic ring-fused cycloalkyl of 5 to 30 carbon atoms, an aromatic ring-fused heterocycloalkyl of 6 to 30 carbon atoms, an aliphatic hydrocarbon ring-fused aryl of 7 to 30 carbon atoms, an aliphatic hydrocarbon ring-fused heteroaryl of 5 to 30 carbon atoms, an aliphatic heterocyclic ring-fused aryl of 6 to 30 carbon atoms, an aliphatic heterocyclic ring-fused heteroaryl of 5 to 30 carbon atoms, an amine of 1 to 30 carbon atoms, a silyl of 1 to 30 carbon atoms, a germanium of 1 to 30 carbon atoms, an oxy of 1 to 30 carbon atoms, a thioxy of 1 to 30 carbon atoms, and a thionyl of 1 to 30 carbon atoms, with one or more hydrogen atoms within the substituents being substitutable with deuterium atoms or tritium atoms.

12. The organic light-emitting diode of claim 10, wherein the compound represented by Chemical Formula 5 contains at least one deuterium atom.

13. The organic light-emitting diode of claim 10, wherein one of the first host and second host within the organic light-emitting diode comprises two or more types of the anthracene compounds represented by Chemical Formula 5.

14. The organic light-emitting diode of claim 13, wherein one or more types of the anthracene compounds represented by Chemical Formula 5 comprise a substituted or unsubstituted aryl of 6 to 30 carbon atoms for Ar₁₂, and
one or more types of the additionally included anthracene compounds represented by Chemical Formula 5 comprise a substituted or unsubstituted heteroaryl of 2 to 30 carbon atoms for Ar₁₂.

15. The organic light-emitting diode of claim 14, wherein Ar₁₂ in the additionally included anthracene compound represented by Chemical Formula 5 is a substituent represented by Structural Formula 1 which is as defined in claim 11.

16. The organic light-emitting diode of claim 10, wherein the first emission layer in the organic light-emitting diode comprises, as a first host, a compound represented by any one selected from Chemical Formulas 1 to 4; and a host compound different from the compound represented by any one selected from Chemical Formulas 1 to 4 in a ratio of 1:9 to 9:1.

17. The organic light-emitting diode of claim 13, wherein the second emission layer in the organic light-emitting diode comprises, as the second host, two different anthracene compounds represented by Chemical Formula 5 in a ratio of 1:9 to 9:1.
